# NEW EUROPEAN PATENT SPECIFICATION

(11) **EP 3 445 850 B2**
(45) Date of publication and mention of the opposition decision: **29.07.2026**
(45) Mention of the grant of the patent: 27.10.2021
(21) Application number: 17722695.8
(22) Date of filing: 19.04.2017
(51) Int. Cl.: C12N 15/10

(54) **METHODS FOR PROVIDING SINGLE-STRANDED RNA**
VERFAHREN ZUR BEREITSTELLUNG VON EINZELSTRÄNGIGER RNA
PROCÉDÉS DE PRODUCTION D'ARN SIMPLE BRIN

(30) Priority: 22.04.2016 WO PCT/EP2016/059056
(43) Date of publication of application: 27.02.2019
(60) Divisional application: 21204500.9
(73) Proprietor: BioNTech SE, 55131 Mainz (DE)
(72) Inventor: BAIERSDÖRFER, Markus, 55131 Mainz (DE); KARIKÓ, Katalin, 55116 Mainz (DE)
(74) Representative: Schnappauf, Georg
(86) International application number: PCT/EP2017/059293
(87) International publication number: WO 2017/182524

(56) References cited:
- WO-A1-2016/193206
- PE'ERY T ET AL: "Synthesis and Purification of Single-Stranded RNA for Use in Experiments with PKR and in Cell-Free Translation Systems", MET, ACADEMIC PRESS, US, vol. 11, no. 4, 1 April 1997 (1997-04-01), pages 371 - 381, XP004466494, ISSN: 1046-2023, DOI: 10.1006/METH.1996.0435
- KATALIN KARIKÓ ET AL: "Generating the optimal mRNA for therapy: HPLC purification eliminates immune activation and improves translation of nucleoside-modified, protein-encoding mRNA", NUCLEIC ACIDS RESEARCH, OXFORD UNIVERSITY PRESS, GB, vol. 39, no. 21, 1 November 2011 (2011-11-01), pages e142 - 1, XP002696190, ISSN: 1362-4962, [retrieved on 20110902], DOI: 10.1093/NAR/GKR695
- R. M. FRANKLIN ET AL: "Purification and properties of the replicative intermediate of the RNA bacteriophage R17.", PROCEEDINGS NATIONAL ACADEMY OF SCIENCES PNAS, vol. 55, no. 6, 1 June 1966 (1966-06-01), US, pages 1504 - 1511, XP055380412, ISSN: 0027-8424, DOI: 10.1073/pnas.55.6.1504
- SYUN-ICHI URAYAMA ET AL: "A New Fractionation and Recovery Method of Viral Genomes Based on Nucleic Acid Composition and Structure Using Tandem Column Chromatography", MICROBES AND ENVIRONMENTS, vol. 30, no. 2, 1 January 2015 (2015-01-01), pages 199 - 203, XP055380506, ISSN: 1342-6311, DOI: 10.1264/jsme2.ME14174
- CASTILLO ANTONIO ET AL: "Rapid isolation of mycoviral double-stranded RNA from Botrytis cinerea and Saccharomyces cerevisiae", VIROLOGY JOURNAL, BIOMED CENTRAL, LONDON, GB, vol. 8, no. 1, 25 January 2011 (2011-01-25), pages 38, XP021089814, ISSN: 1743-422X, DOI: 10.1186/1743-422X-8-38
- T J MORRIS ET AL: "Isolation and Analysis of Double-Stranded RNA from Virus-Infected Plant and Fungal Tissue", PHYTOPATHOLOGY, vol. 69, no. 8, 1 January 1979 (1979-01-01), pages 854 - 858, XP055380533

## Description

### TECHNICAL FIELD OF THE INVENTION

The present invention relates to methods for providing single-stranded RNA (ssRNA).

### BACKGROUND OF THE INVENTION

During synthesis of mRNA by *in vitro* transcription (IVT) using T7 RNA polymerase (cf. Yin et al., Cell 116 (2004), 393-404) significant amounts of aberrant products, including double-stranded RNA (dsRNA) are produced due to unconventional activity of the enzyme (cf. Triana-Alonso et al., JBC 270 (1995), 6298-6307; Cazenave et al., PNAS USA 91(1994), 6972-6976; Gong et al., JBC 281 (2006), 23533-23544). Since dsRNA induces inflammatory cytokines and activates effector enzymes (cf. Karikó et al., Curr. Opin. Drug Discov. Devel. 10 (2007), 523-532) leading to protein synthesis inhibition, it is important to remove dsRNA from the IVT mRNA that will be used as therapeutic.

To date two different methods have been described for the removal of dsRNA from IVT mRNA. One method is the purification of IVT mRNA by ion-pair reversed phase HPLC using a non-porous (cf. Weissman et al., Methods Mol. Biol. 969 (2013), 43-54) or porous (cf. US 8,383,340 B2) C-18 polystyrene-divinylbenzene (PS-DVB) matrix. However, methods using HPLC to purify RNA have several disadvantages such as complex equipment; use of toxic solvents like acetonitrile; long duration of a standard purification run; difficult scale-up; costs; and degradation of long RNA because of shearing.

Alternatively, an enzymatic based method has been established using *E. coli* RNaseIII that specifically hydrolyzes dsRNA but not ssRNA, thereby eliminating dsRNA contaminants from IVT mRNA preparations (cf. WO 2013/102 203 A1). However, it is possible that the RNaseIII induces undesired reactions (such as an undesired immune reaction) in the patient to be treated with the RNA. Thus, before administering the RNA to the patient, it is necessary to remove the enzyme thereby increasing the complexity and cost of the method. Moreover, the use of RNaseIII often leads to a partial degradation of ssRNA, especially long ssRNA, during incubation. This is likely caused by RNaseIII-catalyzed hydrolysis of double-stranded secondary structures contained in ssRNA.

In 1966 a non-ionic interaction was described between unmodified cellulose powder CF-11 and RNA in the presence of EtOH, and was used to separate sRNA ("soluble RNA") from ribosomal RNA (rRNA) by chromatography (Barber, R. Biochim. Biophys. Acta 114 (1966), 422-424). While sRNA eluted with 35% EtOH from the column, the rRNA could be selectively eluted by reducing the EtOH concentration of the chromatography buffer to 15%.

Franklin et al. (PNAS USA 55 (1966), 1504-1511) used the same separation principle to isolate replicative intermediate (RI) RNA of the RNA bacteriophage R17 from total RNA of *E. coli.* Here, the cellulose-bound RI RNA, identified as RNase A-resistant dsRNA, was eluted efficiently only in buffer free of EtOH. This technique was adapted to isolate dsRNA from *Cryphonectria parasitica,* a parasitical fungus of chestnut tree (Day et al., Phytopathology 67 (1977), 1393).

Morris and Dodds (Phytopathology 69 (1977), 854-858) simplified the previously described cellulose-based procedures by selectively pulling down viral dsRNA from plant and fungal RNA isolates in the presence of 15% (v/v) EtOH. This procedure has been used for decades to isolate dsRNA and has undergone only minor modifications during the years, e.g., using commercial minicolumns packed with CF-11 cellulose, to speed up the process and to increase the sample throughput (cf. Castillo et al., Virol. J. 8 (2011), 38; Okada et al., Arch. Virol. 159 (2014), 807-809).

Pe'ery et al. (Methods 11 (1997), 371-381) disclose the synthesis and purification of ssRNA for use in experiments with PKR and in cell-free translation systems. Karikó et al (Nucl. Acids Res. 39 (2011), e142) report that contaminants from *in vitro*-transcribed RNA are a source of innate immune activation and their removal increases RNA translation and eliminates type I interferon and inflammatory cytokine secretion. Urayama et al. (Microbes Environ. 30 (2015), 199-203) describe a fractionation and recovery method of viral genomes based ion nucleic acid composition and structure using tandem column chromatography.

It is an object of the present invention to provide means that address one or more problems described above. In particular, it is an object of the present invention to provide an alternative method for providing ssRNA which is cost effective, simple, and less time-consuming than methods based on HPLC; which avoids toxic substances; which can be easily upscaled; which provides ssRNA in a yield and in a purity comparable to the ssRNA obtained by using HPLC; which does not affect long RNAs; and/or which does not degrade RNA. Such objects underlying the present invention are solved by the subject-matter as disclosed or defined anywhere herein, for example by the subject-matter of the attached claims.

### SUMMARY OF THE INVENTION

In a first aspect, the present invention provides a method for providing ssRNA, comprising (i) producing an RNA preparation comprising ssRNA by *in vitro* transcription; (ii) contacting the RNA preparation with a cellulose material under conditions which allow binding of double-stranded RNA (dsRNA) to the cellulose material and do not allow binding of ssRNA to the cellulose material; and (iii) separating the ssRNA from the cellulose material under conditions which allow binding of dsRNA to the cellulose material and do not allow binding of ssRNA to the cellulose material, wherein in step (ii) the RNA preparation is provided as a liquid comprising ssRNA and a first buffer and/or the cellulose material is provided as a suspension in a first buffer, wherein the first buffer comprises water, ethanol and a salt in a concentration which allows binding of dsRNA to the cellulose material and which does not allow binding of ssRNA to the cellulose material; and the concentration of ethanol in the first buffer is 14 to 20% (v/v), the concentration of the salt in the first buffer is 15 to 70 mM and the first buffer comprises tris(hydroxymethyl)aminomethane (TRIS) as a buffering substance.

Thus, in the first aspect, steps (ii) and (iii) are conducted under conditions which allow binding of dsRNA to the cellulose material and do not allow binding of ssRNA to the cellulose material (this first aspect is sometimes referred to herein as "negative" purification procedure because it allows the selective binding of dsRNA to the cellulose material, whereas ssRNA remains unbound).

In one embodiment of the negative purification procedure, step (ii) comprises mixing the RNA preparation comprising ssRNA with the cellulose material under shaking and/or stirring, preferably for at least 5 min, more preferably for at least 10 min.

In one embodiment of the negative purification procedure, in step (ii) the salt comprised in the first buffer is sodium chloride. In one embodiment, the concentration of ethanol in the first buffer is 14 to 16% (v/v). In one embodiment, the concentration of the salt in the first buffer is 20 to 60 mM. In one embodiment, the first buffer further comprises a chelating agent, preferably EDTA.

In one embodiment of the negative purification procedure, in step (ii) and/or (iii) the mixture of the RNA preparation, the cellulose material, and the first buffer is provided in a tube and step (iii) comprises (1) applying gravity or centrifugal force to the tube such that the liquid and solid phases are separated; and (2) either collecting the supernatant comprising ssRNA or removing the cellulose material. In an alternative embodiment, in step (ii) and/or (iii) the mixture of the RNA preparation, the cellulose material, and the first buffer is provided in a spin column or filter device and step (iii) comprises (1') applying gravity, centrifugal force, pressure, or vacuum to the spin column or filter device such that the liquid and solid phases are separated; and (2') collecting the flow through comprising ssRNA.

In one embodiment of the negative purification procedure, steps (ii) and (iii) are repeated once or two or more times, wherein the ssRNA preparation obtained after step (iii) of one cycle of steps (ii) and (iii) is used as RNA preparation in step (ii) of the next cycle and in step (ii) of each cycle of steps (ii) and (iii) fresh cellulose material is used.

In a second aspect, the present invention provides a method for providing ssRNA, comprising: (i) producing an RNA preparation comprising ssRNA by *in vitro* transcription; (ii) contacting the RNA preparation with a cellulose material under conditions which allow binding of dsRNA and ssRNA to the cellulose material; and (iii) separating the ssRNA from the cellulose material under conditions which allow binding of dsRNA to the cellulose material and do not allow binding of ssRNA to the cellulose material, wherein step (iii) comprises: (1) mixing the cellulose material to which dsRNA and ssRNA are bound with a first buffer under shaking and/or stirring, wherein the first buffer comprises water, ethanol and a salt in a concentration which allows binding of dsRNA to the cellulose material and does not allow binding of ssRNA to the cellulose material; and (2) separating the liquid phase comprising ssRNA from the cellulose material; and the concentration of ethanol in the first buffer is 14 to 20% (v/v), the concentration of the salt in the first buffer is 15 to 70 mM and the first buffer comprises tris(hydroxymethyl)aminomethane (TRIS) as a buffering substance.

Thus, in the second aspect, step (ii) is conducted under conditions which allow binding of dsRNA and ssRNA to the cellulose material; and step (iii) is conducted under conditions which allow binding of dsRNA to the cellulose material and do not allow binding of ssRNA to the cellulose material (this second aspect is sometimes referred to herein as "positive" purification procedure, because first dsRNA and ssRNA are bound to the cellulose material and then ssRNA is selectively released from the cellulose material, whereas dsRNA remains bound).

In one embodiment of the positive purification procedure, step (ii) comprises (1) mixing the RNA preparation comprising ssRNA with the cellulose material under shaking and/or stirring, preferably for at least 5 min, more preferably for at least 10 min; and (2) separating the cellulose material to which dsRNA and ssRNA are bound from the remainder.

In one embodiment of the positive purification procedure, in step (ii) the RNA preparation is provided as a liquid comprising ssRNA and a second buffer and/or the cellulose material is provided as a suspension in a second buffer, wherein the second buffer comprises water, ethanol and a salt, preferably sodium chloride, in a concentration which allows binding of dsRNA and ssRNA to the cellulose material. In one embodiment, the concentration of ethanol in the second buffer is at least 35% (v/v), preferably 38 to 42% (v/v). In one embodiment, the concentration of the salt in the second buffer is 15 to 70 mM, preferably 20 to 60 mM. In one embodiment, the second buffer further comprises a buffering substance, preferably tris(hydroxymethyl)aminomethane (TRIS), and/or a chelating agent, preferably EDTA.

In one embodiment of the positive purification procedure, in step (ii)(1) and/or (ii)(2) the mixture of the RNA preparation and the cellulose material obtained in step (ii)(1) is provided in a tube and step (ii)(2) comprises (2a) applying gravity or centrifugal force to the tube such that the liquid and solid phases are separated; and (2b) either removing the supernatant or collecting the cellulose material to which dsRNA and ssRNA are bound. In an alternative embodiment, in step (ii)(1) and/or (ii)(2) the mixture of the RNA preparation and the cellulose material obtained in step (ii)(1) is provided in a spin column or filter device and step (ii)(2) comprises (2a') applying gravity, centrifugal force, pressure, or vacuum to the spin column or filter device such that the liquid and solid phases are separated; and (2b') discarding the flow through.

In one embodiment of the positive purification procedure, step (ii) further comprises (3) adding an aliquot of the second buffer to the cellulose material to which dsRNA and ssRNA are bound; (4) incubating the resulting mixture under shaking and/or stirring, preferably for at least 5 min, more preferably for at least 10 min; and (5) separating the cellulose material to which dsRNA and ssRNA are bound from the liquid phase; and optionally (6) repeating steps (3) to (5) once or two or more times.

In one embodiment of the positive purification procedure, in step (iii)(1) the cellulose material to which dsRNA and ssRNA are bound is mixed with the first buffer under shaking and/or stirring for at least 5 min, preferably for at least 10 min. In one embodiment, the salt comprised in the first buffer is sodium chloride. In one embodiment, the concentration of ethanol in the first buffer is 14 to 16% (v/v). In one embodiment, the concentration of the salt in the first buffer is 20 to 60 mM. In one embodiment, the first buffer further comprises a chelating agent, preferably EDTA.

In one embodiment of the positive purification procedure, in step (iii) the mixture of the cellulose material and the first buffer is provided in a tube and step (iii)(2) comprises (2a) applying gravity or centrifugal force to the tube such that the liquid and solid phases are separated; and (2b) either collecting the supernatant comprising ssRNA or removing the cellulose material. In an alternative embodiment, in step (iii) the mixture of the cellulose material and the first buffer is provided in a spin column or filter device and step (iii)(2) comprises (2a') applying gravity, centrifugal force, pressure, or vacuum to the spin column or filter device; and (2b') collecting the flow through comprising ssRNA.

In one embodiment of the positive purification procedure, steps (ii) and (iii) are repeated once or two or more times, wherein the ssRNA preparation obtained after step (iii) of one cycle of steps (ii) and (iii) is used as RNA preparation in step (ii) of the next cycle and in step (ii) of each cycle of steps (ii) and (iii) fresh cellulose material is used.

In one embodiment of the positive purification procedure, in step (ii) the cellulose material is provided in a column, step (ii) comprises loading the RNA preparation onto the column under conditions which allow binding of dsRNA and ssRNA to the cellulose material, and step (iii) comprises eluting the ssRNA from the cellulose material under conditions which allow binding of dsRNA to the cellulose material and do not allow binding of ssRNA to the cellulose material. In one embodiment, in step (ii) the RNA preparation is provided and loaded onto the column as a liquid comprising ssRNA and a second buffer, wherein the second buffer comprises water, ethanol and a salt, preferably sodium chloride, in a concentration which allows binding of dsRNA and ssRNA to the cellulose material. In one embodiment, the concentration of ethanol in the second buffer is at least 35% (v/v), preferably 38 to 42% (v/v). In one embodiment, the concentration of the salt in the second buffer is 15 to 70 mM, preferably 20 to 60 mM. In one embodiment, the second buffer further comprises a buffering substance, preferably tris(hydroxymethyl)aminomethane (TRIS), and/or a chelating agent, preferably EDTA. In one embodiment, step (iii) is conducted using the first buffer as eluent, wherein preferably the salt comprised in the first buffer is sodium chloride. In one embodiment, the concentration of ethanol in the first buffer is 14 to 16% (v/v). In one embodiment, the concentration of the salt in the first buffer is 20 to 60 mM. In one embodiment, the first buffer further comprises a chelating agent, preferably EDTA.

In one embodiment of the first and second aspect, the RNA preparation is produced by using an RNA polymerase selected from the group consisting of T3, T7 and SP6 RNA polymerases.

In one embodiment of the first and second aspect, prior to step (ii) the RNA preparation is subjected to at least one pre-purification treatment. In one embodiment, the at least one pre-purification treatment comprises one or more of the following: precipitation of nucleic acids, preferably using lithium chloride; binding of nucleic acids to magnetic beads; ultrafiltration; and degradation of DNA, preferably using duplex-specific nuclease (DSN).

In one embodiment of the first and second aspect, the ssRNA is mRNA or an inhibitory RNA (such as an antisense RNA, siRNA, or miRNA).

In one embodiment of the first and second aspect, the ssRNA has a length of at least 2,700 nt, preferably at least 2,800 nt, at least 2,900 nt, at least 3,000 nt, at least 3,100 nt, at least 3,200 nt, at least 3,300 nt, at least 3,400 nt, such as at least 3500 nt, at least 3,600 nt, at least 3,700 nt, at least 3,800 nt, at least 3,900 nt, at least 4,000 nt, at least 4,100 nt, at least 4,200 nt, at least 4,300 nt, at least 4,400 nt, or at least 4500 nt.

In one embodiment of the first and second aspect, the cellulose material comprises cellulose fibers, preferably cellulose fibers of a grade suitable for use as a partition chromatography reagent. In one embodiment, prior to contacting with the RNA preparation in step (ii), the cellulose material is provided as a washed cellulose material. In one embodiment, the washing of the cellulose material includes (I) mixing the cellulose material with a washing solution under shaking and/or stirring, preferably for at least 5 min, more preferably for at least 10 min; and (II) either removing the liquid or collecting the cellulose material; and optionally (III) repeating steps (I) and (II) once or two or more times. In one embodiment, the washing solution has the composition of (A) the first buffer as defined above or below if step (ii) is conducted under conditions which allow binding of dsRNA to the washed cellulose material and do not allow binding of ssRNA to the washed cellulose material (i.e., in the embodiments of the "negative" purification procedure), or (B) the second buffer as defined above or below if step (ii) is conducted under conditions which allow binding of dsRNA and ssRNA to the washed cellulose material (i.e., in the embodiments of the "positive" purification procedure).

Also described herein is ssRNA which is obtainable by any method of the first or second aspect. The ssRNA may be substantially free of dsRNA and/or substantially free of DNA, preferably substantially free of dsRNA and DNA. This ssRNA can be used in therapy.

Further aspects as well as advantages and novel features of the present invention will become apparent from the following detailed description optionally in conjunction with the accompanying drawings.

### BRIEF DESCRIPTION OF THE DRAWINGS

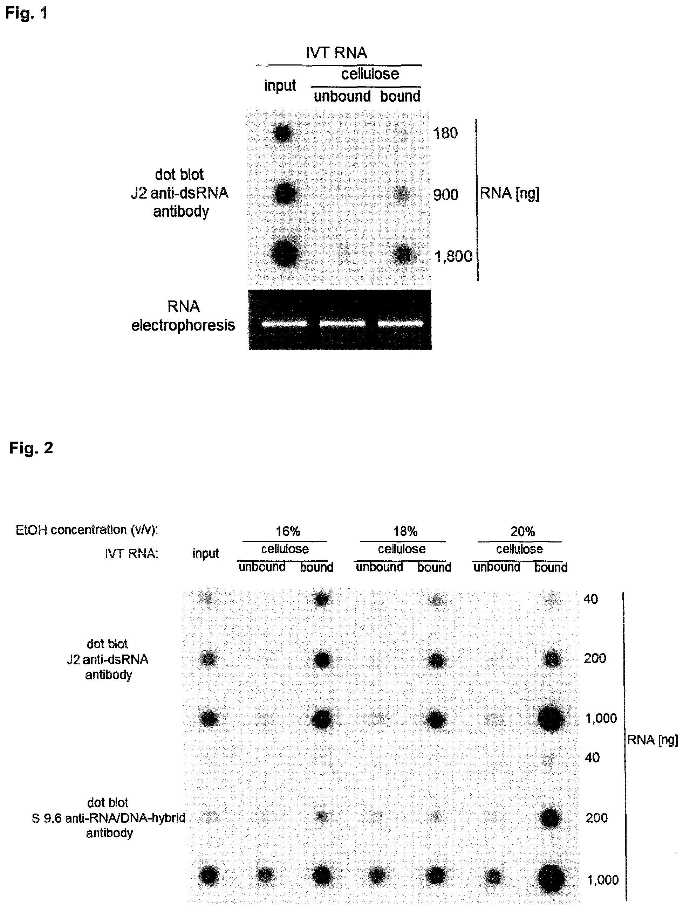

**Figure 1****: Pull-down of dsRNA from IVT RNA by cellulose.** After incubation with a cellulose material in the presence of 1x STE buffer containing 16% (v/v) EtOH the unbound and bound fractions of 50 µg of 2,500 nt long m1Ψ-modified IVT RNA were analyzed for dsRNA contaminants by dot blotting using dsRNA-specific J2 antibody. For comparison unpurified RNA (input) was analyzed in parallel. 180 ng, 900 ng and 1,800 ng RNA of the corresponding RNAs were loaded for dot blot analysis. To control RNA integrity 80 ng of the RNAs were loaded onto a 1.4% (w/v) agarose gel and separated by electrophoresis.
**Figure 2****: The impact of different concentration of EtOH on the efficiency of dsRNA removal from IVT RNA by cellulose.** After incubation with a cellulose material in the presence of 1x STE buffer containing 16% (v/v), 18% (v/v) or 20% (v/v) EtOH the unbound and bound fractions of 50 µg of 1,500 nt-long Ψ-modified and D2-capped IVT RNA were analyzed for dsRNA and RNA/DNA hybrid contaminants by dot blotting using dsRNA-specific J2 antibody or RNA-DNA hybrid-specific S 9.6 antibody, respectively. For comparison unpurified RNA (input) was analyzed in parallel. 40 ng, 200 ng and 1,000 ng RNA of the corresponding RNAs were loaded onto two separate membranes, each hybridized with the indicated antibodies in dot blot analysis.

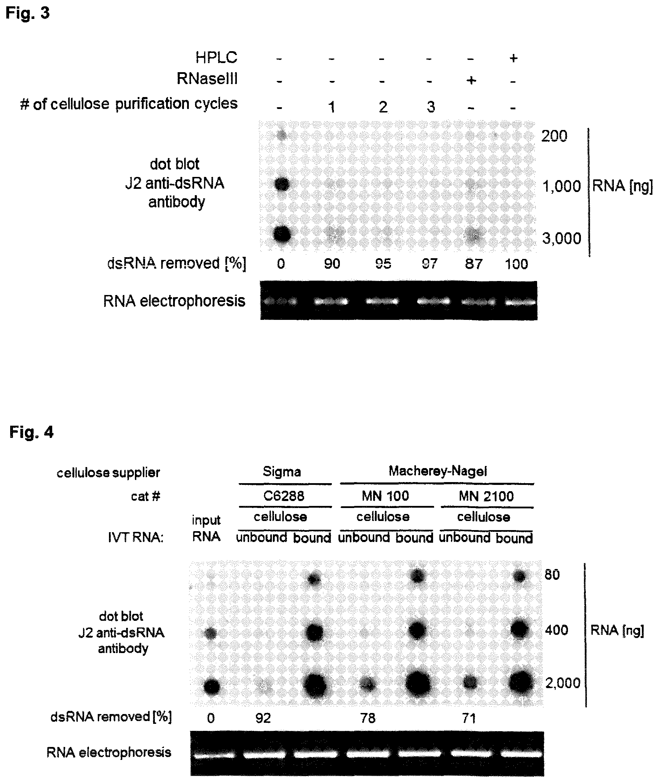

**Figure 3****: Comparison of cellulose purification of IVT RNA to RNaseIII treatment and HPLC purification.** 100 µg of 2,500 nt-long m1Ψ-modified IVT RNA was purified lx, 2x or 3x by cellulose using microcentrifuge spin columns and 1x STE buffer containing 16% (v/v) EtOH. 200 ng, 1,000 ng and 3,000 ng of cellulose-purified RNA were analyzed for dsRNA contaminants by dot blotting using dsRNA-specific J2 antibody. For comparison, the same amounts of unpurified RNA as well as RNaseIIIl-treated and HPLC-purified RNAs were loaded onto the dot blot membrane. Hybridization signals were quantitated by densitometry and values are expressed as percentage of dsRNA removed from unpurified RNA. To control RNA integrity 80 ng of the RNAs were loaded onto a 1.4% (w/v) agarose gel and separated by electrophoresis.
**Figure 4****: Comparison of the performance of different types of cellulose in removing dsRNA from IVT RNA.** The unbound and bound fractions of 100 µg of 1,500 nt-long m1Ψ-modified IVT RNA after 1 cycle of purification using different celluloses (Sigma, C6288; Macherey-Nagel, MN 100 and MN 2100), microcentrifuge spin columns and 1x STE buffer containing 16% (v/v) EtOH were analyzed by dot blotting. Samples of 80 ng, 400 ng and 2,000 ng of RNA were analyzed for dsRNA contaminants using dsRNA-specific J2 antibody. For comparison, the same amount of unpurified RNA (input RNA) was loaded onto the dot blot membrane. Hybridization signals were quantitated by densitometry and values are expressed as percentage of dsRNA removed from unpurified RNA. To monitor RNA integrity 80 ng of the RNAs were loaded onto a 1.4% (w/v) agarose gel and separated by electrophoresis.

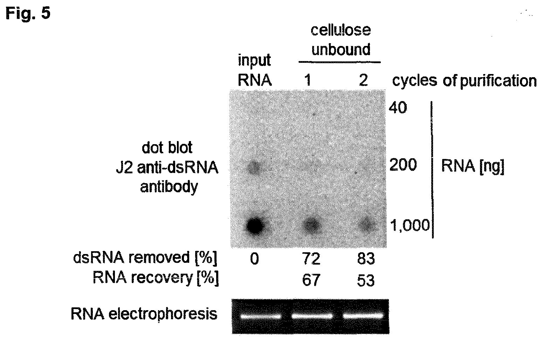

**Figure 5****: The cellulose purification method is scalable.** 5 mg of 1,900 nt-long D1-capped IVT RNA was purified 1x or 2x by cellulose using vacuum-driven filter devices and 1x STE buffer containing 16% (v/v) EtOH. 40 ng, 200 ng and 1,000 ng of cellulose-purified RNA were analyzed for dsRNA contaminants by dot blotting using dsRNA-specific J2 antibody. For comparison, the same amounts of unpurified RNA (input RNA) were loaded onto the dot blot membrane. Hybridization signals were quantitated by densitometry and values are expressed as percentage of dsRNA removed from unpurified RNA. To control RNA integrity 80 ng of the RNAs were loaded onto a 1.4% (w/v) agarose gel and separated by electrophoresis. The RNA recovery rates for both samples are indicated.

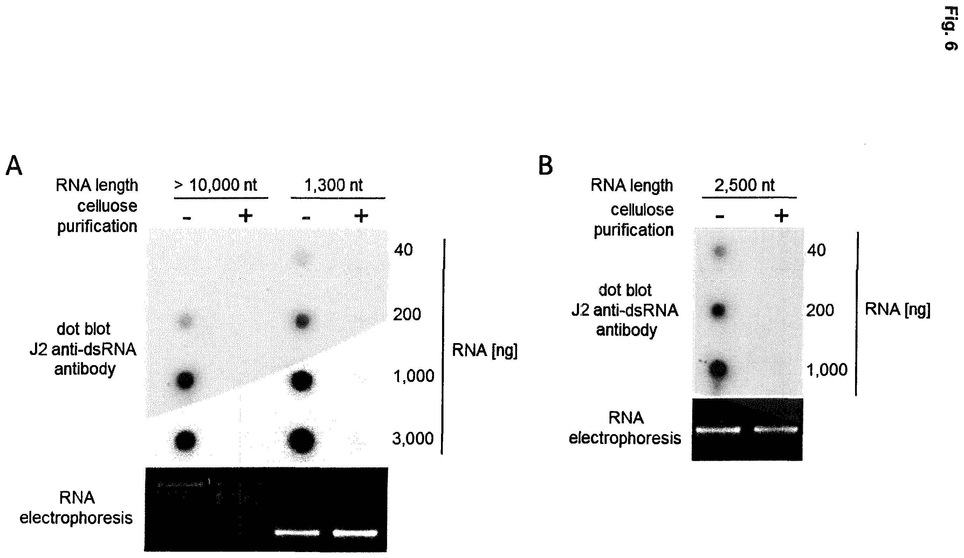

**Figure 6****: Purification of IVT RNA with different length using a "positive" purification procedure.** 400 µg of >10,000 nt-long D1-capped IVT RNA, 1,300 nt-long D2-capped IVT RNA (A) and 2,500 nt-long IVT RNA (uncapped) (B) were used for 2 cycles of cellulose purification. None of the RNAs contained nucleoside modifications. During the first cycle the RNAs were completely bound to cellulose using 1x STE containing 40% (v/v) EtOH prior to elution with 16% (v/v) containing buffer and transfer to a second microcentrifuge column containing a cellulose material ("positive" purification). The indicated amounts of the purified RNAs were analyzed for dsRNA contaminants by dot blotting using dsRNA-specific J2 antibody. For comparison, the same amounts of unpurified RNAs were loaded onto the dot blot membranes. To monitor RNA integrity 80 ng of the RNAs were loaded onto 1.4% (w/v) agarose gels and separated by electrophoresis.

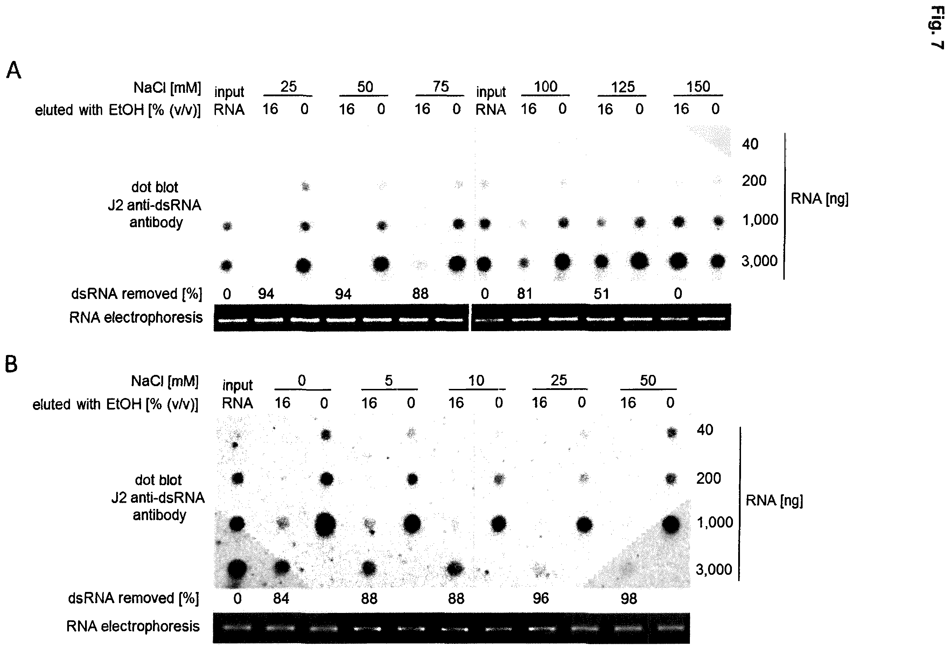

**Figure 7****: Purification of IVT RNA using buffers with different ionic strength.** 250 µg (A) or 160 µg (B) of 1,300 nt-long m1Ψ-modified IVT RNA was cellulose-purified using 1x STE buffers containing 25-150 mM NaCl (A) or 0-50 mM NaCl (B). Prior to elution with corresponding buffers containing 16% (v/v) EtOH followed by elution with 0% (v/v) EtOH buffers the RNA was completely bound to cellulose in the presence of 40% (v/v) EtOH. 40 ng, 200 ng, 1,000 ng and 3,000 ng of the eluted RNAs were analyzed for dsRNA contaminants by dot blotting using dsRNA-specific J2 antibody. Due to low recovery only 40 ng, 200 ng and 1,000 ng of the RNA eluted with 0% (v/v) EtOH could be loaded for dot blot analysis in (B). For comparison, the same amounts of unpurified RNA (input RNA) were loaded onto the dot blot membranes. Hybridization signals were quantitated by densitometry and values are expressed as percentage of dsRNA removed from unpurified RNA. To monitor RNA integrity 80 ng of the RNAs were loaded onto 1.4% (w/v) agarose gels and separated by electrophoresis.

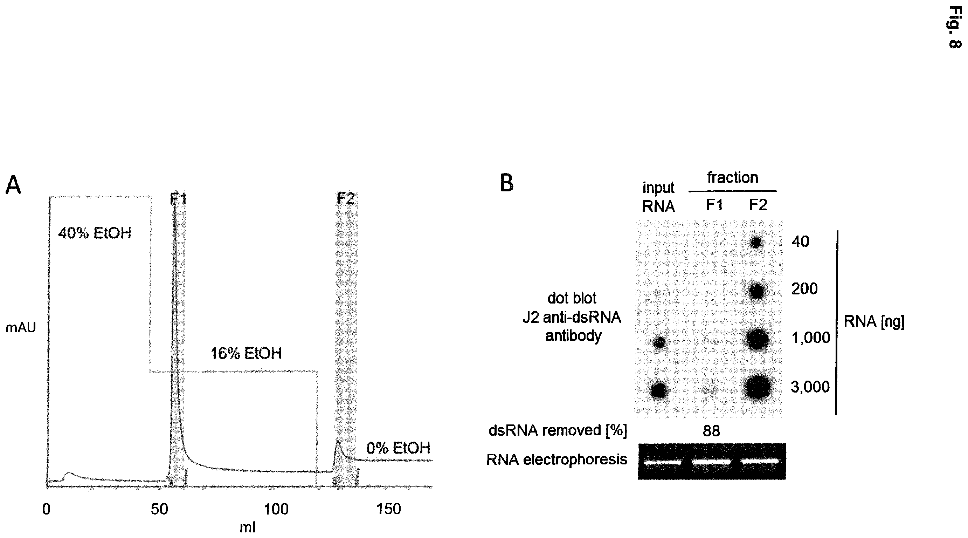

**Figure 8****: Cellulose purification of IVT RNA by FPLC.** (A) An FPLC-chromatogram of 500 µg of 1,300 nt-long m1Ψ-modified IVT RNA is shown, wherein the RNA was loaded onto a XK 16/20 column packed with 4 g of a cellulose material. Binding was performed with 1x STE containing 40% (v/v) EtOH, ssRNA and dsRNA contaminants were eluted by decreasing the EtOH concentration of the running buffer to 16% (v/v) and 0% (v/v), respectively. The fractions indicated by a grey box in the chromatogram were collected (F1: 16% EtOH eluate, F2: 0% EtOH eluate). (B) 40 ng, 200 ng, 1,000 ng and 3,000 ng of the RNAs recovered from fractions F1 and F2 were analyzed for dsRNA contaminants by dot blotting using dsRNA-specific J2 antibody. For comparison, the same amounts of unpurified RNA (input RNA) were loaded onto the dot blot membrane. Hybridization signals were quantitated by densitometry and values are expressed as percentage of dsRNA removed from unpurified RNA. To monitor RNA integrity 80 ng of the RNAs were loaded onto a 1.4% (w/v) agarose gel and separated by electrophoresis.

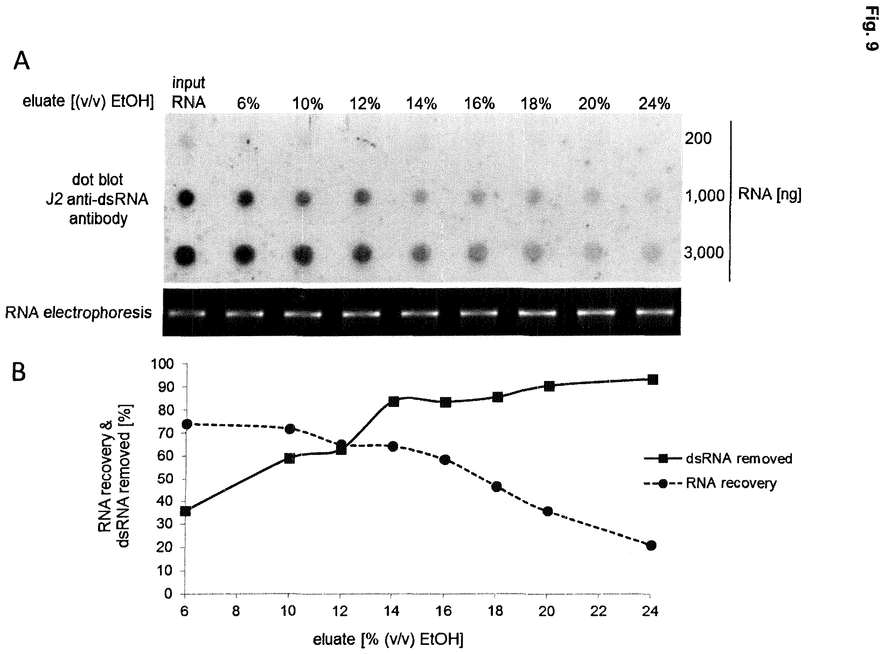

**Figure 9****: Purification of IVT RNA using different EtOH concentrations for ssRNA elution.** 200 µg of 1,500 nt-long D1-capped IVT RNA was cellulose-purified using 1x STE buffer containing 6%, 10%, 12%, 14%, 16, 18%, 20% or 24% (v/v) EtOH to elute ssRNA. Prior to elution the RNA was completely bound to cellulose in the presence of 40% (v/v) EtOH. 200 ng, 1,000 ng and 3,000 ng of the eluted ssRNAs were analyzed for dsRNA contaminants by dot blotting using dsRNA-specific J2 antibody (A). For comparison, the same amounts of unpurified RNA (input RNA) were loaded onto the dot blot membrane. To monitor RNA integrity 80 ng of the RNAs were loaded onto a 1.4% (w/v) agarose gel and separated by electrophoresis. (B) Hybridization signals were quantitated by densitometry and values (expressed as percentage of dsRNA removed from unpurified RNA) were plotted against the EtOH concentrations used for elution (solid line) and compared to the rates of RNA recovered from individual eluates (dashed line).

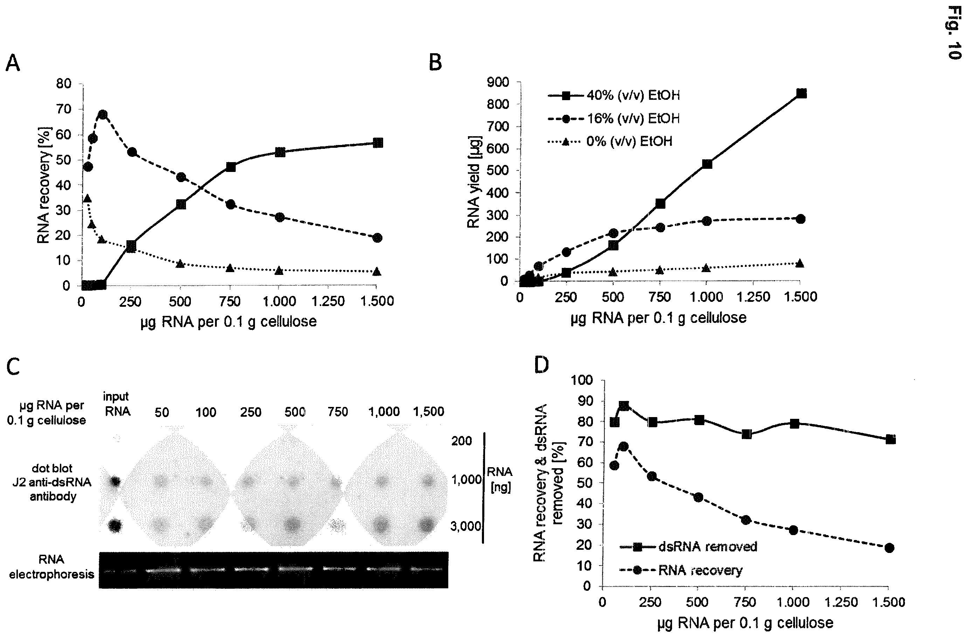

**Figure 10****: Determination of the RNA binding capacity of cellulose.** 0.1 g cellulose (Sigma, C6288) was incubated with 25 µg, 50 µg, 100 µg, 250 µg, 500 µg, 750 µg, 1,000 µg or 1,500 µg of 1,500 nt-long D1-capped IVT RNA in 500 µl of 1x STE containing 40% (v/v) EtOH in a microcentrifuge column. After separation of the unbound RNA by centrifugation the cellulose-bound RNA was eluted stepwise, first with 1x STE containing 16% (v/v) EtOH and finally with H₂O (0% (v/v) EtOH). After precipitation the amounts of RNA recovered from the flow through (A, B; 40% (v/v) EtOH, solid line), the 16% (v/v) EtOH eluate (A, B; dashed line) and the 0% (v/v) EtOH eluate (A, B; dotted line) were determined by spectrophotometry and plotted against the total amount of RNA used for purification. Values are presented as recovery rate relative to the total amount of RNA used (A) or as the total yield of recovered RNA (B). 200 ng, 1,000 ng and 3,000 ng of RNA recovered from the 16% (v/v) eluates was analyzed for dsRNA contaminants by dot blotting using dsRNA-specific J2 antibody (C). For comparison, the same amounts of unpurified RNA (input RNA) were loaded onto the dot blot membrane. To monitor RNA integrity 80 ng of these RNAs were loaded onto a 1.4% (w/v) agarose gel and separated by electrophoresis. Hybridization signals were quantitated by densitometry and values (expressed as percentage of dsRNA removed from unpurified RNA) were plotted against the total amount of RNA used for purification (D; solid line) and compared to the rates of RNA recovered from individual 16% (v/v) EtOH eluates (D; dashed line).

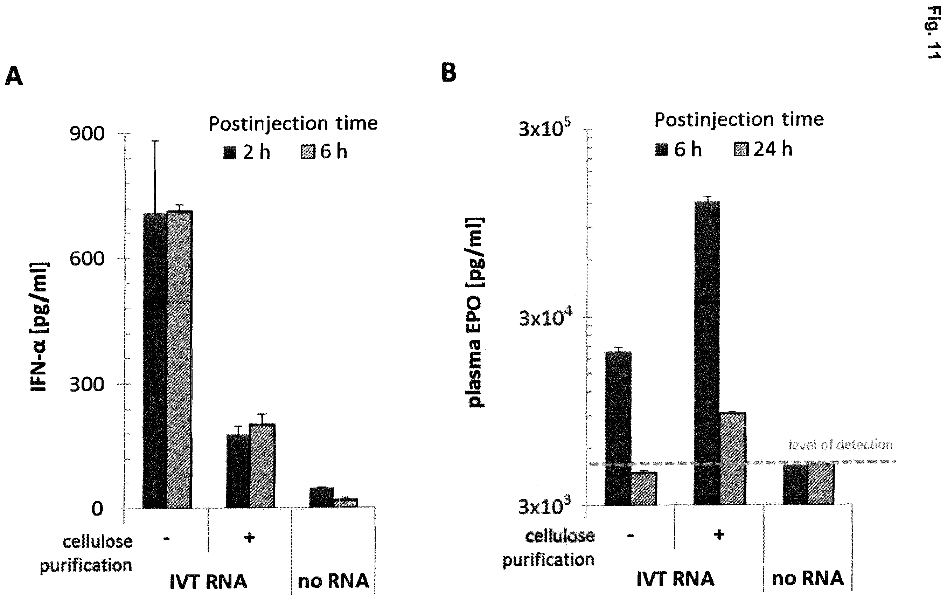

**Figure 11****: Impact of cellulose purification of IVT RNA on its translatability and immunogenicity.** D1-capped IVT RNA (200 µg) encoding murine erythropoietin (EPO) was either left unpurified or was purified by a 2-step procedure using 2 spin columns each filled with a cellulose material: 1^{st} column: positive purification of the IVT RNA (i.e., binding of dsRNA and ssRNA using 1x STE buffer containing 40% (v/v) EtOH; elution of ssRNA using 1x STE buffer containing 16% (v/v) EtOH); 2^{nd} column: negative purification of the eluate from the 1^{st} column (i.e., the eluate containing ssRNA and obtained from the 1^{st} column by using 1x STE buffer containing 16% EtOH)). The flow through obtained from the 2^{nd} column was precipitated with isopropanol/sodium acetate and redissolved in H₂O. Following formulation with TransIT (Mirus Bio) the IVT RNAs were injected intraperitoneally into mice (n=4) at a dose of 3 µg RNA/animal. Blood was withdrawn at 2, 6 and 24 h postinjection and plasma samples were collected. Control mice were injected with TransIT only. Levels of murine interferon alpha (A) and murine EPO (B) were measured using specific ELISA assays (murine interferon alpha-specific ELISA (eBioscience); murine EPO-specific DuoSet ELISA Development kit (R&D)).

### DETAILED DESCRIPTION OF THE PRESENT INVENTION

Although the present invention is further described in more detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described in more detail. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise. For example, if in a preferred embodiment ssRNA comprises a poly(A)-tail consisting of 120 nucleotides and in another preferred embodiment the ssRNA molecule comprises a 5'-cap analog, then in a preferred embodiment, the ssRNA comprises the poly(A)-tail consisting of 120 nucleotides and the 5'-cap analog. Likewise, if in a preferred embodiment the EtOH concentration in the first buffer is 14 to 16% (v/v) and in another preferred embodiment the concentration of a chelating agent in the first buffer is 15 to 40 mM, then in a preferred embodiment, the first buffer comprises EtOH in a concentration of 14 to 16% (v/v) and the chelating agent in a concentration of 15 to 40 mM.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The term "consisting essentially of means excluding other members, integers or steps of any essential significance. The term "comprising" encompasses the term "consisting essentially of' which, in turn, encompasses the term "consisting of'. Thus, at each occurrence in the present application, the term "comprising" may be replaced with the term "consisting essentially of" or "consisting of'. Likewise, at each occurrence in the present application, the term "consisting essentially of' may be replaced with the term "consisting of'.

The terms "a", "an" and "the" and similar references used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by the context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Nothing herein is to be construed as an admission that the invention is not entitled to antedate such disclosure by virtue of prior invention.

The expression "conditions which allow binding of dsRNA to cellulose material" as used herein means conditions which favor (e.g., enhance) the attachment (preferably the non-covalent attachment or adsorption) of the dsRNA to the cellulose material, inhibit the release of dsRNA bound to the cellulose material from the cellulose material, and/or reduce the amount of free dsRNA (i.e., dsRNA which is not bound to the cellulose material). These conditions may be such that they allow or not allow the binding of RNAs other than dsRNA (e.g., ssRNA) to the cellulose material. Thus, in one embodiment, the expression "conditions which allow binding of dsRNA to cellulose material" are "conditions which allow binding of dsRNA to the cellulose material and do not allow binding of ssRNA to the cellulose material". In this embodiment, the conditions (i) favor (e.g., enhance) the attachment (preferably the non-covalent attachment or adsorption) of dsRNA to the cellulose material, inhibit the release of dsRNA bound to the cellulose material from the cellulose material, and/or reduce the amount of free dsRNA (i.e., the amount of dsRNA not bound to the cellulose material), and (ii) favor (e.g., enhance) the unbound state of ssRNA (i.e., the state of ssRNA not attached or adsorbed to the cellulose material), reduce the amount of ssRNA attached (preferably, non-covalently attached or adsorbed) to the cellulose material, and/or inhibit the attachment (preferably, non-covalent attachment or adsorption) of ssRNA to the cellulose material. In an alternative embodiment, the expression "conditions which allow binding of dsRNA to cellulose material" are "conditions which allow binding of dsRNA and ssRNA to the cellulose material". In this alternative embodiment, the conditions favor (e.g., enhance) the attachment (preferably the non-covalent attachment or adsorption) of the dsRNA and ssRNA to the cellulose material, inhibit the release of dsRNA and ssRNA bound to the cellulose material from the cellulose material, and/or reduce the amount of free dsRNA and ssRNA (i.e., the amount of dsRNA and ssRNA not bound to the cellulose material).

The above conditions which allow or do not allow binding of dsRNA/ssRNA to cellulose material can be controlled by the composition of the medium (such as the composition of a buffer) in which the RNA preparation comprising dsRNA/ssRNA is solved or which is added to the cellulose material. In this respect, "composition" means the type and amount of the components contained in the medium (e.g., in the buffer).

Thus, in one embodiment, the "conditions which allow binding of dsRNA to the cellulose material and do not allow binding of ssRNA to the cellulose material" can be achieved by a first medium (e.g., a first buffer) comprising water, ethanol and a salt in a concentration which allows binding of dsRNA to the cellulose material and which does not allow binding of ssRNA to the cellulose material. Therefore, in order to meet these conditions, in step (ii) the RNA preparation can be provided as a liquid comprising ssRNA and the first medium (e.g., the first buffer); the cellulose material can be provided as a suspension in the first medium (e.g., the first buffer) (e.g., as a washed cellulose material, wherein the first medium (e.g., the first buffer) has been used as washing solution); the RNA preparation can be provided as a liquid comprising ssRNA and the first medium (e.g., the first buffer) and the cellulose material can be provided as a suspension in the first medium (e.g., the first buffer); or the RNA preparation can be provided as a liquid comprising ssRNA and the first medium (e.g., the first buffer) and the cellulose material can be provided as washed cellulose material (wherein the first medium (e.g., the first buffer) has been used as washing solution), either in dry form or as suspension in the first medium (e.g., the first buffer).

The expression "in a concentration which allows binding of dsRNA to the cellulose material and which does not allow binding of ssRNA to the cellulose material" means that the concentration of the components (in particular water, ethanol and a salt) in the first medium (e.g., in the first buffer) is sufficient to (i) favor (e.g., enhance) the attachment (preferably the non-covalent attachment or adsorption) of dsRNA to the cellulose material, inhibit the release of dsRNA bound to the cellulose material from the cellulose material into the first medium (e.g., into the first buffer), and/or reduce the amount of free dsRNA (i.e., the amount of dsRNA not bound to the cellulose material) in the first medium (e.g., in the first buffer), and (ii) favor (e.g., enhance) the unbound state of ssRNA (i.e., the state of ssRNA not attached or adsorbed to the cellulose material), reduce the amount of ssRNA attached (preferably, non-covalently attached or adsorbed) to the cellulose material, and/or inhibit the attachment (preferably, non-covalent attachment or adsorption) of ssRNA to the cellulose material.

Furthermore, in one embodiment, the "conditions which allow binding of dsRNA and ssRNA to cellulose material" can be achieved by a second medium (e.g., a second buffer) comprising water, ethanol and a salt in a concentration which allows binding of dsRNA and ssRNA to the cellulose material. Therefore, in order to meet these conditions, in step (ii) the RNA preparation can be provided as a liquid comprising ssRNA and the second medium (e.g., the second buffer); the cellulose material can be provided as a suspension in the second medium (e.g., the second buffer) (e.g., as a washed cellulose material, wherein the second medium (e.g., the second buffer) has been used as washing solution); the RNA preparation can be provided as a liquid comprising ssRNA and the second medium (e.g., the second buffer) and the cellulose material can be provided as a suspension in the second medium (e.g., the second buffer); or the RNA preparation can be provided as a liquid comprising ssRNA and the second medium (e.g., the second buffer) and the cellulose material can be provided as washed cellulose material (wherein the second medium (e.g., the second buffer) has been used as washing solution), either in dry form or as suspension in the second medium (e.g., the second buffer).

The expression "in a concentration which allows binding of dsRNA and ssRNA to the cellulose material" means that the concentration of the components (in particular water, ethanol and a salt) in the second medium (e.g., in the second buffer) is sufficient to favor (e.g., enhance) the attachment (preferably the non-covalent attachment or adsorption) of the dsRNA and ssRNA to the cellulose material, inhibit the release of dsRNA and ssRNA bound to the cellulose material from the cellulose material into the second medium (e.g., into the second buffer), and/or reduce the amount of free dsRNA and ssRNA (i.e., the amount of dsRNA and ssRNA not bound to the cellulose material) in the second medium (e.g., into the second buffer).

The present inventors have surprisingly found that dsRNA, but not ssRNA, is selectively bound to a cellulose material in the presence of ethanol in a concentration of 14 to 20% (v/v). Thus, the "conditions which allow binding of dsRNA to the cellulose material and do not allow binding of ssRNA to the cellulose material" is achieved by the first medium (i. e., the first buffer) as specified above which contains ethanol in a concentration of 14 to 20% (v/v), preferably 14 to 19% (v/v), more preferably 14 to 18% (v/v), such as 14 to 17% (v/v), 14 to 16% (v/v), 15 to 19% (v/v), 15 to 18% (v/v), 15 to 17% (v/v), 16 to 19% (v/v), or 16 to 18% (v/v). The first medium (e.g., the first buffer) comprises, in addition to ethanol in the above disclosed ranges, the salt in a concentration of 15 to 70 mM and TRIS as a buffering substance. In one embodiment, the first medium (e.g., the first buffer) comprises, in addition to ethanol in the above disclosed ranges, the salt in a concentration of 20 to 60 mM such as 25 to 50 mM or 30 to 50 mM. The salt in the first medium (e.g., the first buffer) is preferably sodium chloride. However, based on the information and data provided in the present application, the skilled person can easily determine other salts and their concentrations which are suitable for the first medium (e.g., the first buffer) to be used in the methods of the present invention. Further optional components of the first medium (e.g., the first buffer) comprise a chelating agent (preferably EDTA or nitrilotriacetic acid, more preferably EDTA). In one embodiment, the concentration of the buffering substance in the first medium (e.g., the first buffer) is 5 to 40 mM, preferably 6 to 30 mM, such as 8 to 20 mM or 10 to 15 mM. In one embodiment, the pH of the first medium (e.g., the first buffer) is 6.5 to 8.0, preferably 6.7 to 7.8, such as 6.8 to 7.2 (e.g., when TRIS is the buffering substance). In one embodiment, the concentration of the chelating agent in the first medium (e.g., the first buffer) is 10 to 50 mM, preferably 15 to 40 mM such as 20 to 30 mM.

In one embodiment, the first medium (e.g., the first buffer) comprises water, ethanol, TRIS and EDTA (such as water, ethanol, the salt (preferably sodium chloride), TRIS and EDTA), preferably in the concentrations specified above for the first medium (e.g., the first buffer). However, based on the information and data provided in the present application, the skilled person can easily determine chelating agents other than EDTA and/or salts other than sodium chloride as well as their concentrations which are suitable for the first medium (e.g., the first buffer) to be used in the methods of the present invention. For example, in one embodiment, the first medium (e.g., the first buffer) comprises, in addition to ethanol in the above disclosed ranges (i.e., 14 to 20% (v/v), etc.), TRIS in an amount of 5 to 40 mM and the salt (preferably sodium chloride) in an amount of 15 to 70 mM.

In one embodiment, the "conditions which allow binding of dsRNA and ssRNA to cellulose material" can be achieved by the second medium (e.g., the second buffer) as specified above which contains ethanol in a concentration of at least 35% (v/v), preferably at least 36% (v/v), at least 37% (v/v), at least 38% (v/v), at least 39% (v/v), at least 40% (v/v), such as 35 to 45% (v/v), 36 to 45% (v/v), 37 to 45% (v/v), 38 to 45% (v/v), 38 to 42% (v/v), or 39 to 41% (v/v). In one embodiment, the second medium (e.g., the second buffer) comprises, in addition to ethanol in the above disclosed ranges (i.e., at least 35% (v/v), at least 36% (v/v), at least 37% (v/v), at least 38% (v/v), etc.), the salt in a concentration of 15 to 70 mM, preferably 20 to 60 mM such as 25 to 50 mM or 30 to 50 mM. The salt in the second medium (e.g., the second buffer) is preferably sodium chloride. However, based on the information and data provided in the present application, the skilled person can easily determine other salts and their concentrations which are suitable for the second medium (e.g., the second buffer) to be used in the methods of the present invention. Further optional components of the second medium (e.g., the second buffer) comprise a buffering substance (preferably TRIS or HEPES, more preferably TRIS), and/or a chelating agent (preferably EDTA or nitrilotriacetic acid, more preferably EDTA). In one embodiment, the concentration of the buffering substance in the second medium (e.g., the second buffer) is 5 to 40 mM, preferably 6 to 30 mM, such as 8 to 20 mM or 10 to 15 mM. In one embodiment, the pH of the second medium (e.g., the second buffer) is 6.5 to 8.0, preferably 6.7 to 7.8, such as 6.8 to 7.2 (e.g., when TRIS is the buffering substance) or 7.3 to 7.7 (e.g., when HEPES is the buffering substance). In one embodiment, the concentration of the chelating agent in the second medium (e.g., the second buffer) is 10 to 50 mM, preferably 15 to 40 mM such as 20 to 30 mM.

In one embodiment, the second medium (e.g., the second buffer) comprises water, ethanol, TRIS and EDTA (such as water, ethanol, the salt (preferably sodium chloride), TRIS and EDTA), preferably in the concentrations specified above for the second medium (e.g., the second buffer). However, based on the information and data provided in the present application, the skilled person can easily determine buffering substances other than TRIS and/or chelating agents other than EDTA and/or salts other than sodium chloride as well as their concentrations which are suitable for the second medium (e.g., the second buffer) to be used in the methods of the present invention. In one embodiment, the second medium (e.g., the second buffer) comprises, in addition to ethanol in the above disclosed ranges (i.e., at least 35% (v/v), at least 36% (v/v), at least 37% (v/v), at least 38% (v/v), etc.), TRIS in an amount of 5 to 40 mM and the salt (preferably sodium chloride) in an amount of 15 to 70 mM. In another embodiment, the second medium (e.g., the second buffer) comprises, in addition to ethanol in the above disclosed ranges (i.e., at least 35% (v/v), at least 36% (v/v), at least 37% (v/v), at least 38% (v/v), etc.), HEPES in an amount of 5 to 40 mM and the salt (preferably sodium chloride) in an amount of 100 to 150 mM (e.g., 110 to 140 mM or 120 to 130 mM).

In one embodiment, the first and second media (i.e., the first and second buffers) differ not only in the concentration of ethanol but also in the type and/or concentration of one or more of the other components (such as the salt, the optional buffering substance, and/or the optional chelating agent). In one preferred embodiment, the first and second media (i.e., the first and second buffers) have the same composition (i.e., with respect to the type and concentration of the components other than water, such as the salt, the optional buffering substance and the optional chelating agent) with the exception of the concentration of ethanol.

The expression "separating the ssRNA from the cellulose material under conditions which allow binding of dsRNA to the cellulose material" as used herein means that the phase comprising ssRNA (said phase being preferably liquid) is to be isolated from the cellulose material to which dsRNA is bound. Such isolation/separation can be accomplished in several ways known to the skilled person, e.g., by selectively removing only the cellulose material to which dsRNA is bound (e.g., by using, as the cellulose material, a cellulose which is covalently coupled to magnetic beads and using a magnet) or collecting only the phase comprising ssRNA (e.g., by using a pipette).

For example, in one embodiment, the mixture of the RNA preparation, the cellulose material, and the first buffer is provided in a tube (in this embodiment, it is preferred that (α) the RNA preparation is provided as a liquid comprising ssRNA and the first buffer and/or (β) the cellulose material is provided as washed cellulose material (wherein the first buffer has been used as washing solution), either in dry form or as suspension in the first medium (e.g., the first buffer), and/or (γ) the RNA preparation, the cellulose material, and the first buffer are mixed under shaking and/or stirring (preferably for at least 5 min, more preferably for at least 10 min, such as for 5 to 30 min or 10 to 20 min); it is most preferred that (α) the RNA preparation is provided as a liquid comprising ssRNA and the first buffer, (β) the cellulose material is provided as washed cellulose material (wherein the first buffer has been used as washing solution), either in dry form or as suspension in the first medium (e.g., the first buffer), and (γ) the RNA preparation, the cellulose material, and the first buffer are mixed under shaking and/or stirring, e.g., for 5 to 30 min or 10 to 20 min). In this embodiment, it is preferred that step (iii) comprises (1) applying gravity or centrifugal force (e.g., 10,000 x g to 15,000 x g for 1 to 5 min) to the tube such that the liquid and solid phases are separated (preferably completely separated); and (2) either collecting the supernatant comprising ssRNA (e.g., by using a pipette) or removing the cellulose material (e.g., by using, as the cellulose material, a cellulose which is covalently coupled to magnetic beads and using a magnet). Steps (ii) and (iii) may be repeated once or two or more times (such as once, twice or three times). If steps (ii) and (iii) are repeated, the ssRNA preparation obtained after step (iii) of one cycle is used as RNA preparation in step (ii) of the next (i.e., immediately following) cycle and in each cycle fresh cellulose material (preferably fresh washed cellulose material) is used.

In an alternative embodiment, the mixture of the RNA preparation, the cellulose material, and the first buffer is provided in a spin column or filter device (in this embodiment, it is preferred that (α) the RNA preparation is provided as a liquid comprising ssRNA and the first buffer and/or (β) the cellulose material is provided as washed cellulose material (wherein the first buffer has been used as washing solution), either in dry form or as suspension in the first medium (e.g., the first buffer), and/or (γ) the RNA preparation, the cellulose material, and the first buffer are mixed under shaking and/or stirring (preferably for at least 5 min, more preferably for at least 10 min, such as for 5 to 30 min or 10 to 20 min); it is most preferred that (α) the RNA preparation is provided as a liquid comprising ssRNA and the first buffer, (β) the cellulose material is provided as washed cellulose material (wherein the first buffer has been used as washing solution), either in dry form or as suspension in the first medium (e.g., the first buffer), and (γ) the RNA preparation, the cellulose material, and the first buffer are mixed under shaking and/or stirring, e.g., for 5 to 30 min or 10 to 20 min). In this embodiment, it is preferred that step (iii) comprises (1') applying gravity, centrifugal force (e.g., 10,000 x g to 15,000 x g for 1 to 5 min), pressure (e.g., 1000 hPa to 3000 hPa), or vacuum (e.g., 100 hPa to 900 hPa, such as 200 hPa to 800 hPa) to the spin column or filter device such that the liquid and solid phases are separated (preferably completely separated); and (2') collecting the flow through comprising ssRNA. Steps (ii) and (iii) may be repeated once or two or more times (such as once, twice or three times). If steps (ii) and (iii) are repeated, the ssRNA preparation obtained after step (iii) of one cycle is used as RNA preparation in step (ii) of the next (i.e., immediately following) cycle and in each cycle fresh cellulose material (preferably fresh washed cellulose material) is used.

The expression "separating the cellulose material to which dsRNA and ssRNA are bound from the remainder" as used herein means that the solid phase (i.e., cellulose material) to which dsRNA and ssRNA are attached (preferably non-covalently attached or adsorbed) is to be isolated from the other phase (said other phase being preferably liquid). Such isolation/separation can be accomplished in several ways known to the skilled person, e.g., by selectively collecting only the cellulose material to which dsRNA and ssRNA are bound (e.g., by using, as the cellulose material, a cellulose which is covalently coupled to magnetic beads and using a magnet) or selectively removing only the other phase (e.g., by using a pipette).

For example, in one embodiment, the mixture of the RNA preparation, the cellulose material, and the second buffer is provided in a tube (in this embodiment, it is preferred that (α') the RNA preparation is provided as a liquid comprising ssRNA and the second buffer and/or (β') the cellulose material is provided as washed cellulose material (wherein the second buffer has been used as washing solution), either in dry form or as suspension in the second medium (e.g., the second buffer), and/or (γ') the RNA preparation, the cellulose material, and the second buffer are mixed under shaking and/or stirring (preferably for at least 5 min, more preferably for at least 10 min, such as for 5 to 30 min or 10 to 20 min); it is most preferred that (α') the RNA preparation is provided as a liquid comprising ssRNA and the second buffer, (β') the cellulose material is provided as washed cellulose material (wherein the second buffer has been used as washing solution), either in dry form or as suspension in the second medium (e.g., the second buffer), and (γ') the RNA preparation, the cellulose material, and the second buffer are mixed under shaking and/or stirring, e.g., for 5 to 30 min or 10 to 20 min). In this embodiment, it is preferred that step (ii)(2) (i.e., "separating the cellulose material to which dsRNA and ssRNA are bound from the remainder") comprises (2a) applying gravity or centrifugal force (e.g., 10,000 x g to 15,000 x g for 1 to 5 min) to the tube such that the liquid and solid phases are separated (preferably completely separated); and (2b) either removing the supernatant (e.g., by using a pipette) or collecting the cellulose material to which dsRNA and ssRNA are bound (e.g., by using, as the cellulose material, a cellulose which is covalently coupled to magnetic beads and using a magnet). Step (ii) may further comprise (3) adding an aliquot of the second buffer (preferably the aliquot being 0.5 to 3 times (such as 1 to 2 times) the volume of the cellulose material) to the cellulose material to which dsRNA and ssRNA are bound; (4) incubating the resulting mixture under shaking and/or stirring (preferably for 5 to 20 min or 10 to 15 min); and (5) separating the cellulose material to which dsRNA and ssRNA are bound from the liquid phase (preferably the separation is conducted in the same manner as defined in steps (2a) and (2b), above); and optionally (6) repeating steps (3) to (5) once or two or more times (such as once, twice or three times). Step (iii) comprises (1) mixing the cellulose material to which dsRNA and ssRNA are bound with the first buffer as specified above (i.e., having an EtOH concentration of 14 to 20% (v/v), preferably 14 to 16% (v/v)) under shaking and/or stirring (preferably for at least 5 min, more preferably for at least 10 min, such as for 5 to 30 min or 10 to 20 min); and (2) separating the liquid phase comprising ssRNA from the cellulose material (preferably the step of separating the liquid phase comprising ssRNA from the cellulose material is conducted as specified above, e.g., by applying gravity or centrifugal force (e.g., 10,000 x g to 15,000x g for 1 to 5 min) to the tube such that the liquid and solid phases are separated (preferably completely separated); and (2) either collecting the supernatant comprising ssRNA (e.g., by using a pipette) or removing the cellulose material (e.g., by using, as the cellulose material, a cellulose which is covalently coupled to magnetic beads and using a magnet). Steps (ii) and (iii) may be repeated once or two or more times (such as once, twice or three times). If steps (ii) and (iii) are repeated, the ssRNA preparation obtained after step (iii) of one cycle is used as RNA preparation in step (ii) of the next (i.e., immediately following) cycle and in each cycle fresh cellulose material (preferably fresh washed cellulose material) is used.

In an alternative embodiment, the mixture of the RNA preparation, the cellulose material, and the second buffer is provided in a spin column or filter device (in this embodiment, it is preferred that (α') the RNA preparation is provided as a liquid comprising ssRNA and the second buffer and/or (β') the cellulose material is provided as washed cellulose material (wherein the second buffer has been used as washing solution), either in dry form or as suspension in the second medium (e.g., the second buffer), and/or (γ') the RNA preparation, the cellulose material, and the second buffer are mixed under shaking and/or stirring (preferably for at least 5 min, more preferably for at least 10 min, such as for 5 to 30 min or 10 to 20 min); it is most preferred that (α') the RNA preparation is provided as a liquid comprising ssRNA and the second buffer, (β') the cellulose material is provided as washed cellulose material (wherein the second buffer has been used as washing solution), either in dry form or as suspension in the second medium (e.g., the second buffer), and (γ') the RNA preparation, the cellulose material, and the second buffer are mixed under shaking and/or stirring, e.g., for 5 to 30 min or 10 to 20 min). In this embodiment, it is preferred that step (ii)(2) (i.e., "separating the cellulose material to which dsRNA and ssRNA are bound from the remainder") comprises (2a') applying gravity, centrifugal force (e.g., 10,000 x g to 15,000 x g for 1 to 5 min), pressure (e.g., 1000 hPa to 3000 hPa), or vacuum (e.g., 100 hPa to 900 hPa, such as 200 hPa to 800 hPa) to the spin column or filter device such that the liquid and solid phases are separated (preferably completely separated; and (2b') discarding the flow through. Step (ii) may further comprise (3) adding an aliquot of the second buffer (preferably the aliquot being 0.5 to 3 times (such as 1 to 2 times) the volume of the cellulose material) to the cellulose material to which dsRNA and ssRNA are bound; (4) incubating the resulting mixture under shaking and/or stirring (preferably for 5 to 20 min or 10 to 15 min); and (5) separating the cellulose material to which dsRNA and ssRNA are bound from the liquid phase (preferably the separation is conducted in the same manner as defined in steps (2a') and (2b'), above); and optionally (6) repeating steps (3) to (5) once or two or more times (such as once, twice or three times). Step (iii) comprises (1) mixing the cellulose material to which dsRNA and ssRNA are bound with the first buffer as specified above (i.e., having an EtOH concentration of 14 to 20% (v/v), preferably 14 to 16% (v/v)) under shaking and/or stirring (preferably for at least 5 min, more preferably for at least 10 min, such as for 5 to 30 min or 10 to 20 min); and (2) separating the liquid phase comprising ssRNA from the cellulose material (preferably the step of separating the liquid phase comprising ssRNA from the cellulose material is conducted as specified above, e.g., by applying gravity, centrifugal force (e.g., 10,000 x g to 15,000 x g for 1 to 5 min), pressure (e.g., 1000 hPa to 3000 hPa), or vacuum (e.g., 100 hPa to 900 hPa, such as 200 hPa to 800 hPa) to the spin column or filter device such that the liquid and solid phases are separated (preferably completely separated); and collecting the flow through comprising ssRNA. Steps (ii) and (iii) may be repeated once or two or more times (such as once, twice or three times). If steps (ii) and (iii) are repeated, the ssRNA preparation obtained after step (iii) of one cycle is used as RNA preparation in step (ii) of the next (i.e., immediately following) cycle and in each cycle fresh cellulose material (preferably fresh washed cellulose material) is used.

In one embodiment of the positive purification procedure, the cellulose material is provided in a column (in this embodiment, it is preferred that the cellulose material is provided as washed cellulose material, wherein a second buffer as specified above (i.e., having an EtOH concentration of at least 35% (v/v), at least 36% (v/v), at least 37% (v/v), at least 38% (v/v), etc.) has been used as washing solution). In this embodiment, it is preferred that before the RNA preparation is loaded onto the column in step (ii), the column comprising the cellulose material is equilibrated (i.e., washed) with the second buffer as specified above (e.g., with an aliquot of said second buffer). Thereafter, the RNA preparation (which is preferably provided as a liquid comprising ssRNA and said second buffer) is loaded onto the column (preferably by injection) and preferably the column is washed with said second buffer (e.g., with a further aliquot of said second buffer, preferably 0.5 to 2 times the volume of the cellulose material contained in the column). This washing step is preferred because it can wash away contaminants other than RNA (such contaminants particularly include the starting materials used for generating IVT RNA (which is optionally modified) and their degradation products, e.g., a DNA template; an RNA polymerase (such as T7, T3 or SP6); monoribonucleotides in unmodified form (e.g., rATP, rGTP, rCTP, rUTP, and their analogs having only one or two phosphate groups) or modified form (e.g., r(1mΨ) TP or rΨTP and their analogs having only one or two phosphate groups); pyrophosphate; a cap reagent (i.e., a reagent to introduce a 5'-cap or 5'-cap analog); and additives used for generating IVT RNA (e.g., buffering agents, salts, antioxidizing agents, polyamines (such as spermidine)). Step (iii) is preferably conducted by using a first buffer as specified above (i.e., having an EtOH concentration of 14 to 20% (v/v), preferably 14 to 16% (v/v)) as eluent, thereby releasing the ssRNA from the cellulose material. The compounds (in particular ssRNA, optionally also dsRNA) which are eluted or washed from the column can be detected and/or monitored by using conventional means (e.g., an UV/VIS-detector such as an diode-array-detector), e.g., at a wavelength of 260 nm (for the detection of nucleic acids) and/or 215 nm (for the detection of peptides/proteins) and/or 280 nm (for the detection of peptides/proteins containing aromatic amino acids).

The ssRNA obtained by any of the methods of the present invention (in particular irrespective of whether the "negative" or "positive" purification procedure has been used) may be subjected to further treatments, such as precipitation and/or modification. For example, the ssRNA obtained by the methods of the present invention may be precipitated using conventional methods (e.g., using the "sodium acetate/isopropanol" precipitation method or the "LiCl" precipitation method) resulting in an ssRNA preparation in dried form. The dried ssRNA can be stored (e.g., at -70°C) or can be solved in an appropriate solvent (e.g., water or TE buffer (10 mM TRIS, 1 mM EDTA)) and then stored (e.g., at -70°C) or further used (e.g., for the preparation of a pharmaceutical composition). Alternatively or additionally, the ssRNA can be further modified, e.g., by removing uncapped 5'-triphosphates and/or adding a cap structure, before it is stored (e.g., at -70°C) or used (e.g., for the preparation of a pharmaceutical composition).

As demonstrated in the examples of the present application, the methods of the invention provide several advantages, such as one or more of the following. For example, the methods of the present invention offer a broad spectrum of different purification techniques, including simple centrifugation steps, microcentrifuge spin columns, vacuum-driven filter systems, and FPLC. Compared to HPLC methods, the methods of the present invention are cost effective and simple (no need for complex equipment), avoid toxic substances (such as acetonitrile), and provide ssRNA in a comparatively high purity and yield. Furthermore, because cellulose is a natural product it can be expected that a method of the present invention which is based on cellulose and which is effective in purifying ssRNA (in particular IVT ssRNA) encounters less complexity when transferred into GMP-regulated environments. In addition, it has been demonstrated in the present application that the methods of the present invention can be easily upscaled and are less time consuming than conventional HPLC methods. In this respect, it is noted that conventional HPLC methods (such as those disclosed in Weissman et al., supra) are generally limited by column size and the back pressure issue involved in using large columns. This is not the case for the methods of the present invention. For example, as demonstrated in the present application (cf. Example 5), the purification of 50 to 100 mg IVT RNA can be achieved in less than 2 h when using the methods of the present invention. In contrast, the HPLC column used in Example 3 (Semi-Prep RNASep 100 x 21.1 mm, Transgenomic) with a column volume of 35 ml has a maximum binding capacity of 1 mg IVT RNA. Because a standard purification run using such a HPLC column takes more than 60 min, the purification of 50 mg IVT RNA would take about 50 h compared to only 2 h using the methods of the present invention. Furthermore, the purification of long IVT RNAs using conventional HPLC-based methods causes problems and often leads to (a) high loss of IVT RNA (in particular, when the IVT RNA has a length of at least about 2,700 nt (preferably at least 2,800 nt, at least 2,900 nt, at least 3,000 nt, at least 3,100 nt, at least 3,200 nt, at least 3,300 nt, at least 3,400 nt, more preferably at least 3,500 nt, at least 3,600 nt, at least 3,700 nt, at least 3,800 nt, at least 3,900 nt, at least 4,000 nt, at least 4,100 nt, at least 4,200 nt, at least 4,300 nt, at least 4,400 nt, more preferably at least 4,500 nt, at least 4,600 nt, at least 4,700 nt, at least 4,800 nt, at least 4,900 nt, at least 5,000 nt), because IVT RNAs having such a length do not elute in conventional HPLC-based methods as a defined sharp peak but elute as a broad peak thereby requiring the collection of the eluate (comprising the ssRNA) over prolonged period of time in order to minimize the loss of ssRNA) and/or, more importantly, (b) the degradation of the IVT RNA (in particular, when long IVT RNA (e.g., having a size of least 3,500 nt, such as at least 4,000 nt, at least 4,500 nt, at least 5,000 nt, at least 5,500 nt, at least 6,000 nt, at least 6,500 nt, at least 7,000 nt, at least 7,500 nt, at least 8,000 nt, at least 8,500 nt, at least 9,000 nt, or at least 9,500 nt) is to be purified probably due to shearing during the passage of long RNAs through the tightly packed column material). In contrast, as demonstrated in the present application, using the methods of the present invention for purifying IVT RNAs having a size of about 10,000 nt or greater does not result in the degradation of the RNA. Moreover, it was found that by using the methods of the present invention, it is possible to elute IVT ssRNAs (in particular IVT ssRNAs having a length of at least about 2,700 nt (preferably at least 2,800 nt, at least 2,900 nt, at least 3,000 nt, at least 3,100 nt, at least 3,200 nt, at least 3,300 nt, at least 3,400 nt, more preferably at least 3,500 nt, at least 3,600 nt, at least 3,700 nt, at least 3,800 nt, at least 3,900 nt, at least 4,000 nt, at least 4,100 nt, at least 4,200 nt, at least 4,300 nt, at least 4,400 nt, more preferably at least 4,500 nt, at least 4,600 nt, at least 4,700 nt, at least 4,800 nt, at least 4,900 nt, at least 5,000 nt)) from the cellulose material in a defined sharp peak thereby reducing the amount (i.e., volume) of eluate (comprising the ssRNA) to be collected to a minimum. Finally, the integrity of the purified RNA makes the methods of the present invention superior compared to conventional methods using *E.coli* RNaseIII which often result in a partial degradation of ssRNA, especially long ssRNA, during incubation (probably due to the RNaseIII-catalyzed hydrolysis of double-stranded secondary structures contained in ssRNA).

The term "shaking and/or stirring" as used herein means any action which is suitable to mix (preferably thoroughly mix) a mixture, e.g., a mixture comprising a solid phase (such as a cellulose material) and liquid phase (such as a medium (e.g., a buffer), a washing solution, or an RNA preparation solved in a medium (e.g., a buffer)). Exemplary devices to achieve "shaking and/or stirring" are known to the skilled person and include a shaker, a mixer (e.g., a vortex mixer or a static mixer), a magnetic stirrer (including a stir bar), and a stirring rod, which are available in different sizes depending on the volume of the mixture to be mixed. The shaking and/or stirring of the mixture can be performed for a time sufficient to achieve a thorough mixing, e.g., for a time of at least 1 min (such as at least 2 min, at least 3 min, at least 4 min, at least 5 min, at least 8 min, at least 10 min). The maximum time for shaking and/or stirring of the mixture can be up to 40 min (such as up to 35 min, up to 30 min, up to 28 min, up to 26 min, up to 24 min, up to 22 min, or up to 20 min). Thus, exemplary time ranges for the shaking and/or stirring of the mixture are 5 to 40 min, 5 to 30 min, 5 to 20 min, 10 to 40 min, 10 to 30 min, 10 to 20 min, 15 to 40 min, 15 to 30 min, or 15 to 20 min. Generally, the duration of shaking and/or stirring will depend on factors such as the intended use (e.g., washing of a cellulose material or binding of RNA to a cellulose material) and the amount of solid to be mixed. For example, for washing of a cellulose material the duration of shaking and/or stirring can be in the range of 1 to 15 min, such as 5 to 10 min. For binding of RNA to a cellulose material, the duration of shaking and/or stirring can be in the range of 5 to 20 min (such as 10 to 20 min) when up to 1 g of cellulose material is used, or in the range of 10 to 30 min (such as 15 to 30 min) when more than 1 g of cellulose material is used. Likewise, for realeasing RNA (in particular ssRNA) bound to a cellulose material from the cellulose material, the duration of shaking and/or stirring can be in the range of 5 to 20 min (such as 10 to 20 min) when up to 1 g of cellulose material is used, or in the range of 10 to 30 min (such as 15 to 30 min) when more than 1 g of cellulose material is used.

The term "salt" as used with respect to the first and second media (e.g., the first and second buffers) means any ionic compound which results from the neutralization reaction of an acid and a base. Preferably, the salt (i) is not a buffering substance, (ii) is not a chelating agent, or (iii) is neither a buffering substance nor a chelating agent. Exemplary acids include inorganic acids (such as hydrochloric acid, hydrobromic acid, hydriodic acid, sulfuric acid, nitric acid, phosphoric acid, boric acid, and perchloric acid) and organic acids (e.g., monocarboxylic acids, preferably those having 1 to 5 (such as 1, 2 or 3) carbon atoms, e.g., formic acid, acetic acid, and propionic acid), preferably inorganic acids. Exemplary bases include inorganic bases (such as NH₃, ammonium hydroxide (NH₄OH), and the oxides and hydroxides of metals, preferably the oxides and hydroxides of alkaline, earth, and alkaline earth metals (e.g., the oxides and hydroxides of Li, Na, K, Rb, Be, Mg, Ca, Sr, Al, and Zn)) and organic bases (such as amines, e.g., monoalkyl, dialkyl or trialkylamines), preferably inorganic bases, more preferably the oxides and hydroxides of Li, Na, K, Mg, Ca, Al, and Zn, more preferably the oxides and hydroxides of Li, Na, K, and Zn, such as the oxides and hydroxides of Li, Na, and K. Exemplary salts which can be used with repect to the first and second media (e.g., the first and second buffers) include LiCl, NaCl and KCl, and one especially preferred salt in this respect is NaCl. Preferably, the salt is used in the first and second media (e.g., the first and second buffers) in a concentration which does not result in the precipitation of the RNA in said medium. The concentration of the salt in the first medium (e.g., the first buffer) is 15 to 70 mM, e.g., 20 to 60 mM, 25 to 50 mM or 30 to 50 mM, in particular if the buffering substance is TRIS. In one embodiment, the concentration of the salt in the second medium (e.g., the second buffer) is 15 to 70 mM, e.g., 20 to 60 mM, 25 to 50 mM or 30 to 50 mM, in particular if the buffering substance is TRIS. In one embodiment, the concentration of the salt in the second medium (e.g., the second buffer) is 100 to 200 mM, e.g., 110 to 190 mM, 120 to 180 mM, 130 to 170 mM, 140 to 160 mM or 145 to 155 mM, in particular if the buffering substance is HEPES.

The terms "buffering substance" and "buffering agent" as used herein mean a mixture of compounds capable of keeping the pH of a solution nearly constant even if a strong acid or base is added to the solution. In one embodiment, the buffering substance or buffering agent is a mixture of a weak acid and its conjugate base. In another embodiment, the buffering substance or buffering agent is a mixture of a weak base and its conjugate acid. Preferably, the buffering substance is not a chelating agent. Examples of buffering substances suitable for the first and second media (e.g., the first and second buffers) include tris(hydroxymethyl)aminomethane (TRIS), 4-(2-hydroxyethyl)-1-piperazineethanesulfonic acid (HEPES), 3-morpholino-2-hydroxypropanesulfonic acid (MOPSO), 3-(N-morpholino)propanesulfonic acid (MOPS), N,N-bis(2-hydroxyethyl)-2-aminoethanesulfonic acid (BES), 2-[(2-hydroxy-1,1-bis(hydroxymethyl)ethyl)amino]ethanesulfonic acid (TES), piperazine-N,N'-bis(2-ethanesulfonic acid) (PIPES), and 3-(N,N-bis[2-hydroxyethyl]amino)-2-hydroxypropanesulfonic acid (DIPSO), preferably TRIS or HEPES, more preferably TRIS. The desired pH value (such as pH 6.5 to 8.0, preferably pH 6.6 to 7.8, such as pH 6.8 to 7.6, pH 6.8 to 7.2, pH 6.9 to 7.5, pH 6.9 to 7.3, pH 7.0 to 7.7, pH 7.0 to 7.5, pH 7.0 to 7.3, pH 7.3 to 7.8, pH 7.3 to 7.7, or pH 7.3 to 7.6) can be achieved by adding a sufficient amount of acid (e.g., inorganic acid such as hydrochloric acid) to the corresponding base (e.g., TRIS) or by adding a sufficient amount of base (e.g., inorganic base such as sodium hydroxide) to the corresponding acid (e.g., PIPES if a pH above its pKa of 6.76 (such as a pH of 7.0 or 7.3 to 7.7) is desired). In one embodiment, the concentration of the buffering substance in the first and/or second medium (e.g., the first and/or second buffer) is 5 to 40 mM, e.g., 6 to 30 mM, 8 to 20 mM or 10 to 15 mM.

The term "chelating agent" as used herein with respect to the first and second media (e.g., the first and second buffers) means a compound (preferably an organic compound) which is a polydenate ligand and which is capable of forming two or more (preferably three or more, such as four or more) coordinate bonds to a single central atom (preferably a single metal cation such as Ca²⁺ or Mg²⁺). In this respect, "polydenate" refers to a ligand having more than one (i.e., two or more, preferably three or more, such as four or more) donor groups in a single ligand molecule, wherein donor groups preferably include atoms having free electron pairs (e.g., O⁻, =O, -NH₂, -NRH (wherein R is an organic moiety such as alkyl, in particular C₁₋₃ alkyl), and -NR₂ (wherein each R is independently an organic moiety such as alkyl, in particular C₁₋₃ alkyl)). Preferably, the chelating agent is not a buffering substance. Examples of chelating agents include EDTA, nitrilotriacetic acid, citrate salts (e.g., sodium citrate), 1,4,7,10-tetraazacyclododecane-1,4,7,10-tetraacetic acid (DOTA), 1,4,7-triazacyclononane-1,4,7-trisacetic acid (NOTA), 3,6,9,15-tetraazabicyclo[9.3.1]pentadeca-1(15),11,13-triene-3,6,9-triacetic acid (PCTA), and 1,4,7,10-tetraazacyclododecane-1,4,7-triacetic acid (DO3A), preferably EDTA or nitrilotriacetic acid, more preferably EDTA. In one embodiment, the concentration of the chelating agent in the first and/or second medium (e.g., the first and/or second buffer) is 10 to 50 mM, e.g., 15 to 40 mM or 20 to 30 mM.

The term "cellulose material" as used herein refers to any cellulose fibers, preferably having a grade suitable for use as a partition chromatography reagent. Particular examples of cellulose material suitable for the methods of the invention include CF-11 cellulose powder and commercially available celluloses such as those from Sigma-Aldrich (e.g., Cat. #C6288) and Macherey-Nagel (e.g., MN 100 or MN 2100). In one embodiment, the cellulose material is washed before use in the methods of the present invention. Thus, in one preferred embodiment of the methods of the present invention, the cellulose material is provided as a washed cellulose material, e.g., in dry form or as a suspension in a washing solution (wherein said washing solution may be the first or second medium (e.g., the first or second buffer) as specified herein). The washing of the cellulose material may include (I) mixing the cellulose material with a washing solution under shaking and/or stirring (preferably for at least 5 min, more preferably for at least 10 min, such as for 5 to 10 min); and (II) either removing the liquid (e.g., by using a pipette) or collecting the cellulose material (e.g., by using, as the cellulose material, a cellulose which is covalently coupled to magnetic beads and using a magnet); and optionally (III) repeating steps (I) and (II) once or two or more times (such as once, two times, or three times). For example, when the mixture of the cellulose material and the washing solution is provided in a tube, it is preferred that gravity or centrifugal force (e.g., 4,000 x g to 15,000 x g, such as 5,000 x g to 10,000 x g for 1 to 5 min) is applied to the tube such that the liquid and solid phases are separated (preferably completely separated) and that either the supernatant is removed (e.g., by using a pipette) or the cellulose material is collected (e.g., by using, as the cellulose material, a cellulose which is covalently coupled to magnetic beads and using a magnet). Alternatively, when the mixture of the cellulose material and the washing solution is provided in a spin column or filter device, it is preferred that gravity, centrifugal force (e.g., 4,000 x g to 15,000 x g, such as 5,000 x g to 10,000 x g for 1 to 5 min), pressure (e.g., 1000 hPa to 3000 hPa), or vacuum (e.g., 100 hPa to 900 hPa, such as 200 hPa to 800 hPa) is applied to the spin column or filter device such that the liquid and solid phases are separated (preferably completely separated) and that the flow through is discarded. The composition of the washing buffer preferably depends on the intended mode of selective binding of RNAs to the washed cellulose material: (1) If only dsRNA is to selectively bind to the washed cellulose material, whereas ssRNA is to remain unbound, the washing solution should be such that it allows binding of dsRNA to the cellulose material and it does not allow binding of ssRNA to the cellulose material. Thus, in a preferred embodiment of (1), the washing solution has the composition of the first medium (e.g., the first buffer) as specified above. (2) If both dsRNA and ssRNA are to bind to the washed cellulose material, the washing solution should be such that it allows binding of dsRNA and ssRNA to the cellulose material. Thus, in a preferred embodiment of (2), the washing solution has the composition of the second medium (e.g., the second buffer) as specified above.

After washing the washed cellulose material can be stored (or used in the methods of the present invention) as dry product (i.e., after the washing solution has been completely removed from the washed cellulose as specified herein) or as a suspension in the washing solution. However, if the washed cellulose material stored in the washing solution is to be used in the methods of the invention, it is preferred that before the washed cellulose material is to be contacted with the RNA preparation, the liquid phase (i.e., the washing solution in which the cellulose material is suspended for storage) is removed from the washed cellulose material (e.g., by applying gravity or centrifugal force (as specified above with respect to the washing of the cellulose material) if the washed cellulose is provided in a tube or by applying gravity, centrifugal force, pressure, or vacuum (as specified above with respect to the washing of the cellulose material) if the washed cellulose is provided in a spin column or filter device) and the resulting washed cellulose material as such (i.e., in dry form) is then used in the methods of the invention or is suspended in a washing solution (wherein said washing solution may be the first or second medium (e.g., the first or second buffer) as specified herein) and is then used in the methods of the invention.

The term "fresh cellulose material" as used herein means that said fresh cellulose material has not been brought into contact with an RNA preparation. Such fresh cellulose material can be either unwashed or washed. In a preferred embodiment, the fresh cellulose material is provided as a washed cellulose material as specified above (e.g., in dry form or as a suspension in the washing solution). Thus, depending on the intended use of the washed fresh cellulose material (i.e., to selectively bind either (1) dsRNA but not ssRNA or (2) dsRNA and ssRNA), the washed fresh cellulose material has been obtained by using either (1) the first medium (e.g., the first buffer) as specified above or (2) the second medium (e.g., the second buffer) as specified above.

The ratio of RNA (contained in the RNA preparation) to cellulose material in step (ii) of the methods of the invention is such that the RNA binding capacity of the cellulose material is not exceeded. In a preferred embodiment, the amount of RNA per 100 mg of cellulose material is at most 250 µg, more preferably at most 200 µg, such as at most 150 µg. Thus, in one embodiment, the amount of RNA per 100 mg of cellulose material is in the range of 10 to 250 µg, such as 20 to 220 µg, 30 to 200 µg, 40 to 180 µg, 50 to 160 µg, 60 to 140 µg, 70 to 120 µg, 80 to 110 µg, or 90 to 100 µg. Therefore, in one embodiment, the amount of cellulose material per 1 µg RNA may be at least 0.4 mg, preferably at least 0.5 mg, such as at least 0.67 mg. For example, the amount of cellulose material per 1 µg RNA may be in the range of 0.4 to 10 mg, such as 0.45 to 5 mg, 0.5 to 3.3 mg, 0.56 to 2.5 mg, 0.63 to 2 mg, 0.71 to 1.67 mg, 0.83 to 1.43 mg, 0.91 to 1.25 mg, or 1 to 1.11 mg.

The term "tube" as used herein refers to a container, in particular an elongated container, having only one opening such that compounds and/or liquids can be introduced into and/or removed from the container. In a preferred embodiment, the opening of the tube is configured such that it can be closed by a suitable means, such as a cover lid which is screwable or which fits into the opening in such a way so as to tightly seal the opening. In one embodiment, the tube is configured in such a way that gravity or centrifugal force can be applied to the tube (in order to separate the contents in the tube with respect to their specific gravity) without spilling any of the contents and without damaging the integrity of the tube.

The terms "spin column" and "filter device" as used herein refer to a container, in particular an elongated container, having two openings on opposite sides and a frit or filter, wherein the first opening is such that compounds and/or liquids can be introduced into and/or removed from the container, whereas the second opening is separated from the first opening by the frit or filter such that solid compounds (in particular the cellulose material) are withheld within the container by the frit or filter but that liquids are allowed to pass through to the frit or filter and to the second opening. The frit or filter preferably has a pore size of at most 1 µm, such at most 0.8 µm, at most 0.6 µm, e.g., in the range of 0.30 to 0.60 µm, such as 0.35 to 0.55 µm. In a preferred embodiment, at least the first opening of the spin column or filter device (preferably each of the openings) is configured such that it can be closed by a suitable means, such as a cover lid which is screwable or which fits into the first or second opening in such a way so as to tightly seal the opening. In one embodiment, the spin column or filter device is configured in such a way that gravity, centrifugal force, pressure, or vacuum can be applied to the spin column or filter device (in order to separate the contents in the spin column or filter device) without damaging the integrity of the spin column or filter device. Examples of suitable spin columns include microcentrifuge spin columns (such as those available from Macherey-Nagel, e.g., NucleoSpin Filters (Cat. #740606)), and examples of suitable filter devices include disposable vacuum-driven filter devices (such as those available from Merck Chemicals GmbH / Millipore, e.g., Steriflip-HV, 0.45 µm pore size, PVDF (Cat. #SE1M003M00)).

The terms "liquid" and "liquid phase" as used herein refer to a fluid at standard conditions. Particular examples of a liquid include a medium (e.g., a buffer), a washing solution, and an RNA preparation solved in a medium (e.g., a buffer). The terms "solid" and "solid phase" as used herein refer to a substance or mixture of substances which has a definite shape and volume but which is non-liquid and non-gaseous at standard conditions. A particular example of a solid includes a cellulose material.

The term "standard conditions" as used herein refers to a temperature of 20°C and an absolute pressure of 1,013.25 hPa.

The term "supernatant" as used herein refers to the upper phase which is generated when a liquid phase and a solid phase are mixed and the mixture is allowed to separate (e.g., by applying gravity or centrifugal force). In case the solid phase has a higher specific gravity compared to the liquid phase, the liquid phase will be the supernatant.

The term "flow through" as used herein refers to the liquid phase which passes through a spin column or a filter device.

The term "aliquot" as used herein means a volume of a liquid which is to be added to a solid or which is to be loaded onto the stationary phase of a column, wherein the volume of the aliquot is generally 0.1 to 10 times (such as 0.5 to 5 times, 1 to 4 times, 1 to 3 times, or 1 to 2 times) the volume of the solid or stationary phase. In one embodiment, the liquid is a medium (e.g., a buffer) (such as the first, second or third medium (e.g., the first, second, or third buffer) as specified herein) or a washing solution. In one embodiment, the solid is a cellulose material such as a washed cellulose material.

The term "applying gravity" as used herein means that a container, such as a tube, spin column, or filter device, is subjected to only the "normal" gravitational force of the earth (about 1 x g), i.e., no gravitational force in addition to the "normal" gravitational force of the earth is applied to the container (e.g., a medium is allowed to passively flow through the stationary phase of a column, wherein the column is arranged in such a manner that the longitudinal axis of the column (i.e., the line through both openings of the column) points to the geocenter). In one embodiment, gravity is applied to the container for a duration sufficient to separate (preferably completely separate) the phases (such as a liquid phase and a solid phase) contained in the container.

The term "applying centrifugal force" as used herein means that a container, such as a tube, spin column, or filter device, is subjected to a multiple of the normal gravitational force of the earth (i.e., more than 1 x g, such as at least 2 x g, at least 10 x g, at least 100 x g, and up to 20,000 x g, such as up to 15,000 x g, up to 10,000 x g, up to 5,000 x g, or up to 4,000 x g). A suitable device capable of generating such centrifugal force includes a centrifuge. In one embodiment, centrifugal force is applied to the container for a duration sufficient to separate (preferably completely separate) the phases (such as a liquid phase and a solid phase) contained in the container. Exemplary durations are in the range of 1 min to 30 min (such as 1, 2, 3, 4, or 5 min to 25 min, 5, 6, 7, 8, 9, or 10 min to 20 min or 10 to 15 min). Generally, the level and duration of the centrifugal force applied will depend on factors such as the intended use (e.g., washing of a cellulose material, binding of RNA to a cellulose material, or releasing of RNA from a cellulose material) and the volume and weight of the container (including its contents). For example, a high centrifugal force (such as 10,000 x g to 20,000 x g) applied for a short duration (e.g., 1 to 5 min) may be sufficient for a (complete) separation, whereas a low centrifugal force (such as up to 100 x g) may require a longer duration (such as 20 to 30 min) for a (complete) separation. Likewise, for a small volume and weight (e.g., up to a volume of about 2 ml and a weight of about 2 g) a high centrifugal force (such as 10,000 x g to 20,000 x g) applied for a short duration (e.g., 1 to 5 min) may be sufficient for a (complete) separation, whereas for a greater volume and/or weight, where only a lower centrifugal force (such as 200 x g to 10,000 x g) can be applied, a longer duration (e.g., for 20 to 30 min) may be necessary in order to achieve a (complete) separation.

The term "applying pressure" as used herein means that a container, such as a spin column, filter device, or column, is subjected to a positive force (compared to standard conditions) applied to one opening of the container. In particular, when the container is a column, applying pressure means that a liquid (such as a medium or buffer) is pumped through the container, e.g., by using one or more pumps. The pressure applied in the methods of the present invention is much lower compared to the pressure applied in HPLC methods and preferably is at most 2 MPa (such as at most 1 MPa, at most 5000 hPa, at most 4000 hPa, at most 3000 hPa, at most 2000 hPa).

The term "applying vacuum" as used herein means that a container, such as a spin column, filter device, or column, is subjected to a negative pressure (compared to standard conditions), wherein the negative pressure is preferably applied to an opening of the container. Preferably, a negative pressure is at most 900 hPa, such as at most 800 hPa, at most 700 hPa, at most 600 hPa, at most 500 hPa, at most 400 hPa, at most 300 hPa, at most 200 hPa, or at most 100 hPa. In one embodiment, vacuum is applied to the container for a duration sufficient to separate (preferably completely separate) the phases (such as a liquid phase and a solid phase) contained in the container. Devices for generating a negative pressure are known to the skilled person and include a water-jet vacuum pump.

The expression "repeated once or two or more times" as used herein means that the corresponding step or steps(s) are conducted at least once, such as two or more times, three or more times, four or more times, etc., preferably once, twice or three times.

The expression "one cycle of steps (ii) and (iii)" as used herein means that steps (ii) and (iii) are each conducted only once. If one cycle of steps (ii) and (iii) is completed, optionally a further cycle (also referred to herein as "next cycle") of steps (ii) and (iii) can be conducted. For example, in step (ii) of such a next cycle of steps (ii) and (iii), the RNA preparation obtained after step (iii) of the previous cycle of steps (ii) and (iii) (i.e., an RNA preparation which preferably comprises dsRNA in a lesser amount compared to the RNA preparation used in step (ii) of the previous cycle) is used as RNA preparation. It is preferred that in each cycle of steps (ii) and (iii) fresh cellulose material is used (in order to avoid contamination with e.g., dsRNA). Thus, by conducting steps (ii) and (iii) and optionally repeating these steps once or two or more times, it is possible to remove dsRNA from the ssRNA preparation to such an extent that the finally obtained ssRNA is substantially free from dsRNA and/or substantially free from DNA, preferably substantially free from dsRNA and DNA.

The expression "RNA preparation which comprises dsRNA in a lesser amount compared to the RNA preparation used in step (ii) of the previous cycle" means that conducting one cycle of steps (ii) and (iii) is effective in removing dsRNA such that the total amount of dsRNA in the RNA preparation obtained after step (iii) of one cycle is smaller than the total amount of dsRNA in the RNA preparation used in step (ii) of the previous cycle. Preferably, the first cycle of steps (ii) and (iii) is effective in removing at least 70%, more preferably at least 75% (such as at least 80%, at least 85%, at least 90%) of dsRNA contained in the RNA preparation used in step (ii) of the first cycle of steps (ii) and (iii). In a preferred embodiment, conducting a total of two, three or four cycles of steps (ii) and (iii) is effective in removing at least 95%, more preferably at least 96% (such as at least 97%, at least 98%, at least 99%) of dsRNA contained in the RNA preparation used in step (ii) of the first cycle of steps (ii) and (iii).

The term "eluent" as used herein means a liquid which is capable of altering the binding properties of a compound (such as dsRNA or ssRNA) with respect to a stationary phase (such as a cellulose material). "Altering the binding properties" means increasing or decreasing the ability of a compound (such as dsRNA or ssRNA) to bind to a stationary phase (such as a cellulose material). Preferably, an eluent is capable of decreasing the ability of a compound (such as dsRNA or ssRNA) to bind to a stationary phase (such as a cellulose material). Thus, in this preferred embodiment, (1) if the compound is bound to the stationary phase the step of bringing the stationary phase into contact with the eluent results in the release of the compound from the stationary phase, or (2) if the compound is solved in the eluent, the eluent decreases the compound's ability to bind to the stationary phase, preferably the eluent prevents the binding of the compound to the stationary phase. The term "eluting" as used herein means applying or loading an eluent (on)to a column (including a spin column) containing a stationary phase (such as a cellulose material) onto which a compound (such as ssRNA) is bound in order to release the compound from the stationary phase. A preferred eluent for ssRNA bound to a cellulose material is the first medium (e.g., the first buffer) as specified above, whereas a preferred eluent for dsRNA bound to a cellulose material is a third medium (e.g., a third buffer) which may have the same composition as the first or second medium (e.g., the first or second buffer) but which does not contain EtOH (e.g., the third medium may be water). A preferred washing solution which does not release dsRNA or ssRNA bound to a cellulose material is the second medium (e.g., the second buffer) as specified above.

The term "eluate" as used herein refers to the liquid exiting a column, when an eluent is applied or loaded onto the column.

The term "column" as used herein refers to a container, in particular a cylindrical container, having two openings on opposite sides, at least one frit, and a stationary phase (such as a cellulose material), wherein the first opening is configured so as to allow the introduction of liquids (such as a medium, e.g., a washing solution or a medium, for example, a first or second buffer) into the container, whereas the second opening is separated from the first opening and the stationary phase by the frit such that (i) the stationary phase is withheld within the column by the frit but (ii) liquids are allowed to pass through to frit and to the second opening. Columns can be configured to be useable in HPLC methods or in FPLC methods. However, columns to be used in the methods of the present invention are preferably configured to be useable in FPLC methods.

The term "HPLC" as used herein means high-pressure liquid chromatography, wherein a liquid phase is pumped under high pressure (typically at least 5 MPa, such as 5 to 35 MPa) through a column in order to separate, identify, and/or quantify at least one component in a mixture.

The term "FPLC" as used herein means fast performance liquid chromatography, wherein a liquid phase is allowed to flow through a column in order to separate, identify, and/or quantify at least one component in a mixture. The flow of the liquid through the FPLC column can be achieved by applying gravity or pressure, wherein the pressure preferably is at most 2 MPa (such as at most 1 MPa, at most 5,000 hPa, at most 4,000 hPa, at most 3,000 hPa, at most 2,000 hPa, or at most 1,000 hPa).

The term "pre-purification treatment" as used herein with respect to an RNA preparation means a procedure in order to partially or completely remove contaminants of the RNA preparation, wherein contaminants preferably include all compounds other than RNA, such as the starting materials used for generating IVT RNA (which is optionally modified) and their degradation products, e.g., a DNA template; an RNA polymerase (such as T7, T3 or SP6); monoribonucleotides in unmodified form (e.g., rATP, rGTP, rCTP, rUTP, and their analogs having only one or two phosphate groups) or modified form (e.g., r(1mΨ)TP or rΨTP and their analogs having only one or two phosphate groups); pyrophosphate; a cap reagent (i.e., a reagent to introduce a 5'-cap or 5'-cap analog); and additives used for generating IVT RNA (e.g., buffering agents, salts, antioxidizing agents, and polyamines (such as spermidine)). Examples of suitable pre-purification treatments which are known to the skilled person include precipitation of nucleic acids (preferably using lithium chloride); binding of nucleic acids (in particular RNA) to magnetic beads (e.g., the contaminants which do not bind to the magnetic beads can then be washed away using an appropriate medium); ultrafiltration; and degradation of DNA, preferably using duplex-specific nuclease (DSN). For example, RNA can be precipitated by using the "sodium acetate/isopropanol" precipitation method or the "LiCl" precipitation method (preferably by the "LiCl" precipitation method), both resulting in an RNA preparation in dried form. In each case, the precipitated and dried RNA thus obtained can be dissolved in a suitable amount of water or TE buffer (10 mM TRIS, 1 mM EDTA), both of which are preferably RNase-free.

The "sodium acetate/isopropanol" precipitation method includes the following steps: adding 0.1 volume of 3M sodium acetate (pH 4.0) and 1 volume of isopropanol to an RNA preparation, mixing the resulting mixture, incubating the mixture at -20°C for 1 h, applying centrifugal force (e.g., 14,000 x g for 10 min), removing the supernatant from the RNA pellet, washing the RNA pellet with 200 µl of 70% (v/v) ice-cold EtOH (i.e., adding 200 µl of 70% (v/v) ice-cold EtOH to the RNA pellet, applying centrifugal force (e.g., 14,000 x g for 5 min), and removing the supernatant from the RNA pellet), and drying (preferably air-drying) the RNA pellet (preferably in such a manner so as to remove the ethanol).

The "LiCl" precipitation method includes the following steps: adding lithium chloride (LiCl) to an RNA preparation such that the final LiCl concentration is 2.5 M, incubating the mixture at -20°C for 30 min, applying centrifugal force (e.g., 14,000 x g for 10 min), removing the supernatant from the RNA pellet, washing the RNA pellet with 200 µl of 70% (v/v) ice-cold EtOH (i.e., adding 200 µl of 70% (v/v) ice-cold EtOH to the RNA pellet, applying centrifugal force (e.g., 14,000 x g for 5 min), and removing the supernatant from the RNA pellet), and drying (preferably air-drying) the RNA pellet (preferably in such a manner so as to remove the ethanol).

The term "RNA polymerase" as used herein refers to a DNA-dependent RNA polymerase which produces primary transcript RNA. Examples of RNA polymerases suitable for generating IVT RNA according to the present invention include T7, T3 and SP6 RNA polymerases. A preferred RNA polymerase is T7 RNA polymerase.

The term "substantially free of dsRNA" as used herein in conjunction with ssRNA or an RNA preparation comprising ssRNA, wherein said ssRNA or RNA preparation comprising ssRNA has been subjected to a method of the present invention, means that the amount of dsRNA in the ssRNA or RNA preparation comprising ssRNA has been decreased by at least 70% (preferably at least 75%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%) compared to the amount of dsRNA contained in the ssRNA or RNA preparation comprising ssRNA before said ssRNA or RNA preparation comprising ssRNA has been subjected to the method of the present invention. Preferably, said ssRNA or RNA preparation comprising ssRNA which has been subjected to a method of the present invention has a content of dsRNA such that said ssRNA or RNA preparation comprising ssRNA when administered to a subject does not substantially induce an undesired response (such as an undesired induction of inflammatory cytokines (e.g., IFN-α) and/or an undesired activation of effector enzyme leading to an inhibition of protein synthesis from the ssRNA disclosed herein) in said subject. For example, the terms "substantially free of dsRNA" and "does not substantially induce an undesired response" may mean that, when administered to a subject, an ssRNA or RNA preparation comprising ssRNA, wherein said ssRNA or RNA preparation has been subjected to a method of the present invention, induces inflammatory cytokines (in particular IFN-α) in an amount which is reduced by at least 60% (e.g., at least 62%, at least 64%, at least 66%, at least 68%, at least 70%, at least 72%, at least 74%, at least 76%, at least 78%, at least 80%) compared to a control ssRNA (i.e., an ssRNA or RNA preparation comprising ssRNA which has not been subjected to a method of the present invention). Preferably, the terms "substantially free of dsRNA" and "does not substantially induce an undesired response" mean that, when administered to a subject, an ssRNA or RNA preparation comprising ssRNA, wherein said ssRNA or RNA preparation has been subjected to a method of the present invention and said ssRNA codes for a peptide or protein, results in the translation of the ssRNA into the peptide or protein for at least 10 h (e.g., at least 12 h, at least 14 h, at least 16 h, at least 18 h, at least 20 h, at least 22 h, or at least 24 h) after administration. For example, the content of dsRNA in ssRNA or an RNA preparation comprising ssRNA, wherein said ssRNA or RNA preparation comprising ssRNA has been subjected to a method of the present invention, may be at most 5% by weight (preferably at most 4% by weight, at most 3% by weight, at most 2% by weight, at most 1% by weight, at most 0.5% by weight, at most 0.1% by weight, at most 0.05% by weight, at most 0.01% by weight, at most 0.005% by weight, at most 0.001% by weight), based on the total weight of said ssRNA or RNA preparation comprising ssRNA.

The term "substantially free of DNA" as used herein in conjunction with ssRNA or an RNA preparation comprising ssRNA, wherein said ssRNA or RNA preparation comprising ssRNA has been subjected to a method of the present invention, means that the amount of dsRNA in the ssRNA or RNA preparation comprising ssRNA may be at most 5% by weight (preferably at most 4% by weight, at most 3% by weight, at most 2% by weight, at most 1% by weight, at most 0.5% by weight, at most 0.1% by weight, at most 0.05% by weight, at most 0.01% by weight, at most 0.005% by weight, at most 0.001% by weight), based on the total weight of said ssRNA or RNA preparation comprising ssRNA.

The term "substantially free of dsRNA and DNA" as used herein in conjunction with ssRNA or an RNA preparation comprising ssRNA, wherein said ssRNA or RNA preparation comprising ssRNA has been subjected to a method of the present invention, means that said ssRNA or an RNA preparation comprising ssRNA is substantially free of dsRNA as specified above (e.g., the translation lasts at least 10 h after administration and/or the dsRNA content is at most 5% by weight) and is substantially free of DNA as specified above (e.g., the DNA content is at most 5% by weight).

In the context of the present invention, the term "RNA" relates to a molecule which comprises ribonucleotide residues and preferably is entirely or substantially composed of ribonucleotide residues. "Ribonucleotide" relates to a nucleotide with a hydroxyl group at the 2'-position of a β-D-ribofuranosyl group. The term "RNA" comprises isolated RNA such as partially or completely purified RNA, essentially pure RNA, synthetic RNA, and recombinantly generated RNA and includes modified RNA which differs from naturally occurring RNA by addition, deletion, substitution and/or alteration of one or more nucleotides. Such alterations can include addition of non-nucleotide material, such as to the end(s) of an RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered/modified nucleotides can be referred to as analogs of naturally-occurring nucleotides, and the corresponding RNAs containing such altered/modified nucleotides (i.e., altered/modified RNAs) can be referred to as analogs of naturally-occurring RNAs. A molecule is "substantially composed of ribonucleotide residues" if the content of ribonucleotide residues in the molecule is at least 40% (such as at least 45%, at least 50%, at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

RNA can be isolated from cells, can be made from a DNA template, or can be chemically synthesized using methods known in the art. In preferred embodiments, RNA is synthesized *in vitro* from a DNA template. In one particularly preferred embodiment, RNA, in particular ssRNA such as mRNA or an inhibitory ssRNA (e.g., antisense RNA, siRNA or miRNA), is generated by *in vitro* transcription from a DNA template. The *in vitro* transcription methodology is known to the skilled person; cf., e.g., Molecular Cloning: A Laboratory Manual, 2nd Edition, J. Sambrook et al. eds., Cold Spring Harbor Laboratory Press, Cold Spring Harbor 1989. Furthermore, there is a variety of *in vitro* transcription kits commercially available, e.g., from Thermo Fisher Scientific (such as TranscriptAid^{™} T7 kit, MEGAscript^{®} T7 kit, MAXIscript^{®}), New England BioLabs Inc. (such as HiScribe^{™} T7 kit, HiScribe^{™} T7 ARCA mRNA kit), Promega (such as RiboMAX^{™} HeLaScribe^{®}, Riboprobe^{®} systems), Jena Bioscience (such as SP6 or T7 transcription kits), and Epicentre (such as AmpliScribe^{™}). In one particularly preferred embodiment, RNA is *in vitro* transcribed RNA (IVT RNA). For providing modified RNA, correspondingly modified nucleotides, such as modified naturally occurring nucleotides, non-naturally occurring nucleotides and/or modified non-naturally occurring nucleotides, can be incorporated during synthesis (preferably *in vitro* transcription), or modifications can be effected in and/or added to the RNA after transcription.

According to the invention, preferred as RNA are synthetic oligonucleotides of 6 to 100, preferably 10 to 50, in particular 15 to 30 or 15 to 20 nucleotides or longer transcripts of more than 50 nucleotides, preferably 100 to 15,000, more preferably 50 to 10,000, more preferably 100 to 5,000, in particular 200 to 1,000 nucleotides.

According to the invention, "RNA" includes mRNA, tRNA, rRNA, snRNAs, ssRNA, dsRNAs, and inhibitory RNA.

According to the invention, "ssRNA" includes mRNA and inhibitory ssRNA (such as antisense ssRNA, siRNA, or miRNA).

"ssRNA" means single-stranded RNA. ssRNA may contain self-complementary sequences that allow parts of the RNA to fold and pair with itself to form double helices. The size of the ssRNA may vary from 6 nucleotides to 15,000, preferably 10 to 12,000, in particular 100 to 10,000, 150 to 8,000, 200 to 7,000, 250 to 6,000, or 300 to 5,000 nucleotides. In one embodiment, the ssRNA has a length of at least 2,700 nucleotides (such as at least 2,800, at least 2,900, at least 3,000, at least 3,100, at least 3,200, at least 3,300, at least 3,400, at least 3,500, at least 3,600, at least 3,700, at least 3,800, at least 3,900, at least 4,000, at least 4,100, at least 4,200, at least 4,300, at least 4,400, at least 4,500, at least 4,600, at least 4,700, at least 4,800, at least 4,900, at least 5,000 nucleotides). Long ssRNA as used herein means ssRNA having a size of at least 3,500 nucleotides (such as at least 3,600, at least 3,700, at least 3,800, at least 3,900, at least 4,000, at least 4,100, at least 4,200, at least 4,300, at least 4,400, at least 4,500, at least 4,600, at least 4,700, at least 4,800, at least 4,900, at least 5,000, at least 5,500, at least 6,000, at least 6,500, at least 7,000, at least 7,500, at least 8,000, at least 8,500, at least 9,000, at least 9,500 nucleotides), preferably up to 15,000, such as up to 14,000, up to 13,000 or up to 12,000 nucleotides.

According to the invention, "dsRNA" means double-stranded RNA and is RNA with two partially or completely complementary strands. The size of the strands may vary from 6 nucleotides to 10,000, preferably 10 to 8,000, in particular 200 to 5,000, 200 to 2,000 or 200 to 1,000 nucleotides.

According to the present invention, the term "mRNA" means "messenger-RNA" and relates to a "transcript" which may be generated by using a DNA template and may encode a peptide or protein. Typically, an mRNA comprises a 5'-UTR, a protein coding region, and a 3'-UTR. In the context of the present invention, mRNA is preferably generated by *in vitro* transcription from a DNA template. As set forth above, the *in vitro* transcription methodology is known to the skilled person, and a variety of *in vitro* transcription kits commercially is available. The size of the mRNA may vary from about 1,000 nucleotides to 15,000, preferably 2,000 to 12,000, in particular 2,700 to 11,000, 3000 to 10,000, 3,500 to 9,000, 4,000 to 9,000, 4,500 to 7,000, or 5,000 to 8,000 nucleotides. In one embodiment, the mRNA has a length of at least 2,700 nucleotides (such as at least 2,800, at least 2,900, at least 3,000, at least 3,100, at least 3,200, at least 3,300, at least 3,400, at least 3,500, at least 3,600, at least 3,700, at least 3,800, at least 3,900, at least 4,000, at least 4,100, at least 4,200, at least 4,300, at least 4,400, at least 4,500, at least 4,600, at least 4,700, at least 4,800, at least 4,900, at least 5,000 nucleotides). Long mRNA means mRNA having a size of at least 3,500 nucleotides (such as at least at least 3,600, at least 3,700, at least 3,800, at least 3,900, at least 4,000, at least 4,100, at least 4,200, at least 4,300, at least 4,400, at least 4,500, at least 4,600, at least 4,700, at least 4,800, at least 4,900, at least 5,000, at least 5,500, at least 6,000, at least 6,500, at least 7,000, at least 7,500, at least 8,000, at least 8,500, at least 9,000, at least 9,500 nucleotides), preferably up to 15,000, such as up to 14,000, up to 13,000, up to 12,000, up to 11,000, or up to 10,000 nucleotides.

mRNA only possesses limited half-life in cells and *in vitro.* Thus, according to the invention, the stability and translation efficiency of RNA may be modified as required. For example, mRNA may be stabilized and its translation increased by one or more modifications having a stabilizing effect and/or increasing translation efficiency of mRNA. Such modifications are described, for example, in WO 2007/036366. In order to increase expression of the mRNA disclosed herein, it may be modified within the coding region, i.e., the sequence encoding the expressed peptide or protein, preferably without altering the sequence of the expressed peptide or protein, so as to increase the GC-content to increase mRNA stability and to perform a codon optimization and, thus, enhance translation in cells.

The term "modification" in the context of the RNA, preferably of the ssRNA (such as mRNA) disclosed herein includes any modification of an RNA (preferably ssRNA, such as mRNA) which is not naturally present in said RNA.

In one embodiment of the invention, the ssRNA (preferably mRNA) disclosed herein does not have uncapped 5'-triphosphates. Removal of such uncapped 5'-triphosphates can be achieved by treating ssRNA (preferably mRNA) with a phosphatase.

The ssRNA (preferably mRNA) disclosed herein may have modified ribonucleotides in order to increase its stability and/or decrease cytotoxicity. For example, in one embodiment, in the ssRNA (preferably mRNA) disclosed herein 5-methylcytidine is substituted partially or completely, preferably completely, for cytidine. Alternatively or additionally, in one embodiment, in the ssRNA (preferably mRNA) disclosed herein pseudouridine or N(1)-methylpseudouridine is substituted partially or completely, preferably completely, for uridine. An RNA (preferably ssRNA such as mRNA) which is modified by pseudouridine (substituting partially or completely, preferably completely, for uridine) is referred to herein as "Ψ-modified", whereas the term "1mΨ-modified" means that the RNA (preferably ssRNA such as mRNA) contains N(1)-methylpseudouridine (substituting partially or completely, preferably completely, for uridine).

In one embodiment, the term "modification" relates to providing an RNA (preferably ssRNA, such as mRNA) with a 5'-cap or 5'-cap analog. The term "5'-cap" refers to a cap structure found on the 5'-end of an RNA (preferably ssRNA, such as mRNA) molecule and generally consists of a guanosine nucleotide connected to the RNA (preferably ssRNA, such as mRNA) via an unusual 5' to 5' triphosphate linkage. In one embodiment, this guanosine is methylated at the 7-position. The term "conventional 5'-cap" refers to a naturally occurring RNA 5'-cap, preferably to the 7-methylguanosine cap (m7G). In the context of the present invention, the term "5'-cap" includes a 5'-cap analog that resembles the RNA cap structure and is modified to possess the ability to stabilize RNA (preferably ssRNA, such as mRNA) and/or enhance translation of RNA (preferably ssRNA, such as mRNA) if attached thereto, preferably *in vivo* and/or in a cell.

Preferably, the 5' end of the RNA (preferably ssRNA, such as mRNA) includes a cap structure having the following general formula: 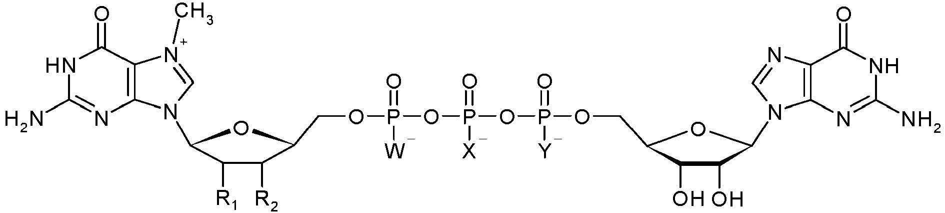 wherein R₁ and R₂ are independently hydroxy or methoxy and W, X and Y are independently oxygen, sulfur, selenium, or BH₃. In a preferred embodiment, R₁ and R₂ are hydroxy and W, X and Y are oxygen. In a further preferred embodiment, one of R₁ and R₂, preferably R₁ is hydroxy and the other is methoxy and W, X and Y are oxygen. In a further preferred embodiment, R₁ and R₂ are hydroxy and one of W, X and Y, preferably X is sulfur, selenium, or BH₃, preferably sulfur, while the other are oxygen. In a further preferred embodiment, one of R₁ and R₂, preferably R₂ is hydroxy and the other is methoxy and one of W, X and Y, preferably X is sulfur, selenium, or BH₃, preferably sulfur while the other are oxygen.

In the above formula, the nucleotide on the right hand side is connected to the RNA (preferably ssRNA, such as mRNA) chain through its 3' group.

Those cap structures wherein at least one of W, X and Y is sulfur, i.e., which have a phosphorothioate moiety, exist in different diastereoisomeric forms all of which are encompassed herein. Furthermore, the present invention encompasses all tautomers and stereoisomers of the above formula.

For example, the cap structure having the above structure, wherein R₁ is methoxy, R₂ is hydroxy, X is sulfur and W and Y are oxygen exists in two diastereoisomeric forms (Rp and Sp). These can be resolved by reverse phase HPLC and are named D1 and D2 according to their elution order from the reverse phase HPLC column. According to the invention, the D1 isomer of m₂^{7,2'-*O*}GppₛpG is particularly preferred. Consequently, the term "D1-capped" as used herein refers to an RNA (preferably ssRNA such as mRNA) which is capped with the D1 isomer of m₂^{7,2'-*O*}GppₛpG as specified above. Similarly, the term "D2-capped" as used herein refers to an RNA (preferably ssRNA such as mRNA) which is capped with the D2 isomer of m₂^{7,2'-*O*}GppₛpG as specified above. Further examples of cap structures are known to the skilled person and include those described in WO 2008/157688.

Providing an RNA (preferably ssRNA, such as mRNA) with a 5'-cap or 5'-cap analog may be achieved by *in vitro* transcription of a DNA template in presence of said 5'-cap or 5'-cap analog, wherein said 5'-cap is co-transcriptionally incorporated into the generated RNA (preferably ssRNA, such as mRNA) strand, or the RNA (preferably ssRNA, such as mRNA) may be generated, for example, by *in vitro* transcription, and the 5'-cap may be attached to the RNA (preferably ssRNA, such as mRNA) post-transcriptionally using capping enzymes, for example, capping enzymes of vaccinia virus.

The RNA (preferably ssRNA, such as mRNA) may comprise further modifications. For example, a further modification of the ssRNA disclosed herein may be an extension or truncation of the naturally occurring poly(A) tail or an alteration of the 5'- or 3'-untranslated (also called "5'- or 3'-non-translated") regions (UTR).

RNA (preferably ssRNA, such as mRNA) having an unmasked poly-A sequence is translated more efficiently than RNA (preferably ssRNA, such as mRNA) having a masked poly-A sequence. The term "poly(A) tail" or "poly-A sequence" relates to a sequence of adenosine (in particular adenylyl) (A) residues which typically is located on the 3'-end of an RNA (preferably ssRNA, such as mRNA) molecule and "unmasked poly-A sequence" means that the poly-A sequence at the 3' end of an RNA (preferably ssRNA, such as mRNA) molecule ends with an A of the poly-A sequence and is not followed by nucleotides other than A located at the 3' end, i.e., downstream, of the poly-A sequence. Furthermore, a long poly-A sequence having a length of about 120 nucleotides results in an optimal transcript stability and translation efficiency of an RNA (preferably ssRNA, such as mRNA).

Therefore, in order to increase stability and/or expression of RNA, preferably of the ssRNA (such as mRNA) disclosed herein, it may be modified so as to be present in conjunction with a poly-A sequence, preferably having a length of 10 to 500, more preferably 30 to 300, even more preferably 65 to 200 and especially 100 to 150 adenosine (in particular adenylyl) residues. In an especially preferred embodiment the poly-A sequence has a length of approximately 120 adenosine (in particular adenylyl) residues. To further increase stability and/or expression of RNA, preferably of the ssRNA (such as mRNA) disclosed herein, the poly-A sequence can be unmasked.

In addition, incorporation of a 3'-UTR into the 3'-non translated region of an RNA (preferably ssRNA, such as mRNA) molecule can result in an enhancement in translation efficiency. A synergistic effect may be achieved by incorporating two or more of such 3'-UTRs. The 3'-UTRs may be autologous or heterologous to the RNA (preferably ssRNA, such as mRNA) into which they are introduced. In one particular embodiment the 3'-UTR is derived from a globin gene or mRNA, such as a gene or mRNA of alpha2-globin, alphal-globin, or beta-globin, preferably beta-globin, more preferably human beta-globin.

A combination of the above described modifications, i.e., incorporation of a poly-A sequence, unmasking of a poly-A sequence, incorporation of one or more 3'-UTRs and replacing one or more naturally occurring nucleotides with synthetic nucleotides (e.g., 5-methylcytidine for cytidine and/or pseudouridine (Ψ) or N(1)-methylpseudouridine (1mΨ) for uridine), has a synergistic influence on the stability of RNA (preferably ssRNA, such as mRNA) and increase in translation efficiency.

The term "inhibitory RNA" as used herein means RNA which selectively hybridizes to and/or is specific for the target mRNA, thereby inhibiting (e.g., reducing) transcription and/or translation thereof. Inhibitory RNA includes RNA molecules having sequences in the antisense orientation relative to the target mRNA. Suitable inhibitory oligonucleotides typically vary in length from five to several hundred nucleotides, more typically about 20 to 70 nucleotides in length or shorter, even more typically about 10 to 30 nucleotides in length. Examples of inhibitory RNA include antisense RNA, ribozyme, iRNA, siRNA and miRNA.

The term "antisense RNA" as used herein refers to an RNA which hybridizes under physiological conditions to DNA comprising a particular gene or to mRNA of said gene, thereby inhibiting transcription of said gene and/or translation of said mRNA. An antisense transcript of a nucleic acid or of a part thereof may form a duplex with naturally occurring mRNA and thus prevent accumulation of or translation of the mRNA. Another possibility is the use of ribozymes for inactivating a nucleic acid. The antisense RNA may hybridize with an N-terminal or 5' upstream site such as a translation initiation site, transcription initiation site or promoter site. In further embodiments, the antisense RNA may hybridize with a 3'-untranslated region or mRNA splicing site.

The size of the antisense RNA may vary from 15 nucleotides to 15,000, preferably 20 to 12,000, in particular 100 to 10,000, 150 to 8,000, 200 to 7,000, 250 to 6,000, 300 to 5,000 nucleotides, such as 15 to 2,000, 20 to 1,000, 25 to 800, 30 to 600, 35 to 500, 40 to 400, 45 to 300, 50 to 250, 55 to 200, 60 to 150, or 65 to 100 nucleotides. In one embodiment, the antisense RNA has a length of at least 2,700 nucleotides (such as at least 2,800, at least 2,900, at least 3,000, at least 3,100, at least 3,200, at least 3,300, at least 3,400, at least 3,500, at least 3,600, at least 3,700, at least 3,800, at least 3,900, at least 4,000, at least 4,100, at least 4,200, at least 4,300, at least 4,400, at least 4,500, at least 4,600, at least 4,700, at least 4,800, at least 4,900, at least 5,000 nucleotides). Long antisense RNA as used herein means antisense RNA having a size of at least 3,500 nucleotides (such as at least 3,600, at least 3,700, at least 3,800, at least 3,900, at least 4,000, at least 4,100, at least 4,200, at least 4,300, at least 4,400, at least 4,500, at least 4,600, at least 4,700, at least 4,800, at least 4,900, at least 5,000, at least 5,500, at least 6,000, at least 6,500, at least 7,000, at least 7,500, at least 8,000, at least 8,500, at least 9,000, at least 9,500 nucleotides), preferably up to 15,000, such as up to 14,000, up to 13,000, up to 12,000, up to 11,000, or up to 10,000 nucleotides.

The stability of antisense RNA may be modified as required. For example, antisense RNA may be stabilized by one or more modifications having a stabilizing effect. Such modifications include modified phosphodiester linkages (such as methylphosphonate, phosphorothioate, phosphorodithioate or phosphoramidate linkages instead of naturally occurring phosphodiester linkages) and 2'-substitutions (e.g., 2'-fluoro, 2'-O-alkyl (such as 2'-O-methyl, 2'-O-propyl, or 2'-O-pentyl) and 2'-O-allyl). For example, in one embodiment of the antisense RNA, phosphorothioate linkages are substituted partially for phosphodiester linkages. Alternatively or additionally, in one embodiment of the antisense RNA, the ribose moiety is substituted partially at the 2'-position with O-alkyl (such as 2'-O-methyl).

An antisense RNA can be targeted to any stretch of approximately 19 to 25 contiguous nucleotides in any of the target mRNA sequences (the "target sequence"). Generally, a target sequence on the target mRNA can be selected from a given cDNA sequence corresponding to the target mRNA, preferably beginning 50 to 100 nt downstream (i.e., in the 3'-direction) from the start codon. The target sequence can, however, be located in the 5'- or 3'-untranslated regions, or in the region nearby the start codon.

Antisense RNA can be obtained using a number of techniques known to those of skill in the art. For example, antisense RNA can be chemically synthesized or recombinantly produced using methods known in the art. Preferably, antisense RNA is transcribed from recombinant circular or linear DNA plasmids using any suitable promoter.

Selection of plasmids suitable for expressing antisense RNA, methods for inserting nucleic acid sequences for expressing the antisense RNA into the plasmid, and IVT methods of *in vitro* transcription of said antisense RNA are within the skill in the art.

An "antisense ssRNA" relates to an antisense RNA as specified above which is single-stranded.

By "small interfering RNA" or "siRNA" as used herein is meant an RNA molecule, preferably greater than 10 nucleotides in length, more preferably greater than 15 nucleotides in length, and most preferably 18, 19, 20, 21, 22, 23, 24, 25, 26, 27, 28, 29, or 30 nucleotides in length that is capable of binding specifically to a portion of a target mRNA. This binding induces a process, in which said portion of the target mRNA is cut or degraded and thereby the gene expression of said target mRNA inhibited. A range of 19 to 25 nucleotides is the most preferred size for siRNAs. Although, in principle, the sense and antisense strands of siRNAs can comprise two complementary, single-stranded RNA molecules, the siRNAs disclosed hereincomprise a single molecule in which two complementary portions are base-paired and are covalently linked by a single-stranded "hairpin" area. That is, the sense region and antisense region can be covalently connected via a linker molecule. The linker molecule can be a polynucleotide or non-nucleotide linker, but is preferably a polynucleotide linker. Without wishing to be bound by any theory, it is believed that the hairpin area of the single-stranded siRNA molecule is cleaved intracellularly by the "Dicer" protein (or its equivalent) to form an siRNA of two individual base-paired RNA molecules.

The siRNA can also comprise a 3'-overhang. As used herein, a "3'-overhang" refers to at least one unpaired nucleotide extending from the 3'-end of an RNA strand. Thus, in one embodiment, the siRNA comprises at least one 3'-overhang of from 1 to about 6 nucleotides (which includes ribonucleotides or deoxynucleotides) in length, preferably from 1 to about 5 nucleotides in length, more preferably from 1 to about 4 nucleotides in length, and particularly preferably from about 2 to about 4 nucleotides in length. In the embodiment in which both strands of the siRNA molecule (i.e., after the single-stranded siRNA molecule is cleaved intracellularly by the "Dicer" protein) comprise a 3'-overhang, the length of the overhangs can be the same or different for each strand. In a most preferred embodiment, the 3'-overhang is present on both strands of the siRNA, and is 2 nucleotides in length. For example, each strand of the siRNA can comprise 3'-overhangs of dideoxythymidylic acid ("TT") or diuridylic acid ("uu").

In order to enhance the stability of the siRNA, the 3'-overhangs can be also stabilized against degradation. In one embodiment, the overhangs are stabilized by including purine nucleotides, such as adenosine or guanosine nucleotides. Alternatively, substitution of pyrimidine nucleotides by modified analogues, e.g., substitution of uridine nucleotides in the 3'-overhangs with 2'-deoxythymidine, is tolerated and does not affect the efficiency of RNAi degradation. In particular, the absence of a 2'-hydroxyl in the 2'-deoxythymidine significantly enhances the nuclease resistance of the 3'-overhang in tissue culture medium.

As used herein, "target mRNA" refers to an RNA molecule that is a target for downregulation.

siRNA disclosed herein can be targeted to any stretch of approximately 19 to 25 contiguous nucleotides in any of the target mRNA sequences (the "target sequence"). Techniques for selecting target sequences for siRNA are given, for example, in Tuschl T. et al., "The siRNA User Guide", revised Oct. 11, 2002. "The siRNA User Guide" is available on the world wide web at a website maintained by Dr. Thomas Tuschl, Laboratory of RNA Molecular Biology, Rockefeller University, New York, USA, and can be found by accessing the website of the Rockefeller University and searching with the keyword "siRNA". Further guidance with respect to the selection of target sequences and/or the design of siRNA can be found on the webpages of Protocol Online (www.protocol-online.com) using the keyword "siRNA". Thus, in one embodiment, the sense strand of the siRNA disclosed herein comprises a nucleotide sequence substantially identical to any contiguous stretch of about 19 to about 25 nucleotides in the target mRNA.

Generally, a target sequence on the target mRNA can be selected from a given cDNA sequence corresponding to the target mRNA, preferably beginning 50 to 100 nt downstream (i.e., in the 3'-direction) from the start codon. The target sequence can, however, be located in the 5'- or 3'-untranslated regions, or in the region nearby the start codon.

siRNA can be obtained using a number of techniques known to those of skill in the art. For example, siRNA can be chemically synthesized or recombinantly produced using methods known in the art, such as the Drosophila *in vitro* system described in U.S. application no. 2002/0086356 of Tuschl et al.. siRNA can be expressed from pol III expression vectors without a change in targeting site, as expression of RNAs from pol III promoters is only believed to be efficient when the first transcribed nucleotide is a purine.

Preferably, siRNA is transcribed from recombinant circular or linear DNA plasmids using any suitable promoter. Suitable promoters for transcribing siRNA disclosed herein from a plasmid include, for example, the U6 or HI RNA pol III promoter sequences and the cytomegalovirus promoter. Selection of other suitable promoters is within the skill in the art.

Selection of plasmids suitable for transcribing siRNA, methods for inserting nucleic acid sequences for expressing the siRNA into the plasmid, and IVT methods of *in vitro* transcription of said siRNA are within the skill in the art.

The term "miRNA" (microRNA) as used herein relates to non-coding RNAs which have a length of 21 to 25 (such as 21 to 23, preferably 22) nucleotides and which induce degradation and/or prevent translation of target mRNAs. miRNAs are typically found in plants, animals and some viruses, wherein they are encoded by eukaryotic nuclear DNA in plants and animals and by viral DNA (in viruses whose genome is based on DNA), respectively. miRNAs are post-transcriptional regulators that bind to complementary sequences on target messenger RNA transcripts (mRNAs), usually resulting in translational repression or target degradation and gene silencing.

miRNA can be obtained using a number of techniques known to those of skill in the art. For example, antisense RNA can be chemically synthesized or recombinantly produced using methods known in the art (e.g., by using commercially available kits such as the miRNA cDNA Synthesis Kit sold by Applied Biological Materials Inc.). Preferably, antisense RNA is transcribed from recombinant circular or linear DNA plasmids using any suitable promoter. Techniques for predicting the secondary structure of RNAs are given, for example, in Sato et al. (Nucleic Acids Res. 37(2009):W277-W280), Hamada et al. (Nucleic Acids Res. 39(2011):W100-W106 2011), and Reuter and Mathews (BMC Bioinformatics 11(2010):129).

The term "nucleoside" relates to compounds which can be thought of as nucleotides without a phosphate group. While a nucleoside is a nucleobase linked to a sugar (e.g., ribose or deoxyribose), a nucleotide is composed of a nucleoside and one or more phosphate groups. Examples of nucleosides include cytidine, uridine, adenosine, and guanosine.

The five standard nucleosides which make up nucleic acids are uridine, adenosine, thymidine, cytidine and guanosine. The five nucleosides are commonly abbreviated to their one letter codes U, A, T, C and G, respectively. However, thymidine is more commonly written as "dT" ("d" represents "deoxy") as it contains a 2'-deoxyribofuranose moiety rather than the ribofuranose ring found in uridine. This is because thymidine is found in deoxyribonucleic acid (DNA) and not ribonucleic acid (RNA). Conversely, uridine is found in RNA and not DNA. The remaining three nucleosides may be found in both RNA and DNA. In RNA, they would be represented as A, C and G, whereas in DNA they would be represented as dA, dC and dG.

The term "stability" of RNA (preferably ssRNA, such as mRNA) relates to the "half-life" of the RNA. "Half-life" relates to the period of time which is needed to eliminate half of the activity, amount, or number of molecules. In the context of the present invention, the half-life of an RNA (preferably ssRNA, such as mRNA or inhibitory ssRNA) is indicative for the stability of said RNA.

Of course, if according to the present invention it is desired to decrease stability of RNA (preferably ssRNA such as mRNA or inhibitory ssRNA), it is possible to modify RNA (preferably ssRNA such as mRNA or inhibitory ssRNA) so as to interfere with the function of elements as described above increasing the stability of RNA (preferably ssRNA such as mRNA or inhibitory ssRNA).

In one embodiment, the ssRNA disclosed herein is (modified) ssRNA, in particular (modified) mRNA, encoding a peptide or protein. According to the invention, the term "ssRNA encoding a peptide or protein" means that the ssRNA, if present in the appropriate environment, preferably within a cell, can direct the assembly of amino acids to produce, i.e., express, the peptide or protein during the process of translation. Preferably, ssRNA (such as mRNA) disclosed herein is able to interact with the cellular translation machinery allowing translation of the peptide or protein.

The term "expression" is used according to the invention in its most general meaning and comprises the production of RNA and/or peptides or proteins, e.g., by transcription and/or translation. With respect to RNA, the term "expression" or "translation" relates in particular to the production of peptides or proteins. It also comprises partial expression of nucleic acids. Moreover, expression can be transient or stable.

In the context of the present invention, the term "transcription" relates to a process, wherein the genetic code in a DNA sequence is transcribed into RNA. Subsequently, the RNA may be translated into protein. According to the present invention, the term "transcription" comprises "*in vitro* transcription", wherein the term *"in vitro* transcription" relates to a process, wherein RNA, in particular ssRNA such as mRNA, is *in vitro* synthesized in a cell-free system, preferably using appropriate cell extracts. Preferably, cloning vectors are applied for the generation of transcripts. These cloning vectors are generally designated as transcription vectors and are according to the present invention encompassed by the term "vector". According to the present invention, the RNA preparation comprises ssRNA produced by *in vitro* transcription, in particular *in vitro* transcription of an appropriate DNA template. The promoter for controlling transcription can be any promoter for any RNA polymerase. Particular examples of RNA polymerases are the T7, T3, and SP6 RNA polymerases. Preferably, the *in vitro* transcription is controlled by a T7, T3, or SP6 promoter. A DNA template for *in vitro* transcription may be obtained by cloning of a nucleic acid, in particular cDNA, and introducing it into an appropriate vector for *in vitro* transcription. The cDNA may be obtained by reverse transcription of RNA.

The cDNA containing vector template may comprise vectors carrying different cDNA inserts which following transcription results in a population of different RNA molecules optionally capable of expressing different peptides or proteins or may comprise vectors carrying only one species of cDNA insert which following transcription only results in a population of one RNA species capable of expressing only one peptide or protein. Thus, it is possible to produce RNA capable of expressing a single peptide or protein only or to produce compositions of different RNAs such as RNA libraries and whole-cell RNA capable of expressing more than one peptide or protein, e.g., a composition of peptides or proteins. The present invention envisions the introduction of all such RNA into cells.

The term "translation" according to the invention relates to the process in the ribosomes of a cell by which a strand of mRNA directs the assembly of a sequence of amino acids to make a peptide or protein.

The term "peptide" as used herein comprises oligo- and polypeptides and refers to substances comprising two or more, preferably 3 or more, preferably 4 or more, preferably 6 or more, preferably 8 or more, preferably 10 or more, preferably 13 or more, preferably 16 more, preferably 21 or more and up to preferably 8, 10, 20, 30, 40 or 50, in particular 100 amino acids joined covalently by peptide bonds. The term "protein" preferentially refers to large peptides, preferably to peptides with more than 100 amino acid residues, but in general the terms "peptide" and "protein" are synonyms and are used interchangeably herein.

According to the present invention, ssRNA such as mRNA may encode a peptide or protein. Accordingly, ssRNA may contain a coding region (open reading frame (ORF)) encoding a peptide or protein. For example, ssRNA may encode and express an antigen or a pharmaceutically active peptide or protein such as an immunologically active compound (which preferably is not an antigen). In this respect, an "open reading frame" or "ORF" is a continuous stretch of codons beginning with a start codon and ending with a stop codon.

The term "pharmaceutically active peptide or protein" includes a peptide or protein that can be used in the treatment of a subject where the expression of a peptide or protein would be of benefit, e.g., in ameliorating the symptoms of a disease or disorder. For example, a pharmaceutically active protein can replace or augment protein expression in a cell which does not normally express a protein or which misexpresses a protein, e.g., a pharmaceutically active protein can compensate for a mutation by supplying a desirable protein. In addition, a "pharmaceutically active peptide or protein" can produce a beneficial outcome in a subject, e.g., can be used to produce a protein to which vaccinates a subject against an infectious disease. Preferably, a "pharmaceutically active peptide or protein" has a positive or advantageous effect on the condition or disease state of a subject when administered to the subject in a therapeutically effective amount. Preferably, a pharmaceutically active peptide or protein has curative or palliative properties and may be administered to ameliorate, relieve, alleviate, reverse, delay onset of or lessen the severity of one or more symptoms of a disease or disorder. A pharmaceutically active peptide or protein may have prophylactic properties and may be used to delay the onset of a disease or to lessen the severity of such disease or pathological condition. The term "pharmaceutically active peptide or protein" includes entire proteins or polypeptides, and can also refer to pharmaceutically active fragments thereof. It can also include pharmaceutically active analogs of a peptide or protein. The term "pharmaceutically active peptide or protein" includes peptides and proteins that are antigens, i.e., the peptide or protein elicits an immune response in a subject which may be therapeutic or partially or fully protective.

Examples of pharmaceutically active proteins include, but are not limited to, cytokines and immune system proteins such as immunologically active compounds (e.g., interleukins, colony stimulating factor (CSF), granulocyte colony stimulating factor (G-CSF), granulocyte-macrophage colony stimulating factor (GM-CSF), erythropoietin, tumor necrosis factor (TNF), interferons, integrins, addressins, selectins, homing receptors, T cell receptors, immunoglobulins, soluble major histocompatibility complex antigens, immunologically active antigens such as bacterial, parasitic, or viral antigens, allergens, autoantigens, antibodies), hormones (insulin, thyroid hormone, catecholamines, gonadotrophines, trophic hormones, prolactin, oxytocin, dopamine, bovine somatotropin, leptins and the like), growth hormones (e.g., human grown hormone), growth factors (e.g., epidermal growth factor, nerve growth factor, insulin-like growth factor and the like), growth factor receptors, enzymes (tissue plasminogen activator, streptokinase, cholesterol biosynthetic or degradative, steroidogenic enzymes, kinases, phosphodiesterases, methylases, de-methylases, dehydrogenases, cellulases, proteases, lipases, phospholipases, aromatases, cytochromes, adenylate or guanylate cyclases, neuramidases and the like), receptors (steroid hormone receptors, peptide receptors), binding proteins (growth hormone or growth factor binding proteins and the like), transcription and translation factors, tumor growth suppressing proteins (e.g., proteins which inhibit angiogenesis), structural proteins (such as collagen, fibroin, fibrinogen, elastin, tubulin, actin, and myosin), and blood proteins (thrombin, serum albumin, Factor VII, Factor VIII, insulin, Factor IX, Factor X, tissue plasminogen activator, protein C, von Willebrand factor, antithrombin III, glucocerebrosidase, erythropoietin granulocyte colony stimulating factor (GCSF) or modified Factor VIII, anticoagulants and the like).

In one embodiment, the pharmaceutically active protein is a cytokine which is involved in regulating lymphoid homeostasis, preferably a cytokine which is involved in and preferably induces or enhances development, priming, expansion, differentiation and/or survival of T cells. In one embodiment, the cytokine is an interleukin. In one embodiment, the pharmaceutically active protein is an interleukin selected from the group consisting of IL-2, IL-7, IL-12, IL-15, and IL-21.

The term "immunologically active compound" relates to any compound altering an immune response, preferably by inducing and/or suppressing maturation of immune cells, inducing and/or suppressing cytokine biosynthesis, and/or altering humoral immunity by stimulating antibody production by B cells. Immunologically active compounds possess potent immunostimulating activity including, but not limited to, antiviral and antitumor activity, and can also down-regulate other aspects of the immune response, for example shifting the immune response away from a TH2 immune response, which is useful for treating a wide range of TH2 mediated diseases. Immunologically active compounds can be useful as vaccine adjuvants.

In one embodiment, ssRNA (such as mRNA) that codes for an antigen such a disease-associated antigen is administered to a mammal, in particular if treating a mammal having a disease involving or expressing the antigen (disease-associated antigen) is desired. The ssRNA is preferably taken up into the mammal's antigen-presenting cells (monocytes, macrophages, dendritic cells or other cells). An antigenic translation product of the ssRNA is formed and the product is displayed on the surface of the cells for recognition by T cells. In one embodiment, the antigen or a product produced by optional procession thereof is displayed on the cell surface in the context of MHC molecules for recognition by T cells through their T cell receptor leading to their activation.

Interferons are important cytokines characterized by antiviral, antiproliferative and immunomodulatory activities. Interferons are proteins that alter and regulate the transcription of genes within a cell by binding to interferon receptors on the regulated cell's surface, thereby preventing viral replication within the cells. The interferons can be grouped into two types. IFN-gamma is the sole type II interferon; all others are type I interferons. Type I and type II interferons differ in gene structure (type II interferon genes have three exons; type I, one), chromosome location (in humans, type II is located on chromosome-12; the type I interferon genes are linked and on chromosome-9), and the types of tissues where they are produced (type I interferons are synthesized ubiquitously, type II by lymphocytes). Type I interferons competitively inhibit each other binding to cellular receptors, while type II interferon has a distinct receptor. According to the invention, the term "interferon" or "IFN" preferably relates to type I interferons, in particular IFN-alpha and IFN-beta.

In one embodiment, RNA, in particular RNA which is to be expressed in a cell, is a single stranded self-replicating RNA. In one embodiment, the self-replicating RNA is single stranded RNA of positive sense. In one embodiment, the self-replicating RNA is viral RNA or RNA derived from viral RNA. In one embodiment, the self-replicating RNA is alphaviral genomic RNA or is derived from alphaviral genomic RNA. In one embodiment, the self-replicating RNA is a viral gene expression vector. In one embodiment, the virus is Semliki forest virus. In one embodiment, the self-replicating RNA contains one or more transgenes which in one embodiment, if the RNA is viral RNA, may partially or completely replace viral sequences such as viral sequences encoding structural proteins.

The term "RNA preparation" as used herein refers to any composition comprising at least one type of the various RNA types specified above (i.e., mRNA, tRNA, rRNA, snRNAs, ssRNA, dsRNAs, and inhibitory ssRNA (such as antisense RNA, siRNA, or miRNA)). The term "RNA preparation comprising ssRNA" as used herein refers to any composition comprising at least ssRNA (however, said composition may also comprise dsRNA). The term "RNA preparation comprising ssRNA produced by *in vitro* transcription" refers to any composition comprising at least ssRNA, wherein said ssRNA has been generated by *in vitro* transcription.

The term *"in vitro* transcription" or "IVT" as used herein means that the transcription (i.e., the generation of RNA) is conducted in a cell-free manner. I.e., IVT does not use living/cultured cells but rather the transcription machinery extracted from cells (e.g., cell lysates or the isolated components thereof, including an RNA polymerase (preferably T7, T3 or SP6 polymerase)).

The term "optional" or "optionally" as used herein means that the subsequently described event, circumstance or condition may or may not occur, and that the description includes instances where said event, circumstance, or condition occurs and instances in which it does not occur.

"Isomers" are compounds having the same molecular formula but differ in structure ("structural isomers") or in the geometrical positioning of the functional groups and/or atoms ("stereoisomers"). "Enantiomers" are a pair of stereoisomers which are non-superimposable mirror-images of each other. A "racemic mixture" or "racemate" contains a pair of enantiomers in equal amounts and is denoted by the prefix (±). "Diastereomers" are stereoisomers which are non-superimposable mirror-images of each other. "Tautomers" are structural isomers of the same chemical substance that spontaneously interconvert with each other, even when pure.

Terms such as "decreasing", "reducing" or "inhibiting" relate to the ability to cause an overall decrease, preferably of 5% or greater, 10% or greater, 20% or greater, more preferably of 50% or greater, and most preferably of 75% or greater, in the level. This also includes a complete or essentially complete decrease, i.e. a decrease to zero or essentially to zero.

Terms such as "increasing", "enhancing", or "prolonging" preferably relate to an increase, enhancement, or prolongation by about at least 10%, preferably at least 20%, preferably at least 30%, preferably at least 40%, preferably at least 50%, preferably at least 80%, preferably at least 100%, preferably at least 200% and in particular at least 300%. These terms may also relate to an increase, enhancement, or prolongation from zero or a non-measurable or non-detectable level to a level of more than zero or a level which is measurable or detectable.

The term "naturally occurring" as used herein refers to the fact that an object can be found in nature. For example, a protein or nucleic acid which is present in an organism (including viruses), can be isolated from a source in nature and has not been intentionally modified by man in the laboratory is naturally occurring.

The ssRNA disclosed herein may be isotopically labeled, i.e., one or more atoms of the ssRNA are replaced by a corresponding atom having the same number of protons but differing in the number of neutrons. For example, a hydrogen atom may be replaced by a deuterium atom. Exemplary isotopes which can be used in the ssRNA disclosed herein include deuterium, ¹¹C, ¹³C, ¹⁴C, ¹⁵N, ¹⁸F, ³²S, ³⁶Cl, and ¹²⁵I. Isotopically labeled ssRNA can be produced by using correspondingly isotopically labeled nucleotides during the *in vitro* transcription or by adding such correspondingly isotopically labeled nucleotides after transcription.

Also described herein is a pharmaceutical composition comprising an ssRNA obtained by a method of the invention and one or more pharmaceutically acceptable excipients. In one embodiment, the pharmaceutical composition comprises an ssRNA obtained by a method of the invention, one or more pharmaceutically acceptable excipients and one or more additional/supplementary active compounds.

Also disclosed herein is an ssRNA as specified above or a pharmaceutical composition as specified herein for use in therapy.

For example, the ssRNA and pharmaceutical compositions disclosed herein may be used in the treatment (including prophylactic treatment) of a condition, disorder or disease selected from the group consisting of infectious diseases (e.g., those caused by viruses, bacteria, fungi or other microorganisms); an undesirable inflammation (such as an immune disorder); and cancer.

Thus, also disclosed is (i) an ssRNA disclosed herein (or a pharmaceutical composition comprising such ssRNA optionally together with a pharmaceutically acceptable excipient) for use in a method of treating a condition, disorder or disease as specified herein, in particular a disease selected from the group consisting of infectious diseases (e.g., those caused by a virus, bacterium, fungus or other microorganism); an undesirable inflammation; and cancer; and (ii) a method of treating an individual with a need thereof, comprising administering a pharmaceutically effective amount of an ssRNA disclosed herein (or a pharmaceutical composition comprising such ssRNA optionally together with a pharmaceutically acceptable excipient), to the individual. In one embodiment, the individual is suffering from, or is susceptible to or at risk of, one or more of the conditions, disorders or diseases disclosed herein. The condition, disorder or disease may be selected from the group consisting of infectious diseases (e.g., those caused by a virus, bacterium, fungus or other microorganism); an undesirable inflammation; and cancer. Moreover, the individual is preferably a mammal and more preferably a human.

Cancer (medical term: malignant neoplasm) is a class of diseases in which a group of cells display uncontrolled growth (division beyond the normal limits), invasion (intrusion on and destruction of adjacent tissues), and sometimes metastasis (spread to other locations in the body via lymph or blood). These three malignant properties of cancers differentiate them from benign tumors, which are self-limited, and do not invade or metastasize. Most cancers form a tumor, i.e., a swelling or lesion formed by an abnormal growth of cells (called neoplastic cells or tumor cells), but some, like leukemia, do not. The term "cancer" according to the invention comprises leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer and the metastases thereof. Examples thereof are lung carcinomas, mamma carcinomas, prostate carcinomas, colon carcinomas, renal cell carcinomas, cervical carcinomas, or metastases of the cancer types or tumors described above. The term cancer according to the invention also comprises cancer metastases.

Examples of cancers treatable with the ssRNA and pharmaceutical compositions disclosed herein include malignant melanoma, all types of carcinoma (colon, renal cell, bladder, prostate, non-small cell and small cell lung carcinoma, etc.), lymphomas, sarcomas, blastomas, gliomas, etc.

Malignant melanoma is a serious type of skin cancer. It is due to uncontrolled growth of pigment cells, called melanocytes.

According to the invention, a "carcinoma" is a malignant tumor derived from epithelial cells. This group represents the most common cancers, including the common forms of breast, prostate, lung and colon cancer.

Lymphoma and leukemia are malignancies derived from hematopoietic (blood-forming) cells.

A sarcoma is a cancer that arises from transformed cells in one of a number of tissues that develop from embryonic mesoderm. Thus, sarcomas include tumors of bone, cartilage, fat, muscle, vascular, and hematopoietic tissues.

Blastic tumor or blastoma is a tumor (usually malignant) which resembles an immature or embryonic tissue. Many of these tumors are most common in children.

A glioma is a type of tumor that starts in the brain or spine. It is called a glioma because it arises from glial cells. The most common site of gliomas is the brain.

By "metastasis" is meant the spread of cancer cells from its original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor, i.e., a secondary tumor or metastatic tumor, at the target site depends on angiogenesis. Tumor metastasis often occurs even after the removal of the primary tumor because tumor cells or components may remain and develop metastatic potential. In one embodiment, the term "metastasis" according to the invention relates to "distant metastasis" which relates to a metastasis which is remote from the primary tumor and the regional lymph node system.

Exemplary immune disorders include, but are not limited to, autoimmune diseases (for example, diabetes mellitus, arthritis (including rheumatoid arthritis, juvenile rheumatoid arthritis, osteoarthritis and psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjogren's Syndrome, Crohn's disease, aphthous ulcer, iritis, conjunctivitis, keratoconjunctivitis, ulcerative colitis, asthma, allergic asthma, sepsis and septic shock, inflammatory bowel disorder, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens- Johnson syndrome, glomerulonephritis, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

Exemplary viruses include, but are not limited to, are human immunodeficiency virus (HIV), Epstein-Barr virus (EBV), cytomegalovirus (CMV) (e.g., CMV5), human herpesviruses (HHV) (e.g., HHV6, 7 or 8), herpes simplex viruses (HSV), bovine herpes virus (BHV) (e.g., BHV4), equine herpes virus (EHV) (e.g., EHV2), human T-Cell leukemia viruses (HTLV)5, Varicella-Zoster virus (VZV), measles virus, papovaviruses (JC and BK), hepatitis viruses (e.g., HBV or HCV), myxoma virus, adenovirus, parvoviruses, polyoma virus, influenza viruses, papillomaviruses and poxviruses such as vaccinia virus, and molluscum contagiosum virus (MCV), and lyssaviruses. Such virus may or may not express an apoptosis inhibitor. Exemplary diseases caused by viral infection include, but are not limited to, chicken pox, Cytomegalovirus infections, genital herpes, Hepatitis B and C, influenza, and shingles, and rabies.

Exemplary bacteria include, but are not limited to, *Campylobacter jejuni,* Enterobacter species, *Enterococcus faecium, Enterococcus faecalis, Escherichia coli* (e.g., F. coli 0157:H7), Group A streptococci, *Haemophilus influenzae, Helicobacter pylori,* listeria, *Mycobacterium tuberculosis, Pseudomonas aeruginosa, S. pneumoniae, Salmonella, Shigella, Staphylococcus aureus,* and *Staphylococcus epidermidis,* and *Borrelia* and *Rickettsia.* Exemplary diseases caused by bacterial infection include, but are not limited to, anthrax, cholera, diphtheria, foodborne illnesses, leprosy, meningitis, peptic ulcer disease, pneumonia, sepsis, septic shock, syphilis, tetanus, tuberculosis, typhoid fever, and urinary tract infection, and Lyme disease and Rocky Mountain spotted fever.

Particular examples of infectious diseases treatable with the ssRNA and pharmaceutical compositions disclosed herein include viral infectious diseases, such as AIDS (HIV), hepatitis A, B or C, herpes, herpes zoster (chicken-pox), German measles (rubella virus), yellow fever, dengue fever; infectious diseases caused by flaviviruses; influenza; hemorrhagic infectious diseases (Marburg or Ebola viruses); bacterial infectious diseases (such as Legionnaire's disease (*Legionella*), gastric ulcer (*Helicobacter*), cholera (*Vibrio*), infections by *E. coli, Staphylococci, Salmonella* or *Streptococci* (tetanus); infections by protozoan pathogens such as malaria, sleeping sickness, leishmaniasis, toxoplasmosis, i.e. infections by *Plasmodium, Trypanosoma, Leishmania* and *Toxoplasma;* or fungal infections, which are caused, e.g., by *Cryptococcus neoformans, Histoplasma capsulatum, Coccidioides immitis, Blastomyces dermatitidis* or *Candida albicans.*

The ssRNA and pharmaceutical compositions disclosed herein can be used alone or in conjunction with one or more additional/supplementary active compounds which can be administered prior to, simultaneously with or after administration of the ssRNA or pharmaceutical composition disclosed herein. Such one or more additional/supplementary active compounds include chemotherapeutic drugs for cancer patients (e.g. gemcitabine, etopophos, cis-platin, carbo-platin), antiviral agents, anti-parasite agents, anti-bacterial agents, immunotherapeutic agents (e.g., antigens or fragments thereof (in particular immunogenic fragments thereof)), and adjuvants, and, if administered simultaneously with the ssRNA disclosed herein, may be present in a pharmaceutical composition disclosed herein.

In particular, the one or more additional/supplementary active compounds can comprise an immunotherapeutic agent, preferably an immunotherapeutic agent inducing or effecting a targeted, i.e., specific, immune reaction. Thus, in one embodiment, the ssRNA and pharmaceutical compositions disclosed herein can be used in conjunction with an immunotherapeutic agent, preferably an immunotherapeutic agent inducing or effecting a targeted, i.e., specific, immune reaction. Such immunotherapeutic agents include agents directed against a disease-associated antigen such as therapeutic antibodies or agents inducing an immune response directed against a disease-associated antigen or cells expressing a disease-associated antigen. Useful immunotherapeutic agents include proteins or peptides inducing a B cell or T cell response against the disease-associated antigen or cells expressing the disease-associated antigen. These proteins or peptides may comprise a sequence essentially corresponding to or being identical to the sequence of the disease-associated antigen or one or more fragments thereof. In one embodiment, the protein or peptide comprises the sequence of an MHC presented peptide derived from the disease-associated antigen. Instead of administering the protein or peptide it is also possible to administer nucleic acid, preferably mRNA, encoding the protein or peptide. The RNA encoding the protein or peptide may be the ssRNA disclosed herein. Alternatively or additionally, the RNA encoding the protein or peptide may be a different RNA not according to the present invention which RNA may be administered simultaneously with (in this case the RNA may form part of a pharmaceutical composition disclosed herein) and/or prior to and/or after administration of a pharmaceutical composition disclosed herein. Accordingly, the pharmaceutical composition disclosed herein may be used in genetic vaccination, wherein an immune response is stimulated by introduction into a subject a suitable nucleic acid molecule (DNA or mRNA) which codes for an antigen or a fragment thereof.

In one embodiment, a disease-associated antigen is a tumor-associated antigen. In this embodiment, the ssRNA and pharmaceutical compositions disclosed herein may be useful in treating cancer or cancer metastasis. Preferably, the diseased organ or tissue is characterized by diseased cells such as cancer cells expressing a disease-associated antigen and/or being characterized by association of a disease-associated antigen with their surface. Immunization with intact or substantially intact tumor-associated antigen or fragments thereof such as MHC class I and class II peptides or nucleic acids, in particular mRNA, encoding such antigen or fragment makes it possible to elicit an MHC class I and/or a class II type response and thus, stimulate T cells such as CD8+ cytotoxic T lymphocytes which are capable of lysing cancer cells and/or CD4+ T cells. Such immunization may also elicit a humoral immune response (B cell response) resulting in the production of antibodies against the tumor-associated antigen. Furthermore, antigen presenting cells (APC) such as dendritic cells (DCs) can be loaded with MHC class I-presented peptides directly or by transfection with nucleic acids encoding tumor antigens or tumor antigen peptides *in vitro* and administered to a patient.

According to the present invention, a tumor-associated antigen preferably comprises any antigen which is characteristic for tumors or cancers as well as for tumor or cancer cells with respect to type and/or expression level. In one embodiment, the term "tumor-associated antigen" relates to proteins that are under normal conditions, i.e., in a healthy subject, specifically expressed in a limited number of organs and/or tissues or in specific developmental stages, for example, the tumor-associated antigen may be under normal conditions specifically expressed in stomach tissue, preferably in the gastric mucosa, in reproductive organs, e.g., in testis, in trophoblastic tissue, e.g., in placenta, or in germ line cells, and are expressed or aberrantly expressed in one or more tumor or cancer tissues. In this context, "a limited number" preferably means not more than 3, more preferably not more than 2 or 1. The tumor-associated antigens in the context of the present invention include, for example, differentiation antigens, preferably cell type specific differentiation antigens, i.e., proteins that are under normal conditions specifically expressed in a certain cell type at a certain differentiation stage, cancer/testis antigens, i.e., proteins that are under normal conditions specifically expressed in testis and sometimes in placenta, and germ line specific antigens. In the context of the present invention, the tumor-associated antigen is preferably associated with the cell surface of a cancer cell and is preferably not or only rarely expressed in normal tissues. Preferably, the tumor-associated antigen or the aberrant expression of the tumor-associated antigen identifies cancer cells. In the context of the present invention, the tumor-associated antigen that is expressed by a cancer cell in a subject, e.g., a patient suffering from a cancer disease, is preferably a self-protein in said subject. In preferred embodiments, the tumor-associated antigen in the context of the present invention is expressed under normal conditions specifically in a tissue or organ that is non-essential, i.e., tissues or organs which when damaged by the immune system do not lead to death of the subject, or in organs or structures of the body which are not or only hardly accessible by the immune system. In one embodiment, the amino acid sequence of the tumor-associated antigen is identical between the tumor-associated antigen which is expressed in normal tissues and the tumor-associated antigen which is expressed in cancer tissues. Preferably, a tumor-associated antigen is presented in the context of MHC molecules by a cancer cell in which it is expressed.

Examples for differentiation antigens which ideally fulfill the criteria for tumor-associated antigens as contemplated by the present invention as target structures in tumor immunotherapy, in particular, in tumor vaccination are the cell surface proteins of the claudin family, such as CLDN6 and CLDN18.2. These differentiation antigens are expressed in tumors of various origins, and are particularly suited as target structures in connection with antibody-mediated cancer immunotherapy due to their selective expression (no expression in a toxicity relevant normal tissue) and localization to the plasma membrane.

Further examples for antigens that may be useful in the present invention are p53, ART-4, BAGE, beta-catenin/m, Bcr-abL CAMEL, CAP-1, CASP-8, CDC27/m, CDK4/m, CEA, CLAUDIN-12, c-MYC, CT, Cyp-B, DAM, ELF2M, ETV6-AML1, G250, GAGE, GnT-V, Gap100, HAGE, HER-2/neu, HPV-E7, HPV-E6, HAST-2, hTERT (or hTRT), LAGE, LDLR/FUT, MAGE-A, preferably MAGE-A1, MAGE-A2, MAGE-A3, MAGE-A4, MAGE-A5, MAGE-A6, MAGE-A7, MAGE-A8, MAGE-A9, MAGE-A10, MAGE-A11, or MAGE-A12, MAGE-B, MAGE-C, MART-1/Melan-A, MC1R, Myosin/m, MUC1, MUM-1, -2, -3, NA88-A, NF1, NY-ESO-1, NY-BR-1, p190 minor BCR-abL, Pm1/RARa, PRAME, proteinase 3, PSA, PSM, RAGE, RU1 or RU2, SAGE, SART-1 or SART-3, SCGB3A2, SCP1, SCP2, SCP3, SSX, SURVIVIN, TEL/AML1, TPI/m, TRP-1, TRP-2, TRP-2/INT2, TPTE and WT, preferably WT-1.

An "antigen" is to be understood as meaning any structure which can cause the formation of antibodies and/or the activation of a cellular immune response. Examples of antigens are polypeptides, proteins, cells, cell extracts, carbohydrates/polysaccharides, polysaccharide conjugates, lipids, and glycolipids. These antigens may be tumor antigens or viral, bacterial, fungal and protozoological antigens or allergens. The term "antigen" also includes derivatized antigens as secondary substance which becomes antigenic - and sensitizing - only through transformation (e.g., intermediately in the molecule, by completion with body protein), and conjugated antigens which, through artificial incorporation of atomic groups (e.g., isocyanates, diazonium salts), display a new constitutive specificity. The antigen may be present in the vaccine disclosed herein in the form of a hapten coupled to a suitable carrier. Suitable carriers are known to those ordinarily skilled in the art and include e.g. human serum albumin (HSA), polyethylene glycols (PEG). The hapten may be coupled to the carrier by processes well-known in the prior art, e.g., in the case of a polypeptide carrier via an amide bond to a Lys residue.

The term "immunogenicity" refers to the ability of a particular substance, in particular RNA (preferably ssRNA, such as mRNA), to provoke an immune response in the body of a subject such as a human. In other words, immunogenicity is the ability to induce an immune response.

"Inducing an immune response" may mean that there was no immune response before inducing an immune response, but it may also mean that there was a certain level of immune response before inducing an immune response and after inducing an immune response said immune response is enhanced. Thus, "inducing an immune response" includes "enhancing an immune response". Preferably, after inducing an immune response in a subject, said subject is protected from developing a disease such as a cancer or infectious disease or the disease condition is ameliorated by inducing an immune response.

The term "immunotherapy" relates to a treatment preferably involving a specific immune reaction and/or immune effector function(s).

The term "immunization" or "vaccination" describes the process of treating a subject for therapeutic or prophylactic reasons.

The terms "subject", "patient", or "individual", relate to vertebrates. For example, vertebrates in the context of the present invention are mammals, birds (e.g., poultry), reptiles, amphibians, bony fishes, and cartilaginous fishes, in particular domesticated animals of any of the foregoing as well as animals in captivity such as animals of zoos, and are preferably mammals. Mammals in the context of the present invention include, but are not limited to, humans, non-human primates, domesticated mammals, such as dogs, cats, sheep, cattle, goats, pigs, horses etc., laboratory mammals such as mice, rats, rabbits, guinea pigs, etc. as well as mammals in captivity such as mammals of zoos. The term "subject" as used herein also includes humans.

Terms such as "transferring", "transfecting" or "introducing into cells" are used interchangeably herein and relate to the introduction of nucleic acids, in particular exogenous or heterologous nucleic acids, in particular ssRNA into a cell. According to the present invention, the cell can form part of an organ, a tissue and/or an organism.

The pharmaceutical compositions disclosed herein are generally applied in "pharmaceutically acceptable amounts" and in "pharmaceutically acceptable preparations". The term "pharmaceutically acceptable" refers to the non-toxicity of a material which does not interact with the action of the active agent(s) of the pharmaceutical composition.

According to the present invention, the administration of a nucleic acid (such as ssRNA) is either achieved as naked nucleic acid or in combination with one or more pharmaceutically acceptable excipients. Preferably, administration of nucleic acids is in the form of naked nucleic acids. Preferably, the RNA is administered in combination with stabilizing substances such as RNase inhibitors. The present invention also envisions the repeated introduction of nucleic acids into cells to allow sustained expression for extended time periods.

Cells can be transfected with any excipients (in particular carriers) with which ssRNA can be associated, e.g., by forming complexes with the ssRNA or forming vesicles in which the ssRNA is enclosed or encapsulated, resulting in increased stability of the ssRNA compared to naked ssRNA. Excipients (in particular carriers) useful according to the invention include, for example, lipid-containing carriers such as cationic lipids, liposomes, in particular cationic liposomes, and micelles, and nanoparticles. Cationic lipids may form complexes with negatively charged nucleic acids. Any cationic lipid may be used according to the invention. Furthermore, cells can be taken from a subject, the cells can be transfected with ssRNA or a pharmaceutical composition disclosed herein, and the transfected cells can be inserted into the subject.

Preferably, the introduction of ssRNA which encodes a peptide or polypeptide into a cell, in particular into a cell present *in vivo,* results in expression of said peptide or polypeptide in the cell. In particular embodiments, the targeting of the nucleic acids to particular cells is preferred. In such embodiments, a carrier which is applied for the administration of the nucleic acid to a cell (for example, a retrovirus or a liposome), exhibits a targeting molecule. For example, a molecule such as an antibody which is specific for a surface membrane protein on the target cell or a ligand for a receptor on the target cell may be incorporated into the nucleic acid carrier or may be bound thereto. In case the nucleic acid is administered by liposomes, proteins which bind to a surface membrane protein which is associated with endocytosis may be incorporated into the liposome formulation in order to enable targeting and/or uptake. Such proteins encompass capsid proteins or fragments thereof which are specific for a particular cell type, antibodies against proteins which are internalized, proteins which target an intracellular location, etc.

The term "excipient" when used herein is intended to indicate all substances in a pharmaceutical composition which are not active agents (e.g., which are therapeutically inactive ingredients that do not exhibit any therapeutic effect in the amount/concentration used), such as, e.g., salts, carriers, binders, lubricants, thickeners, surface active agents, dispersing agents, preservatives, emulsifiers, buffering agents, wetting agents, flavoring agents, colorants, stabilizing agents (such as RNase inhibitors) or antioxidants all of which are preferably pharmaceutically acceptable.

"Pharmaceutically acceptable salts" comprise, for example, acid addition salts which may, for example, be formed by using a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, suitable pharmaceutically acceptable salts may include alkali metal salts (e.g., sodium or potassium salts); alkaline earth metal salts (e.g., calcium or magnesium salts); ammonium (NH₄⁺); and salts formed with suitable organic ligands (e.g., quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, galactate, galacturonate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphthoate, iodide, isobutyrate, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, teoclate, tosylate, triethiodide, undecanoate, valerate, and the like (see, for example, S. M. Berge et al., "Pharmaceutical Salts", J. Pharm. Sci., 66, pp. 1-19 (1977)). Salts which are not pharmaceutically acceptable may be used for preparing pharmaceutically acceptable salts and are included in the invention.

The compositions disclosed herein may comprise a pharmaceutically acceptable carrier. As used herein, "pharmaceutically acceptable carrier" includes any and all solvents, dispersion media, coatings, isotonic and absorption delaying agents, and the like that are physiologically compatible. The "pharmaceutically acceptable carrier" may be in the form of a solid, semisolid, liquid, or combinations thereof.

Pharmaceutically acceptable carriers include sterile aqueous solutions or dispersions, sterile nonaqueous solutions or dispersions and sterile powders for the extemporaneous preparation of sterile injectable solutions or dispersions. The use of such media and agents for pharmaceutically active agents is known in the art. Except insofar as any conventional media or agent is incompatible with the active agent, use thereof in the pharmaceutical compositions disclosed herein is contemplated. Exemplary pharmaceutically acceptable carriers for an injectable formulation include water, an isotonic buffered saline solution (e.g., Ringer or Ringer lactate), ethanol, polyols (e.g., glycerol), polyalkylene glycols (e.g., propylene glycol and liquid polyethylene glycol), hydrogenated naphthalenes, and, in particular, biocompatible lactide polymers (e.g., lactide/glycolide copolymers or polyoxyethylene/polyoxypropylene copolymers).

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Suitable buffering agents for use in the pharmaceutical compositions disclosed herein include acetic acid in a salt, citric acid in a salt, boric acid in a salt and phosphoric acid in a salt.

Suitable preservatives for use in the pharmaceutical compositions disclosed herein include various antibacterial and antifungal agents, such as benzalkonium chloride, chlorobutanol, paraben, sorbic acid, and thimerosal. Prevention of the presence of microorganisms may also be ensured by sterilization procedures (e.g., sterilization filtration, in particular sterilization microfiltration).

The pharmaceutical composition disclosed herein may be administered to an individual by any route, preferably parenterally. The expressions "parenteral administration" and "administered parenterally" as used herein mean modes of administration other than enteral administration ("enteral administration" and "administered enterally" as used herein mean that the drug administered is taken up by the stomach and/or the intestine). Parenteral administration is usually by injection and/or infusion and includes, without limitation, intravenous, intramuscular, intraarterial, intrathecal, intracapsular, intraosseous, intraorbital, intracardiac, intradermal, intraperitoneal, transtracheal, subcutaneous, subcuticular, intraarticular, subcapsular, intracerebral, intracerebroventricular, subarachnoid, intraspinal, epidural intrasternal, and topical administration.

The ssRNA or pharmaceutical composition disclosed herein can be administered by a variety of methods known in the art. As will be appreciated by the skilled artisan, the route and/or mode of administration will vary depending upon the desired results.

The active agents (i.e., the ssRNA disclosed herein and optionally one or more additional/supplementary active compounds) can be prepared with carriers that will protect the compounds against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Methods for the preparation of such formulations are generally known to those skilled in the art. See, e.g., Sustained and Controlled Release Drug Delivery Systems, J. R. Robinson, ed., Marcel Dekker, Inc., New York, 1978.

To administer the active agent (i.e., the ssRNA disclosed herein and optionally one or more additional/supplementary active compounds) by certain routes of administration, it may be necessary to coat the active agent with, or co-administer the compound with, a material to prevent its inactivation and/or to increase the effectiveness of the active agent (in particular the ssRNA disclosed herein) to be translated. For example, the active agent may be administered to an individual in an appropriate carrier, for example, lipid-containing carriers (in particular cationic lipids), liposomes (such as water-in-oil-in-water CGF emulsions as well as conventional liposomes (Strejan et al., J. Neuroimmunol. 7: 27 (1984)), in particular cationic liposomes), micelles, nanoparticles in which the ssRNA is enclosed or encapsulated, or a diluent. Pharmaceutically acceptable diluents include saline and aqueous buffered solutions.

Pharmaceutical compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), suitable mixtures thereof, vegetable oils, such as olive oil, and injectable organic esters, such as ethyl oleate. The proper fluidity can be maintained, for example, by the use of a coating material such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the pharmaceutical composition. Prolonged absorption of the injectable compositions can be brought about by including in the composition an agent that delays absorption, for example, monostearate salts and gelatin.

Generally, dispersions are prepared by incorporating the active agent into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active agent plus any additional desired ingredient from a previously sterile-filtered solution thereof.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate pharmaceutical compositions in unit dosage form for ease of administration and uniformity of dosage. Unit dosage form as used herein refers to physically discrete units suited as unitary dosages for the individuals to be treated; each unit contains a predetermined quantity of active agent calculated to produce the desired therapeutic effect in association with the required pharmaceutical carrier. The specification for the unit dosage forms disclosed herein are dictated by and directly dependent on (a) the unique characteristics of the active agent and the particular therapeutic effect to be achieved, and (b) the limitations inherent in the art of compounding such an active agent for the treatment of sensitivity in individuals. The amount of active agent (in particular, the amount of ssRNA) which can be combined with a carrier material to produce a pharmaceutical composition (such as a single dosage form) will vary depending upon the individual being treated, and the particular mode of administration. The amount of active agent which can be combined with a carrier material to produce a single dosage form will generally be that amount of the composition which produces a therapeutic effect.

Generally, out of 100% (for the pharmaceutical formulations/compositions), the amount of active agent (in particular, the amount of the ssRNA disclosed herein, optionally together with one or more additional/supplementary active compounds, if present in the pharmaceutical formulations/compositions) will range from about 0.01% to about 99%, preferably from about 0.1% to about 70%, most preferably from about 1% to about 30%, wherein the reminder is preferably composed of the one or more pharmaceutically acceptable excipients.

The amount of active agent, e.g., an ssRNA disclosed herein, in a unit dosage form and/or when administered to an individual or used in therapy, may range from about 0.001 mg to about 1000 mg (for example, from about 0.01 mg to about 500 mg, from about 0.1 mg to about 100 mg such as from about 1 mg to about 50 mg) per unit, administration or therapy. In certain embodiments, a suitable amount of such active agent may be calculated using the mass or body surface area of the individual, including amounts of between about 0.1 mg/kg and 10 mg/kg (such as between about 0.2 mg/kg and 5 mg/kg), or between about 0.1 mg/m² and about 400 mg/m² (such as between about 0.3 mg/m² and about 350 mg/m² or between about 1 mg/m² and about 200 mg/m²).

Regardless of the route of administration selected, the active agents (i.e., the ssRNA and optionally one or more additional/supplementary active compounds), which may be used in a suitable hydrated form, and/or the pharmaceutical compositions disclosed herein, are formulated into pharmaceutically acceptable dosage forms by conventional methods known to those of skill in the art (cf., e.g., Remington, "The Science and Practice of Pharmacy" edited by Allen, Loyd V., Jr., 22nd edition, Pharmaceutical Sciences, September 2012; Ansel et al., "Pharmaceutical Dosage Forms and Drug Delivery Systems", 7th edition, Lippincott Williams & Wilkins Publishers, 1999.).

Actual dosage levels of the active agents in the pharmaceutical compositions disclosed herein may be varied so as to obtain an amount of the active agent which is effective to achieve the desired therapeutic response for a particular patient, composition, and mode of administration, without being toxic to the patient. The selected dosage level will depend upon a variety of pharmacokinetic factors including the activity of the particular compositions disclosed herein employed, the route of administration, the time of administration, the rate of excretion of the particular active agent being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start with doses of the active agents employed in the pharmaceutical composition at levels lower than that required in order to achieve the desired therapeutic effect and gradually increase the dosage until the desired effect is achieved. In general, a suitable daily dose of a pharmaceutical composition disclosed herein will be that amount of the active agent which is the lowest dose effective to produce a therapeutic effect. Such an effective dose will generally depend upon the factors described above. It is preferred that administration be parenteral, such as intravenous, intramuscular, intraperitoneal, or subcutaneous, preferably administered proximal to the site of the target. The administration can also be intra-tumoral. If desired, the effective daily dose of a pharmaceutical composition may be administered as two, three, four, five, six or more sub-doses administered separately at appropriate intervals throughout the day, optionally, in unit dosage forms. While it is possible for an active agent (in particular ssRNA) disclosed herein to be administered alone, it is preferable to administer the active agent as a pharmaceutical formulation/composition.

In one embodiment, the ssRNA or pharmaceutical compositions disclosed herein may be administered by infusion, preferably slow continuous infusion over a long period, such as more than 24 hours, in order to reduce toxic side effects. The administration may also be performed by continuous infusion over a period of from 2 to 24 hours, such as of from 2 to 12 hours. Such regimen may be repeated one or more times as necessary, for example, after 6 months or 12 months.

The pharmaceutical composition disclosed herein can be formulated for parenteral administration by injection, for example, by bolus injection or continuous infusion. Formulations for injection can be presented in units dosage form (e.g., in phial, in multi-dose container), and with an added preservative. The pharmaceutical composition disclosed herein can take such forms as suspensions, solutions or emulsions in oily or aqueous vehicles, and can contain formulatory agents such as suspending, stabilizing, or dispersing agents. Alternatively, the agent can be in powder form for constitution with a suitable vehicle (e.g., sterile pyrogen-free water) before use. Typically, pharmaceutical compositions for intravenous administration are solutions in sterile isotonic aqueous buffer. Where necessary, the pharmaceutical composition can also include a solubilizing agent and a local anesthetic such as lignocaine to ease pain at the site of the injection. Generally, the ingredients are supplied either separately or mixed together in unit dosage form, for example, as a dry lyophilised powder or water free concentrate in a hermetically sealed container such as an ampoule or sachette indicating the quantity of active agent. Where the pharmaceutical composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

Pharmaceutical compositions can be administered with medical devices known in the art. For example, in a preferred embodiment, a pharmaceutical composition disclosed herein can be administered with a needleless hypodermic injection device, such as the devices disclosed in US 5,399,163; US 5,383,851; US 5,312,335; US 5,064,413; US 4,941,880; US 4,790,824; or US 4,596,556. Examples of well-known implants and modules useful in the present disclosure include those described in: US 4,487,603, which discloses an implantable micro-infusion pump for dispensing medication at a controlled rate; US 4,486,194, which discloses a therapeutic device for administering medicants through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,916, which discloses an osmotic drug delivery system.

Many other such implants, delivery systems, and modules are known to those skilled in the art. In certain embodiments, ssRNA or pharmaceutical compositions disclosed herein can be formulated to ensure proper distribution *in vivo.* For example, the blood-brain barrier (BBB) excludes many highly hydrophilic compounds. To ensure that the ssRNA or pharmaceutical compositions disclosed herein cross the BBB (if desired), they can be formulated, for example, in liposomes. For methods of manufacturing liposomes, see, e.g., US 4,522,811; US 5,374,548; and US 5,399,331. The liposomes may comprise one or more moieties which are selectively transported into specific cells or organs, and thus enhance targeted drug delivery (see, e.g., V.V. Ranade (1989) J. Clin. Pharmacol. 29: 685). Exemplary targeting moieties include folate or biotin (see, e.g., US 5,416,016 to Low et al.); mannosides (Umezawa et al., (1988) Biochem. Biophys. Res. Commun. 153: 1038); antibodies (P.G. Bloeman et al. (1995) FEBS Lett. 357: 140; M. Owais et al. (1995) Antimicrob. Agents Chemother. 39: 180); and surfactant protein A receptor (Briscoe et al. (1995) Am. J. Physiol. 1233: 134).

The ssRNA disclosed herein may be formulated in liposomes. In a more preferred embodiment, the liposomes include a targeting moiety. In a most preferred embodiment, the ssRNA in the liposomes is delivered by bolus injection to a site proximal to the desired area. Such liposome-based composition should be fluid to the extent that easy syringability exists, should be stable under the conditions of manufacture and storage and should be preserved against the contaminating action of microorganisms such as bacteria and fungi.

A "therapeutically effective dosage" for treatment can be measured by objective responses which can either be complete or partial. A complete response (CR) is defined as no clinical, radiological or other evidence of a condition, disorder or disease. A partial response (PR) results from a reduction in disease of greater than 50%. Median time to progression is a measure that characterizes the durability of the objective response.

A "therapeutically effective dosage" for treatment can also be measured by its ability to stabilize the progression of a condition, disorder or disease, e.g., by using appropriate animal model systems and/or *in vitro* assays known to the skilled person. A therapeutically effective amount of an active agent refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition. Thus, a therapeutically effective amount of an active agent can cure, heal, alleviate, relieve, alter, remedy, ameliorate, improve or affect the condition, disorder or disease or the symptoms of the condition, disorder or disease or the predisposition toward the condition, disorder or disease in an individual. One of ordinary skill in the art would be able to determine such amounts based on such factors as the disease, disorder or condition to be treated, the severity of the disease, disorder or condition, the parameters of the individual to be treated (including age, physiological condition, size and weight), the duration of treatment, the type of an accompanying therapy (if present), the specific route of administration and similar factors. Accordingly, the doses administered of the active agents described herein may depend on various of such parameters. In the case that a reaction in an individual/patient is insufficient with an initial dose, higher doses (or effectively higher doses achieved by a different, more localized route of administration) may be used.

The pharmaceutical composition disclosed herein may take the form of a vaccine preparation comprising the ssRNA disclosed herein and at least one antigen such as an antigen as discussed above or a fragment thereof (in particular an immunogenic fragment thereof), or a nucleic acid, in particular RNA, encoding said antigen or fragment.

The pharmaceutical composition disclosed herein can also, if desired, be presented in a pack, kit or dispenser device which can contain one or more unit dosage forms containing the active agent (i.e., the ssRNA and optionally one or more additional/supplementary active compounds). The pack can for example comprise metal or plastic foil, such as blister pack. The pack, kit or dispenser device can be accompanied with instruction for administration.

The one or more additional/supplementary active compounds may comprise an immunomodulating agent such as anti-CTL-A4 or anti-PDl or anti-PDLl or anti-regulatory T-cell reagents such as an anti-CD25 antibody or cyclophosphamide.

The pharmaceutical compositions disclosed herein may be administered together with supplementing immunity-enhancing substances such as one or more adjuvants and may comprise one or more immunity-enhancing substances to further increase its effectiveness, preferably to achieve a synergistic effect of immunostimulation.

The term "adjuvant" relates to compounds which prolong or enhance or accelerate an immune response. Various mechanisms are possible in this respect, depending on the various types of adjuvants. For example, compounds which allow the maturation of the DC, e.g. lipopolysaccharides or CD40 ligand, form a first class of suitable adjuvants. Generally, any agent which influences the immune system of the type of a "danger signal" (LPS, GP96, dsRNA etc.) or cytokines, such as GM-CSF, can be used as an adjuvant which enables an immune response to be intensified and/or influenced in a controlled manner. CpG oligodeoxynucleotides can optionally also be used in this context, although their side effects which occur under certain circumstances, as explained above, are to be considered. In case the ssRNA (preferably mRNA) disclosed herein in one embodiment may encode an immunostimulating agent and said immunostimulating agent encoded by said ssRNA is to act as the primary immunostimulant, however, only a relatively small amount of CpG DNA is necessary (compared with immunostimulation with only CpG DNA). Particularly preferred adjuvants are cytokines, such as monokines, lymphokines, interleukins or chemokines, e.g. IL-1, IL-2, IL-3, IL-4, IL-5, IL-6, IL-7, IL-8, IL-9, IL-10, IL-12, IFN-α, IFN-γ, GM-CSF, LT-α, or growth factors, e.g. hGH. Lipopeptides, such as Pam3Cys, are also suitable for use as adjuvants in the pharmaceutical compositions disclosed herein.

Treatment may be provided at home, the doctor's office, a clinic, a hospital's outpatient department, or a hospital. Treatment generally begins under medical supervision so that medical personnel can observe the treatment's effects closely and make any adjustments that are needed. The duration of the treatment depends on the age and condition of the patient, as well as how the patient responds to the treatment.

A person having a greater risk of developing a condition, disorder or disease may receive prophylactic treatment to inhibit or delay symptoms of the condition, disorder or disease.

The term "treatment" is known to the person of ordinary skill, and includes the application or administration of an active agent (e.g., a pharmaceutical composition containing said active agent) or procedure to an individual/patient or application or administration of an active agent (e.g., a pharmaceutical composition containing said active agent) or procedure to a cell, cell culture, cell line, sample, tissue or organ isolated from a subject, who has a condition, disorder or disease, a symptom of the condition, disorder or disease or a predisposition toward a condition, disorder or disease, with the purpose to cure, heal, alleviate, relieve, alter, remedy, ameliorate, improve, affect or prevent the condition, disorder or disease, the symptoms of the condition, disorder or disease or the predisposition toward the condition, disorder or disease (e.g., to prevent or eliminate a disease, including reducing the size of a tumor or the number of tumors in a subject; arrest or slow a disease in a subject; inhibit or slow the development of a new disease in a subject; decrease the frequency or severity of symptoms and/or recurrences in a subject who currently has or who previously has had a disease; and/or prolong, i.e. increase the lifespan of the subject). In particular, the term "treatment of a disease" includes curing, shortening the duration, ameliorating, preventing, slowing down or inhibiting progression or worsening, or preventing or delaying the onset of a disease or the symptoms thereof. Hence, the term "treatment" can include prophylactic treatment of a condition, disorder or disease, or the symptom of a condition, disorder or disease. An active agent, when used in treatment, includes the ssRNA disclosed herein as well as the one or more additional/supplementary active compounds described herein and includes, but is not limited to, other therapeutically active compounds that may be small molecules, peptides, peptidomimetics, polypeptides/proteins, antibodies, other polynucleotides such as DNA or dsRNA, cells, viruses, ribozymes, and antisense oligonucleotides.

The present invention is illustrated by the following examples which illustrate preferred embodiments of the invention and should not be interpreted to limit the scope of the present invention as defined in the claims. Those examples which are not covered by the appending claims are given for comparative purposes only.

### EXAMPLES

### Abbreviations

- EtOH:: ethanol
- h:: hour(s)
- hPa:: hectopascal
- min:: minute(s)
- mM:: millimolar (10⁻³ mol/1)
- MPa:: megapascal
- nt:: nucleotide(s)
- sec:: second(s)
- v/v:: volume %

### Experimental Procedures

### Cellulose purification of IVT RNA

Unless otherwise indicated cellulose powder consisting of medium size fibers (Sigma-Aldrich, Cat. #C6288) and 1x STE buffer (10 mM TRIS, pH 7.0, 50 mM NaCl, 20 mM EDTA) was used for the purification procedures. All experiments were performed at room temperature.

### "Negative" purification procedure

The "negative" purification procedure is based on the incubation of IVT RNA with cellulose in 1x STE buffer containing 16% EtOH. This condition allows the selective binding of dsRNA to cellulose while ssRNA remains in the soluble fraction.

Cellulose was first suspended in 1x STE buffer containing 16% (v/v) EtOH at a concentration of 0.2 g cellulose/ml and incubated for 10 min under vigorous shaking. After centrifugation for 5 min at 4,000 x g the cellulose was resuspended in 1x STE buffer containing 16% (v/v) EtOH at a concentration of 0.2 g cellulose/ml (washed cellulose).

For pull-down experiments 500 µl of washed cellulose slurry was transferred to a 1.5 ml tube and centrifuged for 5 min at 14,000 x g. After removal of the supernatant 50 µg IVT RNA in 100 µl 1x STE buffer containing 16% (v/v) EtOH was added to the cellulose and incubated for 15 min under vigorous shaking. After centrifugation for 5 min at 14,000 x g the supernatant was removed and the nucleic acids were precipitated. The cellulose was then incubated for 15 min with 100 µl 1x STE buffer containing no EtOH under vigorous shaking to release the bound nucleic acids. After centrifugation for 5 min at 14,000 x g the supernatant was removed and the eluted nucleic acids were precipitated.

For cellulose purification using microcentrifuge spin columns (NucleoSpin Filters, Macherey-Nagel, Cat. #740606) 600 µl of pre-washed cellulose slurry (0.12 g cellulose) was transferred to a spin column and centrifuged for 60 sec at 14,000 x g. The flow through was discarded and 500 µl of 1x STE buffer containing 16% (v/v) EtOH was added to the spin column and incubated for 5 min under vigorous shaking to resuspend the cellulose. After centrifugation for 60 sec at 14,000 x g the flow through was discarded and IVT RNA (50-500 µg) in 300-500 µl 1x STE buffer containing 16% (v/v) EtOH was added to the spin column and incubated for 20 min under vigorous shaking to resuspend the cellulose. The spin column was then centrifuged for 60 sec at 14,000 x g and the flow through collected for nucleic acid precipitation. When multiple cycles of cellulose purification were performed, the flow through was directly transferred to a freshly prepared cellulose spin column and the procedure was repeated. Finally, by adding 300-500 µl 1x STE buffer the cellulose-bound nucleic acids were released during incubation for 20 min under vigorous shaking and centrifugation of the spin column for 60 sec at 14,000 x g.

Upscaling of the purification process was performed in 50 ml tubes using 1.5 g cellulose and 5 mg of IVT RNA in 15 ml 1x STE buffer containing 16% (v/v) EtOH. The RNA was added to the dry cellulose and incubated under magnetic stirring for 30 min. Unbound nucleic acids were recovered by filtration using a disposable vacuum-driven filter device (Steriflip-HV, 0.45 µm pore size, PVDF, Merck Chemicals GmbH/ Millipore, Cat. #SE1M003M00). Where indicated the filtrate was used for a second cycle of purification by adding 1.5 g fresh cellulose and repeating the process. Finally, the nucleic acids in the filtrate were precipitated by adding an equal volume of isopropanol.

### "Positive" purification procedure

The principle of the "positive" purification procedure is to bind first all RNA to cellulose by incubation of IVT RNA with cellulose in 1x STE buffer containing 40% EtOH. In a second step, ssRNA is selectively released by incubation in 1x STE buffer containing 16% EtOH while under these conditions dsRNA remains bound to the cellulose fibers.

Before use cellulose was suspended in 1x STE buffer containing 40% (v/v) EtOH at a concentration of 0.2 g cellulose/ml and incubated for 10 min under vigorous shaking. After centrifugation for 5 min at 4,000 x g the cellulose was resuspended in 1x STE buffer containing 40% (v/v) EtOH at a concentration of 0.2 g cellulose/ml (washed cellulose).

For cellulose purification using microcentrifuge spin columns (NucleoSpin Filters, Macherey-Nagel, Cat. #740606) 600 µl of washed cellulose slurry (0.12 g cellulose) was transferred to a spin column and centrifuged for 60 sec at 14,000 x g. The flow through was discarded and 500 µl of 1x STE buffer containing 40% (v/v) EtOH was added to the spin column and incubated for 5 min under vigorous shaking to resuspend the cellulose. After centrifugation at 14,000 x g for 60 sec the flow through was discarded and IVT RNA (50-500 µg) in 300-500 µl 1x STE buffer containing 40% (v/v) EtOH was added to the spin column and incubated for 20 min under vigorous shaking to resuspend the cellulose. The spin column was then centrifuged at 14,000 x g for 60 sec and the flow through collected for nucleic acid precipitation. By adding 300-500 µl 1x STE buffer containing 16% (v/v) EtOH ssRNA was released from the cellulose during incubation for 20 min under vigorous shaking and centrifugation of the spin column for 60 sec at 14,000 x g. When multiple cycles of cellulose purification were performed, the flow through was directly transferred to a freshly prepared cellulose spin column and the procedure repeated. Finally, by adding 300-500 µl 1x STE buffer the cellulose-bound nucleic acids were released during incubation for 20 min under vigorous shaking and centrifugation of the spin column for 60 sec at 14,000 x g.

For FPLC using cellulose as stationary phase, first a cellulose slurry (0.2 g/ml) in 1x STE buffer containing 40% (v/v) EtOH was prepared and stirred for 30 min. 20 ml of this slurry (4 g cellulose) were used to pack a XK 16/20 column (GE Healthcare Life Sciences, Cat #28-9889-37). The column bed had a final height of about 5 cm. Chromatography was performed using an AKTA Avant 25 system (GE Healthcare Life Sciences) and monitoring the UV (260 nm) absorbance. As binding buffer 1x STE buffer containing 40% (v/v) EtOH (buffer B) and as elution buffer 1x STE buffer (buffer A) was used. The column was equilibrated for 15 min with 100% buffer B at a flow rate of 2 ml/min. After injection of 500 µg RNA (sample volume: 500 µl) the flow rate was reduced to 1 ml/min for 40 min. The ssRNA was eluted using 40% buffer B (16% (v/v) EtOH) for 40 min at a flow rate of 2 ml/min. Finally, by changing the buffer composition to 0% buffer B (0% EtOH) the dsRNA was eluted from the column. The chromatographic peaks were collected and the nucleic acids precipitated for further analysis.

### Isopropanol precipitation of nucleic acids

The RNA obtained from cellulose purifications was precipitated by adding 0.1 volumes of 3 M sodium acetate (pH 4.0) and 1 volume of isopropanol. After vortexing the samples were incubated for 1 h at -20°C followed by centrifugation for 10 min at 14,000 x g. The RNA pellet was washed with 200 µl of ice-cold 70% (v/v) EtOH, air-dried and dissolved in a suitable volume of nuclease-free H₂O. RNA concentrations were measured spectrophotometrically using the Nanodrop system (Eppendorf).

### Dot blot analysis

To determine the amount of dsRNA and RNA-DNA hybrid contaminants serial dilutions of RNA samples with different concentrations were prepared and increasing amounts of RNA (usually 40 ng, 200 ng and 1,000 ng) were spotted (0.5 µl) onto a nylon blotting membrane (Nytran SuPerCharge (SPC) Nylon Blotting Membrane (GE Healthcare Life Sciences, Cat. #10416216)). The membrane was then blocked for 1h in TBS-T buffer (20 mM TRIS pH 7.4, 137 mM NaCl, 0.1% (v/v) TWEEN-20) containing 5% (w/v) skim milk powder. For detection of dsRNA the membrane was incubated for 1h with J2 dsRNA-specific mouse mAb (English & Scientific Consulting, Szirák, Hungary) diluted at a ratio of 1:10,000 in TBS-T buffer containing 1% (w/v) skim milk powder. Where indicated S 9.6 RNA-DNA-hybrid-specific mouse mAb IgG2a (KeraFAST, Cat. #ENH001) diluted at a ratio of 1:10,000 was used to detect RNA-DNA hybrid contaminants. After washing with TBS-T the membrane was incubated for 1h with HRP-conjugated donkey anti-mouse IgG (Jackson ImmunoResearch, Cat. #715-035-150) diluted at a ratio of 1:10,000 in TBS-T buffer containing 1% (w/v) skim milk powder, washed with TBS-T and developed using Amersham ECL Prime Western Blotting Detection Reagent (Fisher Scientific, Cat. # RPN2232) and the ChemiDoc MP Imaging system (BIO-RAD). Where indicated hybridization signal intensities were quantitated by densitometry using Image Lab 5.1 software (BIO-RAD).

### Agarose gel electrophoresis

To monitor the integrity of purified RNAs and to verify the amounts loaded onto the dot blots agarose gel electrophoresis was used. Equal amounts of RNA (e.g., 2 µl) of the 40 ng/µl serial RNA dilution prepared for dot blotting were analyzed. The RNA samples were denatured according to Masek et al. (Anal. Biochem. 336 (2005), 46-50) by mixing 2 µl of RNA (80 ng) with 6 µl of formamide and incubating for 5 min at 65°C before loading onto a 1.4% (w/v) agarose gel containing 0.005% (v/v) GelRed^{™} Nucleic Acid Gel Stain (Biotium Inc., Cat. #41003). Electrophoresis was performed at 100 V for 20 min using TAE (40 mM TRIS acetate, 1 mM EDTA) as running buffer followed by imaging of the gel using the Gel Doc^{™} EZ Imager system (BIO-RAD).

### Example 1 - Pull-down of dsRNA from IVT RNA using cellulose

To test the feasibility of applying cellulose to remove dsRNA contaminants from IVT RNA first a simple pull-down experiment was performed. 50 µg of a 2,500 nt-long N¹-methyl-pseudouridine (m1Ψ)-modified IVT RNA, that was pre-purified by lithium chloride (LiCl) precipitation from the IVT reaction, was incubated with 0.1 g cellulose in the presence of 1x STE buffer containing 16% (v/v) EtOH. After centrifugation the unbound RNA in the supernatant was precipitated. Cellulose-bound RNA was recovered by resuspension of the cellulose in 1x STE containing no EtOH, centrifugation and precipitation of the supernatant. The dsRNA content of RNA from both fractions as well as of the starting RNA material was analyzed by dot blot using the dsRNA-specific J2 antibody. Integrity of the RNAs was monitored by agarose gel electrophoresis.

Dot blot analysis shows that compared to the untreated input IVT RNA the dsRNA content in the unbound RNA fraction after incubation with cellulose is strongly reduced (Fig. 1). This is due to the selective binding of contaminating dsRNA to the cellulose material in the presence of 16% (v/v) EtOH that allows the separation of dsRNA from ssRNA by sedimentation of the cellulose. After separation the dsRNA contaminants can be released from the cellulose using a buffer that does not contain EtOH. This is confirmed by demonstrating significant amounts of J2 reactive RNA in the bound RNA fraction (Fig. 1). Furthermore, electrophoresis of the RNA demonstrates that the RNA integrity is preserved during this cellulose purification procedure. This example demonstrates the successful use of cellulose to remove dsRNA contaminants from IVT RNA.

### Example 2 - Impact of different concentrations of EtOH on the efficiency of dsRNA removal from IVT RNA by cellulose

In a next step the above described purification method (cf. Example 1) was adapted for using microcentrifuge spin columns to separate unbound RNA from cellulose. The advantage of this technique is the complete removal of liquid and thus unbound RNA from cellulose by centrifugation. Further, it was tested whether increasing the EtOH concentration during incubation of the IVT RNA with cellulose up to 18% (v/v) or 20% (v/v) will increase the efficiency of dsRNA removal. First, 50 µg of 1,500 nt-long pseudouridine (Ψ)-modified and D2-capped IVT RNA, that was pre-purified from the IVT reaction by magnetic beads, was incubated in a microcentrifuge spin column with 0.1 g cellulose in the presence of 1x STE buffer containing 16% (v/v), 18% (v/v) or 20% (v/v) EtOH. After centrifugation the unbound RNA was collected by centrifugation of the column and precipitated. Cellulose-bound RNA was recovered by adding 1x STE containing no EtOH to the column, resuspension of the cellulose by vigorous shaking and finally centrifugation and precipitation. The dsRNA content of RNA from both fractions as well as of the starting RNA material was analyzed by dot blot using the dsRNA-specific J2 antibody. A second membrane was loaded with the same amounts of the different RNA fractions and hybridized with the RNA/DNA hybrid-specific S 9.6 antibody to test whether these IVT RNA contaminants can also be removed by cellulose purification.

Compared to the unpurified IVT RNA the dsRNA content in all unbound RNA fractions (flow through) is strongly reduced after incubation with cellulose (Fig. 2). The amount of RNA/DNA hybrids in these fractions, however, is only slightly decreased demonstrating that RNA/DNA hybrids do not bind efficiently to cellulose under the tested conditions and thus cannot be removed from IVT RNA by using cellulose. Increasing the EtOH concentration from 16% (v/v) to 18% (v/v) or 20% (v/v) does not significantly increase the efficiency of dsRNA removal. The high amounts of J2-reactive RNA in the bound RNA fractions indicate the enrichment of dsRNA (Fig. 2), confirming the separation of dsRNA contaminants and ssRNA by this method. This result further shows the successful adaption of the "negative" cellulose purification procedure to a microcentrifuge spin column format.

### Example 3 - Comparison of cellulose purification to RNaseIII treatment and HPLC purification

To test whether multiple cycles of cellulose purification according to the above described method (cf. Example 2) using microcentrifuge spin columns enhances the efficiency of dsRNA removal, 100 µg of 2,500 nt-long m1Ψ-modified IVT RNA, that was pre-purified by lithium chloride (LiCl) precipitation from the IVT reaction, was purified 1x, 2x or 3x as described above using 1x STE buffer containing 16% (v/v) EtOH. Further, the RNAs purified by cellulose were compared to IVT RNA that was either treated with *E. coli* RNaseIII (0.2 U/100 µg RNA) for 30 min at 37°C or purified by HPLC according to the protocol described by Weissman et al. (supra). The dsRNA content of all RNAs was analyzed by dot blot using the dsRNA-specific J2 antibody and quantitated by densitometric analysis of the hybridization signals. RNA integrity was monitored by agarose gel electrophoresis.

Increasing the number of cellulose purification cycles increases the amount of dsRNA removed from IVT RNA (Fig. 3). While one cycle of purification removes about 90% of dsRNA contaminants, this amount is increased up to 95% and 97%, when performing 2 and 3 purification cycles, respectively. Interestingly, one cycle of cellulose purification eliminates nearly the same amount of dsRNA as the treatment of IVT RNA with RNaseIII. Furthermore, conducting 3 cycles of cellulose purification comes very close to the efficiency of the HPLC purification. Thus, the efficiency of cellulose purification ranges between that of RNaselll treatment and HPLC purification.

### Example 4 - Comparison of the performance of different brands of cellulose in removing dsRNA from IVT RNA

To test whether removal of dsRNA contaminants is restricted to a specific cellulose brand used in the above described experiment (Sigma-Aldrich, Cat. #C6288) or whether cellulose from other suppliers can also be used, we tested the performance of two other cellulose types from Macherey-Nagel (MN 100, MN 2100). First, 100 µg of 1,500 nt-long m1Ψ-modified IVT RNA, that was pre-purified by lithium chloride (LiCl) precipitation from the IVT reaction, was incubated in a microcentrifuge spin column with 0.15 g of the different types of cellulose in the presence of 1x STE buffer containing 16% (v/v) EtOH. After centrifugation the unbound RNA was collected by centrifuging the column and precipitating the RNA in the flow through. Cellulose-bound RNA was recovered by adding 1x STE to the column, followed by resuspension of the cellulose by vigorous shaking and finally centrifugation and precipitation. The dsRNA content of all RNA samples was analyzed by dot blot using the dsRNA-specific J2 antibody and quantitated by densitometric analysis of the hybridization signals. RNA integrity was monitored by agarose gel electrophoresis.

Independent of the cellulose used for purification the dsRNA content in all unbound RNA fractions is strongly reduced compared to the unpurified input RNA (Fig. 4). The high amounts of J2-reactive RNA in the bound RNA fractions indicate the enrichment of dsRNA confirming the separation of dsRNA contaminants and ssRNA by all cellulose types tested.

### Example 5 - Scalability of the cellulose purification method

An important point was to test whether the cellulose purification method is scalable. Therefore, an experiment was performed to remove dsRNA contaminants from 5 mg of 1,900 nt-long D1-capped IVT RNA, that was pre-purified by from the IVT reaction by magnetic beads. The RNA was incubated with 1.5 g of cellulose (Sigma, Cat. #C6288) in 15 ml of 1x STE buffer containing 16% (v/v) EtOH. The unbound RNA was separated from the cellulose by a vacuum-driven filter device (0.45 µM pore size) and precipitated. With another 5 mg of the same RNA 2 purification cycles were performed. The dsRNA content of both purified RNAs was analyzed by dot blot using the dsRNA-specific J2 antibody and quantitated by densitometric analysis of the intensities of the hybridization signals. RNA integrity was monitored by agarose gel electrophoresis.

The result of the dot blot analysis shows that after 1 cycle of purification with 1.5 g cellulose 72% of dsRNA contaminants is removed from 5 mg of IVT RNA. The purification efficiency can be further increased up to 83% by a second purification cycle with 1.5 g of fresh cellulose. As expected, the rate of RNA recovery decreases from 67% after 1 cycle of purification to 53% after the second cycle, which is still acceptable and is comparable to the recovery rate of about 50% achieved by the HPLC purification protocol described by Weismann et al. (supra). This result clearly demonstrates that the cellulose purification method of the present invention can be upscaled to remove dsRNA contaminants from several mg of IVT RNA in one single batch purification.

### Example 6 - Purification of IVT RNA with different length using a "positive" purification procedure

In a next step it was tested whether it is feasible to first bind all RNA components of an IVT RNA preparation to cellulose in the presence of high EtOH concentrations before selectively releasing the ssRNA fraction by decreasing the EtOH concentration to 16% (v/v). Under this condition dsRNA contaminants should stay bound to the cellulose material and thus be separated from ssRNA ("positive" purification). This procedure would be advantageous over the "negative" purification since it would also allow the removal of non-nucleic acid contaminants (e.g. proteins, free nucleotides), which do not bind to the cellulose in the presence of EtOH. Therefore, experiments were performed in which 400 µg of three IVT RNAs with different lengths (1,300 nt, 2,500 nt and >10,000 nt) and cap structures (D1, D2, no cap) were bound completely to 0.12 g cellulose in a microcentrifuge spin column using 1x STE buffer containing 40% (v/v) EtOH. The ssRNAs were eluted with 1x STE buffer containing 16% (v/v) EtOH and transferred to a second spin column containing 1.2 mg fresh cellulose. After incubation under vigorous shaking and after centrifugation, the RNAs in the flow through were precipitated and analyzed for dsRNA contaminants by dot blotting using the dsRNA-specific J2 antibody. RNA integrities were monitored by agarose gel electrophoresis.

Independent from the RNA length the dsRNA content of all IVT RNAs is reduced significantly to a barely detectable level after cellulose purification (Fig. 6) demonstrating the feasibility of the above described "positive" purification procedure. Unexpectedly, also the >10,000 nt long IVT RNA could be successfully purified from dsRNA contaminants (Fig. 6A). This shows that purification is not restricted to shorter RNA (such as 1,300 - 2,500 nt) and suggests that RNA length is not a limiting factor for successful purification. However, compared to the recovery rate of both, 1,300 nt and 2,500 nt-long RNAs (45-55% recovery) the recovery rate of the long IVT RNA is lower (35% recovery). Although the integrity of the > 10,000 nt-long IVT RNA is lower than that of both shorter IVT RNAs, it is not negatively affected by the cellulose purification method according to the present invention. Since the RNAs used for these experiments carried different 5' cap structures (10,000 nt: D1 cap, 1,300 nt: D2 cap, 2,500 nt: no cap) said examples demonstrate that this structural feature is not a critical factor for the successful purification of IVT RNA by cellulose.

### Example 7 - Purification of IVT RNA using buffers with different ionic strength

The stability of double-stranded nucleic acids is influenced by the ionic strength of the environment. While high salt concentrations promote the formation of double-stranded structures, their dissociation to single stranded nucleic acids is enhanced under low salt concentrations. To analyze the impact of the buffer's ionic strength on the efficiency of dsRNA removal by cellulose a 1,300 nt-long m1Ψ-modified IVT RNA, that was pre-purified by lithium chloride (LiCl) precipitation from the IVT reaction, was incubated in a 1.5 ml tube with 0.1 g of cellulose in 500 µl 1x STE buffer containing 40% (v/v) EtOH and different concentrations of NaCl (0-150 mM). After centrifugation the supernatant was removed and the cellulose resuspended in 500 µl of corresponding 1x STE buffers containing 16% (v/v) EtOH to release the ssRNA. After centrifugation the supernatant was collected and the RNA recovered by precipitation. In a final step the cellulose was resuspended in 500 µl of the corresponding 1x STE buffers containing no EtOH, centrifuged and the RNA recovered by precipitation of the supernatant. The dsRNA content of RNA from both fractions (16% EtOH eluate, 0% EtOH eluate) as well as of the starting RNA material (IVT RNA) was analyzed by dot blot using the dsRNA-specific J2 antibody and quantitated by densitometric analysis of the hybridization signals. Integrity of the RNAs was monitored by agarose gel electrophoresis.

The efficiency of dsRNA removal by cellulose is influenced by the NaCl concentration in the STE buffer. In the presence of 25 mM or 50 mM NaCl 94%-98% of the dsRNA contaminants are eliminated from the 16% EtOH eluate (Fig. 7A, B). Either increasing the NaCl concentration to 75 mM (Fig. 7A) or decreasing it to 10 mM (Fig. 7B) reduces the efficiency of purification. NaCl concentrations above 125 mM lead to a significant decrease in efficiency of purification (Fig. 7A). This result demonstrates that the NaCl concentration of the STE buffer used can influence the purification efficiency which is highest in a range between 25 and 50 mM NaCl. The strong reactivity of all of the 0% EtOH eluates, except for the 150 mM NaCl sample (Fig. 7A), with J2 antibody reflects the enrichment of dsRNA contaminants in these samples.

### Example 8 - Cellulose purification of IVT RNA by FPLC

Since separation of ssRNA from dsRNA contaminants by cellulose using a "positive" purification procedure is feasible (cf. Examples 6 and 7) it was tried to adapt the purification protocol for FPLC. 4 g of cellulose was used as stationary phase to pack a XK 16/20 column. After equilibration with 1x STE buffer containing 40% (v/v) EtOH 500 µg of 1,300 nt-long m1Ψ-modified IVT RNA, that was pre-purified by lithium chloride (LiCl) precipitation from the IVT reaction, was loaded onto the column. The bound ssRNA and dsRNA were eluted by reducing the EtOH concentration of the buffer to 16% (v/v) and 0% (v/v), respectively, and the fractions were collected. The RNA was recovered by precipitation and the dsRNA content of RNA from both fractions (F1: 16% EtOH eluate, F2: 0% EtOH eluate) as well as the starting RNA material (input RNA) was analyzed by dot blot using the dsRNA-specific J2 antibody. Integrity of the RNAs was monitored by agarose gel electrophoresis.

The elution profile (absorbance at 260 nm) of the chromatogram shows that a high percentage of the loaded IVT RNA binds to the cellulose material in the presence of 40% (v/v) EtOH. Only minor amounts of RNA remain unbound and elute from the column under these conditions (Fig. 8A). Upon decreasing the EtOH concentration to 16% (v/v) most of the RNA elutes indicated by a sharp single peak in the UV absorbance. This peak was collected (fraction F1) and contains the purified ssRNA. Compared to the unpurified input RNA about 88% of the dsRNA content was removed from this fraction (Fig. 8B). After reducing the EtOH concentration of the buffer further to 0% (v/v) only minor amounts of RNA elute from the column (fraction F2). The dot blot analysis revealed that dsRNA is enriched in this RNA fraction (Fig. 8B). This confirms the separation of ssRNA from dsRNA and demonstrates the successful use of cellulose as stationary phase for FPLC purification of IVT RNA to remove dsRNA contaminants.

### Example 9 - Purification of IVT RNA using different EtOH concentrations for ssRNA elution

To optimize the protocol of the "positive" cellulose purification procedure the impact of different EtOH concentrations on the efficiency of dsRNA removal and RNA recovery was tested. 200 µg of 1,500 nt-long D1-capped IVT RNA, that was pre-purified from the IVT reaction by magnetic beads, was incubated with 0.1 g of washed cellulose in 500 µl 1x STE buffer containing 40% (v/v) EtOH in a microcentrifuge spin column. The cellulose-bound ssRNA was eluted with 1x STE buffer containing 6, 10, 12, 14, 16, 18, 20 or 24% (v/v) EtOH and recovered from the eluate by precipitation. The dsRNA content of RNA obtained from the different eluates as well as of the starting RNA material (input RNA) was analyzed by dot blot using the dsRNA-specific J2 antibody and quantitated by densitometric analysis of the hybridization signals. Integrity of the RNAs was confirmed by agarose gel electrophoresis.

The optimal range of EtOH concentrations for elution of ssRNA during a "positive" purification procedure is 14-16% (v/v) (Fig. 9A, B). At these EtOH concentrations 83-84% of dsRNA remained bound to the cellulose and 58-64% of the ssRNA is recovered from the corresponding eluates. Increasing the EtOH to 18% (v/v) or 20% (v/v) further improves the efficiency of dsRNA removal (85% or 90%, respectively) but significantly reduces the RNA recovery rate (47% or 36%, respectively). In contrast, decreasing the EtOH concentration to 12% (v/v) worsens the purification efficiency (63% of dsRNA removed) without significantly improving the RNA recovery. This result demonstrates that the efficiency of dsRNA removal and the recovery rate of RNA from the eluates correlate inversely. Further, the relative purity of the ssRNA with regard to dsRNA contaminants can be controlled by the EtOH concentration used for elution. Higher EtOH concentrations lead to a more efficient removal of dsRNA, however, at the cost of a reduced ssRNA recovery. Therefore, by adjusting the EtOH concentration for elution of ssRNA, the dsRNA contaminants/RNA recovery ratio can be adjusted to meet the purity/cost requirements of IVT RNA for different applications.

### Example 10 - Determination of the RNA binding capacity of cellulose

For upscaling the "positive" cellulose purification procedure it is important to know the RNA-binding capacity of the cellulose to minimize RNA loss caused by overloading. To determine the RNA-binding capacity we incubated a fixed amount of washed cellulose (100 mg, Sigma, C6288) with 25 µg, 50 µg, 100 µg, 250 µg, 500 µg, 750 µg, 1,000 µg or 1,500 µg of 1,500 nt-long D1-capped IVT RNA, that was pre-purified from the IVT reaction by magnetic beads, in 500 µl 1x STE buffer containing 40% (v/v) EtOH in a microcentrifuge spin column. After centrifugation the ssRNA was eluted with 500 µl 1x STE buffer containing 16% (v/v) EtOH. Finally, the RNA that was still bound to the cellulose was released by incubation with H₂O (0% (v/v) EtOH). The RNA in the flow through (40% (v/v) EtOH) and both, the 16% (v/v) and 0% (v/v) eluate were recovered by precipitation and the amount of recovered RNA determined by spectrophotometry. In addition, the dsRNA content of RNA obtained from the 16% (v/v) EtOH eluates as well as of the starting RNA material (input RNA) was analyzed by dot blot using the dsRNA-specific J2 antibody to monitor the efficiency of dsRNA removal in the individual samples. Integrity of these RNAs was monitored by agarose gel electrophoresis.

The maximum RNA binding capacity of the cellulose tested (Sigma, C6288) ranges between 100 µg and 250 µg RNA per 100 mg cellulose which corresponds to 1-2.5 mg RNA per 1 g of cellulose. This is reflected by the fact that the RNA recovery rate (Fig. 10A) and the yield of RNA (Fig. 10B) recovered from the 40% (v/v) EtOH flow through, which represents the unbound RNA fraction, steadily increases when 250 µg or more RNA is used for purification with 100 mg cellulose. The amount of bound RNA in the 16% (v/v) EtOH and the 0% (v/v) EtOH eluates, however, does not increase accordingly when 250 µg or more RNA is used for purification, which consequently leads to a decreased RNA recovery rate from both fractions (Fig. 10A, B). The maximum RNA recovery rate from the 16% (v/v) EtOH eluate is achieved when 100 µg of RNA were used for purification with 100 mg cellulose (68% RNA recovery). Further, at this ratio of RNA:cellulose the highest purification efficiency of 88% dsRNA removal is reached (Fig. 10C, D). Interestingly, the relative amount of dsRNA removed from IVT RNA does not significantly decrease when the binding RNA-capacity of the cellulose is exceeded.

### Example 11 - Impact of cellulose purification of IVT RNA on its translatability and immunogenicity

As set forth above, IVT RNA contains dsRNA contaminants due to aberrant activity of T7 RNA polymerase. However, dsRNA induces inflammatory cytokines (such as interferon) by activating different cellular sensors, including RIG-I, MDA5 and TLR3, and also inhibits translation directly by activating protein kinase R (PKR) and oligoadenylate synthetase (OAS). To test whether IVT RNA subjected to a method of the present invention induces less inflammatory cytokines and/or can be translated more efficiently compared to IVT RNA which has not been subjected to a method of the present invention, IVT RNA encoding murine erythropoietin (EPO) was either left unpurified or was purified by a 2-step procedure using 2 spin columns each filled with a cellulose material (0.12 g cellulose (Sigma, C6288)). First, the IVT RNA was subjected to a positive purification procedure as described above using the 1^{st} spin column (i.e., incubating the IVT RNA with the cellulose material in the 1^{st} spin column and in the presence of 1x STE buffer containing 40% (v/v) EtOH for binding of dsRNA and ssRNA to the cellulose material; applying centrifugal force to the 1^{st} spin column; discarding the flow through; eluting the ssRNA from the 1^{st} spin column by adding 1x STE buffer containing 16% (v/v) EtOH and applying centrifugal force to the 1^{st} spin column). Then, the thus obtained eluate containing ssRNA was subjected to a negative purification procedure as described above using the 2^{nd} spin column (i.e., incubating the eluate containing the ssRNA with the cellulose material in the 2^{nd} spin column; applying centrifugal force to the 2^{nd} spin column; and collecting the flow through). The flow through obtained from the 2^{nd} spin column was then precipitated with isopropanol/sodium acetate and redissolved in H₂O. Following formulation with TransIT (Mirus Bio) the IVT RNAs were injected intraperitoneally into mice (n=4) at a dose of 3 µg RNA/animal. Blood was withdrawn at 2, 6 and 24 h postinjection and plasma samples were collected. Control mice were injected with TransIT only. Levels of murine interferon alpha (IFN-α) and murine EPO were measured using specific ELISA assays (murine interferon alpha-specific ELISA (eBioscience); murine EPO-specific DuoSet ELISA Development kit (R&D)).

As shown in Fig. 11A, the IVT RNA subjected to a method of the present invention induced significantly less IFN-α compared to unpurified IVT RNA. Thus, this example demonstrates that the method of the present invention efficiently removes contaminating double-stranded molecules from the IVT RNA. Most likely the residual IFN-α induced by the cellulose-purified IVT RNA was due to activation of TLR7 by the ssRNA that contains uridines.

Furthermore, Fig. 11B shows that an EPO-encoding IVT RNA preparation which has been subjected to a method of the present invention, thus lacking protein synthesis inhibitory dsRNA, translated very efficiently, resulting in high EPO levels in the plasma even 24 h after administration of the IVT RNA purified according to the present invention. In contrast, an EPO-encoding IVT RNA preparation which has not been subjected to a method of the present invention but which was left unpurified translated less efficiently due to protein synthesis inhibition by direct and IFN-α-mediated effects of dsRNA.

## Claims

1. A method for providing single-stranded RNA (ssRNA), comprising:
(i) producing an RNA preparation comprising ssRNA by *in vitro* transcription;
(ii) contacting the RNA preparation with a cellulose material under conditions which allow binding of double-stranded RNA (dsRNA) to the cellulose material and do not allow binding of ssRNA to the cellulose material; and
(iii) separating the ssRNA from the cellulose material under conditions which allow binding of dsRNA to the cellulose material and do not allow binding of ssRNA to the cellulose material,
wherein
in step (ii) the RNA preparation is provided as a liquid comprising ssRNA and a first buffer and/or the cellulose material is provided as a suspension in a first buffer, wherein the first buffer comprises water, ethanol and a salt in a concentration which allows binding of dsRNA to the cellulose material and which does not allow binding of ssRNA to the cellulose material; and
the concentration of ethanol in the first buffer is 14 to 20% (v/v), the concentration of the salt in the first buffer is 15 to 70 mM and the first buffer comprises tris(hydroxymethyl)aminomethane (TRIS) as a buffering substance.

2. A method for providing single-stranded RNA (ssRNA), comprising:
(i) producing an RNA preparation comprising ssRNA by *in vitro* transcription;
(ii) contacting the RNA preparation with a cellulose material under conditions which allow binding of double-stranded RNA (dsRNA) and ssRNA to the cellulose material; and
(iii) separating the ssRNA from the cellulose material under conditions which allow binding of dsRNA to the cellulose material and do not allow binding of ssRNA to the cellulose material,
wherein
step (iii) comprises:
(1) mixing the cellulose material to which dsRNA and ssRNA are bound with a first buffer under shaking and/or stirring, wherein the first buffer comprises water, ethanol and a salt in a concentration which allows binding of dsRNA to the cellulose material and does not allow binding of ssRNA to the cellulose material; and
(2) separating the liquid phase comprising ssRNA from the cellulose material;
and
the concentration of ethanol in the first buffer is 14 to 20% (v/v), the concentration of the salt in the first buffer is 15 to 70 mM and the first buffer comprises tris(hydroxymethyl)aminomethane (TRIS) as a buffering substance.

3. The method of claim 1, wherein step (ii) comprises mixing the RNA preparation comprising ssRNA with the cellulose material under shaking and/or stirring, preferably for at least 5 min, more preferably for at least 10 min.

4. The method of claim 3, wherein
(a) the salt comprised in the first buffer is sodium chloride; and/or
(b) the concentration of ethanol in the first buffer is 14 to 16% (v/v); and/or
(c) the concentration of the salt in the first buffer is 20 to 60 mM; and/or
(d) the first buffer further comprises a chelating agent, preferably EDTA; and/or
(e) in step (iii)
(α) the mixture of the RNA preparation, the cellulose material, and the first buffer is provided in a tube and step (iii) comprises (1) applying gravity or centrifugal force to the tube such that the liquid and solid phases are separated; and (2) either collecting the supernatant comprising ssRNA or removing the cellulose material; or
(β) the mixture of the RNA preparation, the cellulose material, and the first buffer is provided in a spin column or filter device and step (iii) comprises (1') applying gravity, centrifugal force, pressure, or vacuum to the spin column or filter device such that the liquid and solid phases are separated; and (2') collecting the flow through comprising ssRNA.

5. The method of any one of claims 1, 3, and 4, wherein steps (ii) and (iii) are repeated once or two or more times, wherein the ssRNA preparation obtained after step (iii) of one cycle of steps (ii) and (iii) is used as RNA preparation in step (ii) of the next cycle and in step (ii) of each cycle of steps (ii) and (iii) fresh cellulose material is used.

6. The method of claim 2, wherein step (ii) comprises
(1) mixing the RNA preparation comprising ssRNA with the cellulose material under shaking and/or stirring, preferably for at least 5 min, more preferably for at least 10 min; and
(2) separating the cellulose material to which dsRNA and ssRNA are bound from the remainder.

7. The method of claim 6, wherein in step (ii) the RNA preparation is provided as a liquid comprising ssRNA and a second buffer and/or the cellulose material is provided as a suspension in a second buffer, wherein the second buffer comprises water, ethanol and a salt, preferably sodium chloride, in a concentration which allows binding of dsRNA and ssRNA to the cellulose material.

8. The method of claim 7, wherein
(a) the concentration of ethanol in the second buffer is at least 35% (v/v), preferably 38 to 42% (v/v); and/or
(b) the concentration of the salt in the second buffer is 15 to 70 mM, preferably 20 to 60 mM; and/or
(c) the second buffer further comprises a buffering substance, preferably tris(hydroxymethyl)aminomethane (TRIS), and/or a chelating agent, preferably EDTA; and/or
(d) in step (ii)(2)
(α) the mixture of the RNA preparation and the cellulose material obtained in step (ii)(1) is provided in a tube and step (ii)(2) comprises (2a) applying gravity or centrifugal force to the tube such that the liquid and solid phases are separated; and (2b) either removing the supernatant or collecting the cellulose material to which dsRNA and ssRNA are bound; or
(β) the mixture of the RNA preparation and the cellulose material obtained in step (ii)(1) is provided in a spin column or filter device and step (ii)(2) comprises (2a') applying gravity, centrifugal force, pressure, or vacuum to the spin column or filter device such that the liquid and solid phases are separated; and (2b') discarding the flow through; and/or
(e) step (ii) further comprises (3) adding an aliquot of the second buffer to the cellulose material to which dsRNA and ssRNA are bound; (4) incubating the resulting mixture under shaking and/or stirring, preferably for at least 5 min, more preferably for at least 10 min; and (5) separating the cellulose material to which dsRNA and ssRNA are bound from the liquid phase; and optionally (6) repeating steps (3) to (5) once or two or more times.

9. The method of any one of claims 2 and 6 to 8, wherein
(a) in step (iii)(1) the cellulose material to which dsRNA and ssRNA are bound is mixed with the first buffer under shaking and/or stirring for at least 5 min, preferably for at least 10 min; and/or
(b) the salt comprised in the first buffer is sodium chloride; and/or
(c) the concentration of ethanol in the first buffer is 14 to 16% (v/v); and/or
(d) the concentration of the salt in the first buffer is 20 to 60 mM; and/or
(e) the first buffer further comprises a chelating agent, preferably EDTA; and/or
(f) in step (iii)
(α) the mixture of the cellulose material and the first buffer is provided in a tube and step (iii)(2) comprises (2a) applying gravity or centrifugal force to the tube such that the liquid and solid phases are separated; and (2b) either collecting the supernatant comprising ssRNA or removing the cellulose material; or
(β) the mixture of the cellulose material and the first buffer is provided in a spin column or filter device and step (iii)(2) comprises (2a') applying gravity, centrifugal force, pressure, or vacuum to the spin column or filter device; and (2b') collecting the flow through comprising ssRNA; and/or
(g) steps (ii) and (iii) are repeated once or two or more times, wherein the ssRNA preparation obtained after step (iii) of one cycle of steps (ii) and (iii) is used as RNA preparation in step (ii) of the next cycle and in step (ii) of each cycle of steps (ii) and (iii) fresh cellulose material is used.

10. The method of claim 2, wherein in step (ii) the cellulose material is provided in a column, step (ii) comprises loading the RNA preparation onto the column under conditions which allow binding of dsRNA and ssRNA to the cellulose material, and step (iii) comprises eluting the ssRNA from the cellulose material under conditions which allow binding of dsRNA to the cellulose material and do not allow binding of ssRNA to the cellulose material.

11. The method of claim 10, wherein in step (ii) the RNA preparation is provided and loaded on the column as a liquid comprising ssRNA and a second buffer, wherein the second buffer comprises water, ethanol and a salt, preferably sodium chloride, in a concentration which allows binding of dsRNA and ssRNA to the cellulose material.

12. The method of claim 11, wherein
(a) the concentration of ethanol in the second buffer is at least 35% (v/v), preferably 38 to 42% (v/v); and/or
(b) the concentration of the salt in the second buffer is 15 to 70 mM, preferably 20 to 60 mM; and/or
(c) the second buffer further comprises a buffering substance, preferably tris(hydroxymethyl)aminomethane (TRIS), and/or a chelating agent, preferably EDTA; and/or
(d) step (iii) is conducted using the first buffer as eluent, wherein the first buffer is preferably the first buffer defined in claim 9(b), claim 9(c), claim 9(d), and/or claim 9(e).

13. The method of any one of claims 1 to 12, wherein
(a) the RNA preparation is produced by using an RNA polymerase selected from the group consisting of T3, T7 and SP6 RNA polymerases; and/or
(b) prior to step (ii) the RNA preparation is subjected to at least one pre-purification treatment, wherein the at least one pre-purification treatment preferably comprises one or more of the following: precipitation of nucleic acids; binding of nucleic acids to magnetic beads; ultrafiltration; and degradation of DNA; and/or
(c) the ssRNA is mRNA or an inhibitory RNA, such as antisense RNA, siRNA, or miRNA; and/or
(d) the ssRNA has a length of at least 2700 nt, preferably at least 3000 nt, more preferably at least 3500 nt, more preferably at least 4500 nt; and/or
(e) the cellulose material comprises cellulose fibers, preferably cellulose fibers of a grade suitable for use as a partition chromatography reagent; and/or
(f) prior to contacting with the RNA preparation in step (ii) the cellulose material is provided as a washed cellulose material.

14. The method of claim 13(f), wherein the washing of the cellulose material includes
(I) mixing the cellulose material with a washing solution under shaking and/or stirring, preferably for at least 5 min; and
(II) either removing the liquid or collecting the cellulose material; and optionally
(III) repeating steps (I) and (II) once or two or more times.

15. The method of claim 14, wherein
(A) if step (ii) is conducted under conditions which allow binding of dsRNA to the washed cellulose material and do not allow binding of ssRNA to the washed cellulose material, the washing solution has the composition of the first buffer defined in claim 1, claim 4(a), claim 4(b), claim 4(c), and/or claim 4(d), or
(B) if step (ii) is conducted under conditions which allow binding of dsRNA and ssRNA to the washed cellulose material, the washing solution has the composition of the second buffer defined in claim 7, claim 8(a), claim 8(b), and/or claim 8(c).

## Patentansprüche

1. Verfahren zum Bereitstellen von einzelsträngiger RNA (ssRNA), umfassend:
(i) Herstellen einer RNA-Zubereitung, die ssRNA umfasst, durch In-vitro-Transkription;
(ii) In-Kontakt-Bringen der RNA-Zubereitung mit einem Cellulose-Material unter Bedingungen, die ein Binden von doppelsträngiger RNA (dsRNA) an das Cellulose-Material erlauben und ein Binden von ssRNA an das Cellulose-Material nicht erlauben; und
(iii) Separieren der ssRNA von dem Cellulose-Material unter Bedingungen, die ein Binden von dsRNA an das Cellulose-Material erlauben und ein Binden von ssRNA an das Cellulose-Material nicht erlauben,
wobei
in Schritt (ii) die RNA-Zubereitung als eine Flüssigkeit, die ssRNA und einen ersten Puffer umfasst, bereitgestellt wird und/oder das Cellulose-Material als eine Suspension in einem ersten Puffer bereitgestellt wird, wobei der erste Puffer Wasser, Ethanol und ein Salz in einer Konzentration, die ein Binden von dsRNA an das Cellulose-Material erlaubt und die ein Binden von ssRNA an das Cellulose-Material nicht erlaubt, umfasst; und
die Konzentration an Ethanol in dem ersten Puffer 14 bis 20% (Vol./Vol.) beträgt, die Konzentration des Salzes in dem ersten Puffer 15 bis 70 mM beträgt und der erste Puffer Tris(hydroxymethyl)aminomethan (TRIS) als eine Puffersubstanz umfasst.

2. Verfahren zum Bereitstellen von einzelsträngiger RNA (ssRNA), umfassend:
(i) Herstellen einer RNA-Zubereitung, die ssRNA umfasst, durch In-vitro- Transkription;
(ii) In-Kontakt-Bringen der RNA-Zubereitung mit einem Cellulose-Material unter Bedingungen, die ein Binden von doppelsträngiger RNA (dsRNA) und ssRNA an das Cellulose-Material erlauben; und
(iii) Separieren der ssRNA von dem Cellulose-Material unter Bedingungen, die ein Binden von dsRNA an das Cellulose-Material erlauben und ein Binden von ssRNA an das Cellulose-Material nicht erlauben,
wobei
Schritt (iii) umfasst:
(1) Mischen des Cellulose-Materials, an das dsRNA und ssRNA gebunden sind, mit einem ersten Puffer unter Schütteln und/oder Rühren, wobei der erste Puffer Wasser, Ethanol und ein Salz in einer Konzentration, die ein Binden von dsRNA an das Cellulose-Material erlaubt und ein Binden von ssRNA an das Cellulose-Material nicht erlaubt, umfasst; und
(2) Separieren der flüssigen Phase, die ssRNA umfasst, von dem Cellulose-Material;
und
die Konzentration an Ethanol in dem ersten Puffer 14 bis 20% (Vol./Vol.) beträgt, die Konzentration des Salzes in dem ersten Puffer 15 bis 70 mM beträgt und der erste Puffer Tris(hydroxymethyl)aminomethan (TRIS) als eine Puffersubstanz umfasst.

3. Verfahren nach Anspruch 1, wobei Schritt (ii) ein Mischen der RNA-Zubereitung, die ssRNA umfasst, mit dem Cellulose-Material unter Schütteln und/oder Rühren, vorzugsweise für mindestens 5 min, mehr bevorzugt für mindestens 10 min, umfasst.

4. Verfahren nach Anspruch 3, wobei
(a) das in dem ersten Puffer umfasste Salz Natriumchlorid ist; und/oder
(b) die Konzentration an Ethanol in dem ersten Puffer 14 bis 16% (Vol./Vol.) beträgt; und/oder
(c) die Konzentration des Salzes in dem ersten Puffer 20 bis 60 mM beträgt; und/oder
(d) der erste Puffer ferner ein Chelatierungsmittel, vorzugsweise EDTA, umfasst; und/oder
(e) in Schritt (iii)
(α) das Gemisch der RNA-Zubereitung, des Cellulose-Materials und des ersten Puffers in einem Röhrchen bereitgestellt wird und Schritt (iii) (1) Anwenden von Schwerkraft oder Zentrifugalkraft auf das Röhrchen, so dass die flüssige und feste Phase separiert werden; und (2) entweder Sammeln des Überstandes, der ssRNA umfasst, oder Entfernen des Cellulose-Materials umfasst; oder
(β) das Gemisch der RNA-Zubereitung, des Cellulose-Materials und des ersten Puffers in einer Spinsäule oder Filtervorrichtung bereitgestellt wird und Schritt (iii) (1') Anwenden von Schwerkraft, Zentrifugalkraft, Druck oder Vakuum auf die Spinsäule oder Filtervorrichtung, so dass die flüssige und feste Phase separiert werden; und (2') Sammeln des Durchflusses, der ssRNA umfasst, umfasst.

5. Verfahren nach einem jeglichen der Ansprüche 1, 3 und 4, wobei Schritte (ii) und (iii) einmal oder zweimal oder mehrmals wiederholt werden, wobei die ssRNA-Zubereitung, die nach Schritt (iii) eines Zyklus der Schritte (ii) und (iii) erhalten wurde, als RNA-Zubereitung in Schritt (ii) des nächsten Zyklus verwendet wird und in Schritt (ii) eines jeden Zyklus der Schritte (ii) und (iii) frisches Cellulose-Material verwendet wird.

6. Verfahren nach Anspruch 2, wobei Schritt (ii) umfasst
(1) Mischen der RNA-Zubereitung, die ssRNA umfasst, mit dem Cellulose-Material unter Schütteln und/oder Rühren, vorzugsweise für mindestens 5 min, mehr bevorzugt für mindestens 10 min; und
(2) Separieren des Cellulose-Materials, an das dsRNA und ssRNA gebunden sind, von dem Rest.

7. Verfahren nach Anspruch 6, wobei in Schritt (ii) die RNA-Zubereitung als eine Flüssigkeit, die ssRNA und einen zweiten Puffer umfasst, bereitgestellt wird und/oder das Cellulose-Material als eine Suspension in einem zweiten Puffer bereitgestellt wird, wobei der zweite Puffer Wasser, Ethanol und ein Salz, vorzugsweise Natriumchlorid, in einer Konzentration, die ein Binden von dsRNA und ssRNA an das Cellulose-Material erlaubt, umfasst.

8. Verfahren nach Anspruch 7, wobei
(a) die Konzentration an Ethanol in dem zweiten Puffer mindestens 35% (Vol./Vol.), vorzugsweise 38 bis 42% (Vol./Vol.), beträgt; und/oder
(b) die Konzentration des Salzes in dem zweiten Puffer 15 bis 70 mM, vorzugsweise 20 bis 60 mM, beträgt; und/oder
(c) der zweite Puffer ferner eine Puffersubstanz, vorzugsweise Tris(hydroxymethyl)-aminomethan (TRIS), und/oder ein Chelatierungsmittel, vorzugsweise EDTA, umfasst; und/oder
(d) in Schritt (ii)(2)
(α) das Gemisch der RNA-Zubereitung und des Cellulose-Materials, das in Schritt (ii)(1) erhalten wurde, in einem Röhrchen bereitgestellt wird und Schritt (ii)(2) (2a) Anwenden von Schwerkraft oder Zentrifugalkraft auf das Röhrchen, so dass die flüssige und feste Phase separiert werden; und (2b) entweder Entfernen des Überstandes oder Sammeln des Cellulose-Materials, an das dsRNA und ssRNA gebunden sind, umfasst; oder
(β) das Gemisch der RNA-Zubereitung und des Cellulose-Materials, das in Schritt (ii)(1) erhalten wurde, in einer Spinsäule oder Filtervorrichtung bereitgestellt wird und Schritt (ii)(2) (2a') Anwenden von Schwerkraft, Zentrifugalkraft, Druck oder Vakuum auf die Spinsäule oder Filtervorrichtung, so dass die flüssige und feste Phase separiert werden; und (2b') Verwerfen des Durchflusses umfasst; und/oder
(e) Schritt (ii) ferner (3) Zugeben eines Aliquots des zweiten Puffers zu dem Cellulose-Material, an das dsRNA und ssRNA gebunden sind; (4) Inkubieren des sich ergebenen Gemisches unter Schütteln und/oder Rühren, vorzugsweise für mindestens 5 min, mehr bevorzugt für mindestens 10 min; und (5) Separieren des Cellulose-Materials, an das dsRNA und ssRNA gebunden sind, von der flüssigen Phase; und gegebenenfalls (6) Wiederholen der Schritte (3) bis (5) einmal oder zweimal oder mehrmals umfasst.

9. Verfahren nach einem jeglichen der Ansprüche 2 und 6 bis 8, wobei
(a) in Schritt (iii)(1) das Cellulose-Material, an das dsRNA und ssRNA gebunden sind, mit dem ersten Puffer unter Schütteln und/oder Rühren, vorzugsweise für mindestens 5 min, mehr bevorzugt für mindestens 10 min, gemischt wird; und/oder
(b) das in dem ersten Puffer umfasste Salz Natriumchlorid ist; und/oder
(c) die Konzentration an Ethanol in dem ersten Puffer 14 bis 16% (Vol./Vol.) beträgt; und/oder
(d) die Konzentration des Salzes in dem ersten Puffer 20 bis 60 mM beträgt; und/oder
(e) der erste Puffer ferner ein Chelatierungsmittel, vorzugsweise EDTA, umfasst; und/oder
(f) in Schritt (iii)
(α) das Gemisch des Cellulose-Materials und des ersten Puffers in einem Röhrchen bereitgestellt wird und Schritt (iii)(2) (2a) Anwenden von Schwerkraft oder Zentrifugalkraft auf das Röhrchen, so dass die flüssige und feste Phase separiert werden; und (2b) entweder Sammeln des Überstandes, der ssRNA umfasst, oder Entfernen des Cellulose-Materials umfasst; oder
(β) das Gemisch des Cellulose-Materials und des ersten Puffers in einer Spinsäule oder Filtervorrichtung bereitgestellt wird und Schritt (iii)(2) (2a') Anwenden von Schwerkraft, Zentrifugalkraft, Druck oder Vakuum auf die Spinsäule oder Filtervorrichtung; und (2b') Sammeln des Durchflusses, der ssRNA umfasst, umfasst; und/oder
(g) Schritte (ii) und (iii) einmal oder zweimal oder mehrmals wiederholt werden, wobei die ssRNA-Zubereitung, die nach Schritt (iii) eines Zyklus der Schritte (ii) und (iii) erhalten wurde, als RNA-Zubereitung in Schritt (ii) des nächsten Zyklus verwendet wird und in Schritt (ii) eines jeden Zyklus der Schritte (ii) und (iii) frisches Cellulose-Material verwendet wird.

10. Verfahren nach Anspruch 2, wobei in Schritt (ii) das Cellulose-Material in einer Säule bereitgestellt wird, Schritt (ii) ein Laden der RNA-Zubereitung auf die Säule unter Bedingungen, die ein Binden von dsRNA und ssRNA an das Cellulose-Material erlauben, umfasst und Schritt (iii) ein Eluieren der ssRNA von dem Cellulose-Material unter Bedingungen, die ein Binden von dsRNA an das Cellulose-Material erlauben und ein Binden von ssRNA an das Cellulose-Material nicht erlauben, umfasst.

11. Verfahren nach Anspruch 10, wobei in Schritt (ii) die RNA-Zubereitung als eine Flüssigkeit, die ssRNA und einen zweiten Puffer umfasst, bereitgestellt und auf die Säule geladen wird, wobei der zweite Puffer Wasser, Ethanol und ein Salz, vorzugsweise Natriumchlorid, in einer Konzentration, die ein Binden von dsRNA und ssRNA an das Cellulose-Material erlaubt, umfasst.

12. Verfahren nach Anspruch 11, wobei
(a) die Konzentration an Ethanol in dem zweiten Puffer mindestens 35% (Vol./Vol.), vorzugsweise 38 bis 42% (Vol./Vol.), beträgt; und/oder
(b) die Konzentration des Salzes in dem zweiten Puffer 15 bis 70 mM, vorzugsweise 20 bis 60 mM, beträgt; und/oder
(c) der zweite Puffer ferner eine Puffersubstanz, vorzugsweise Tris(hydroxymethyl)-aminomethan (TRIS), und/oder ein Chelatierungsmittel, vorzugsweise EDTA, umfasst; und/oder
(d) Schritt (iii) unter Verwendung des ersten Puffers als Elutionsmittel durchgeführt wird, wobei der erste Puffer vorzugsweise der in Anspruch 9(b), Anspruch 9(c), Anspruch 9(d) und/oder Anspruch 9(e) definierte erste Puffer ist.

13. Verfahren nach einem jeglichen der Ansprüche 1 bis 12, wobei
(a) die RNA-Zubereitung unter Verwendung einer RNA-Polymerase, ausgewählt aus der Gruppe, bestehend aus T3-, T7- und SP6-RNA-Polymerasen, hergestellt wird, und/oder
(b) vor Schritt (ii) die RNA-Zubereitung mindestens einer Voraufreinigungsbehandlung unterzogen wird, wobei die mindestens eine Voraufreinigungsbehandlung vorzugsweise eines oder mehrere der nachstehenden umfasst: Präzipitation von Nukleinsäuren, Binden von Nukleinsäuren an Magnetkügelchen, Ultrafiltration und Abbau von DNA; und/oder
(c) die ssRNA mRNA oder eine hemmende RNA, wie eine Antisense-RNA, siRNA oder miRNA, ist; und/oder
(d) die ssRNA eine Länge von mindestens 2700 nt, vorzugsweise mindestens 3000 nt, mehr bevorzugt mindestens 3500 nt, mehr bevorzugt mindestens 4500 nt, aufweist; und/oder
(e) das Cellulose-Material Cellulose-Fasern, vorzugsweise Cellulose-Fasern einer Güte, die für eine Verwendung als ein Verteilungschromatographiereagenz geeignet ist, umfasst; und/oder
(f) vor dem In-Kontakt-Bringen der RNA-Zubereitung in Schritt (ii) das Cellulose-Material als ein gewaschenes Cellulose-Material bereitgestellt wird.

14. Verfahren nach Anspruch 13(f), wobei das Waschen des Cellulose-Materials beinhaltet
(I) Mischen des Cellulose-Materials mit einer Waschlösung unter Schütteln und/oder Rühren, vorzugsweise für mindestens 5 min; und
(II) entweder Entfernen der Flüssigkeit oder Sammeln des Cellulose-Materials; und gegebenenfalls
(III) Wiederholen der Schritte (I) und (II) einmal oder zweimal oder mehrmals.

15. Verfahren nach Anspruch 14, wobei
(A) falls Schritt (ii) unter Bedingungen durchgeführt wird, die ein Binden von dsRNA an das gewaschene Cellulose-Material erlauben und ein Binden von ssRNA an das gewaschene Cellulose-Material nicht erlauben, die Waschlösung die Zusammensetzung des ersten Puffers, der in Anspruch 1, Anspruch 4(a), Anspruch 4(b), Anspruch 4(c) and/oder Anspruch 4(d) definiert ist, aufweist, oder
(B) falls Schritt (ii) unter Bedingungen durchgeführt wird, die ein Binden von dsRNA und ssRNA an das gewaschene Cellulose-Material erlauben, die Waschlösung die Zusammensetzung des zweiten Puffers, der in Anspruch 7, Anspruch 8(a), Anspruch 8(b) und/oder Anspruch 8(c) definiert ist, aufweist.

## Revendications

1. Procédé de fourniture d'ARN simple brin (ARNsb), comprenant :
(i) la production d'une préparation d'ARN comprenant de l'ARNsb par transcription *in vitro ;*
(ii) la mise en contact de la préparation d'ARN avec un matériau cellulosique dans des conditions qui permettent la liaison d'ARN double brin (ARNdb) au matériau cellulosique et ne permettent pas la liaison d'ARNsb au matériau cellulosique ; et
(iii) la séparation de l'ARNsb du matériau cellulosique dans des conditions qui permettent la liaison d'ARNdb au matériau cellulosique et ne permettent pas la liaison d'ARNsb au matériau cellulosique,
dans lequel
à l'étape (ii) la préparation d'ARN est fournie sous la forme d'un liquide comprenant de l'ARNdb et un premier tampon et/ou le matériau cellulosique est fourni sous la forme d'une suspension dans un premier tampon, dans lequel le premier tampon comprend de l'eau, de l'éthanol et un sel à une concentration qui permet la liaison d'ARNdb au matériau cellulosique et qui ne permet pas la liaison d'ARNsb au matériau cellulosique ; et
la concentration de l'éthanol dans le premier tampon est de 14 à 20 % (v/v), la concentration du sel dans le premier tampon est de 15 à 70 mM et le premier tampon comprend du tris(hydroxyméthyl)amino-méthane (TRIS) comme substance tampon.

2. Procédé de fourniture d'ARN simple brin (ARNsb), comprenant :
(i) la production d'une préparation d'ARN comprenant de l'ARNsb par transcription *in vitro ;*
(ii) la mise en contact de la préparation d'ARN avec un matériau cellulosique dans des conditions qui permettent la liaison d'ARN double brin (ARNdb) et d'ARNsb au matériau cellulosique ; et
(iii) la séparation de l'ARNsb du matériau cellulosique dans des conditions qui permettent la liaison d'ARNdb au matériau cellulosique et ne permettent pas la liaison d'ARNsb au matériau cellulosique,
dans lequel
l'étape (iii) comprend :
(1) le mélange du matériau cellulosique auquel l'ARNdb et l'ARNsb sont liés avec un premier tampon sous secousse et/ou agitation, dans lequel le premier tampon comprend de l'eau, de l'éthanol et un sel à une concentration qui permet la liaison d'ARNdb au matériau cellulosique et ne permet pas la liaison d'ARNsb au matériau cellulosique ; et
(2) la séparation de la phase liquide comprenant de l'ARNsb du matériau cellulosique ;
et
la concentration de l'éthanol dans le premier tampon est de 14 à 20 % (v/v), la concentration du sel dans le premier tampon est de 15 à 70 mM et le premier tampon comprend du tris(hydroxyméthyl)amino-méthane (TRIS) comme substance tampon.

3. Procédé selon la revendication 1, dans lequel l'étape (ii) comprend le mélange de la préparation d'ARN comprenant de l'ARNsb avec le matériau cellulosique sous secousse et/ou agitation, de préférence pendant au moins 5 min, de manière davantage préférée pendant au moins 10 min.

4. Procédé selon la revendication 3, dans lequel
(a) le sel compris dans le premier tampon est le chlorure de sodium ; et/ou
(b) la concentration d'éthanol dans le premier tampon est de 14 à 16 % (v/v) ; et/ou
(c) la concentration du sel dans le premier tampon est de 20 à 60 mM ; et/ou
(d) le premier tampon comprend en outre un agent chélatant, de préférence l'EDTA ; et/ou
(e) à l'étape (iii)
(α) le mélange de la préparation d'ARN, du matériau cellulosique, et du premier tampon est fourni dans un tube et l'étape (iii) comprend (1) l'application de gravité ou de force centrifuge au tube de sorte que les phases liquide et solide se séparent ; et (2) soit le recueil du surnageant comprenant de l'ARNsb, soit l'élimination du matériau cellulosique ; ou
(β) le mélange de la préparation d'ARN, du matériau cellulosique, et du premier tampon est fourni dans une colonne de spin ou un dispositif de filtre et l'étape (iii) comprend (1') l'application de gravité, de force centrifuge, de pression, ou de vide à la colonne de spin ou au dispositif de filtre de sorte que les phases liquide et solide se séparent ; et (2') le recueil de l'écoulement traversant comprenant de l'ARNsb.

5. Procédé selon l'une quelconque des revendications 1, 3, et 4, dans lequel les étapes (ii) et (iii) sont répétées une fois ou deux fois ou davantage, dans lequel la préparation d'ARNsb obtenue après l'étape (iii) d'un cycle d'étapes (ii) et (iii) est utilisée en tant que préparation d'ARN à l'étape (ii) du cycle suivant et à l'étape (ii) de chaque cycle d'étapes (ii) et (iii) un matériau cellulosique neuf est utilisé.

6. Procédé selon la revendication 2, dans lequel l'étape (ii) comprend
(1) le mélange de la préparation d'ARN comprenant de l'ARNsb avec le matériau cellulosique sous secousse et/ou agitation, de préférence pendant au moins 5 min, de manière davantage préférée pendant au moins 10 min ; et
(2) la séparation du matériau cellulosique auquel sont liés l'ARNdb et l'ARNsb du reste.

7. Procédé selon la revendication 6, dans lequel à l'étape (ii) la préparation d'ARN est fournie sous la forme d'un liquide comprenant de l'ARNsb et un deuxième tampon et/ou le matériau cellulosique est fourni sous la forme d'une suspension dans un deuxième tampon, dans lequel le deuxième tampon comprend de l'eau, de l'éthanol et un sel, de préférence le chlorure de sodium, à une concentration qui permet la liaison d'ARNdb et d'ARNsb au matériau cellulosique.

8. Procédé selon la revendication 7, dans lequel
(a) la concentration d'éthanol dans le deuxième tampon est d'au moins 35 % (v/v), de préférence de 38 à 42 % (v/v) ; et/ou
(b) la concentration du sel dans le deuxième tampon est de 15 à 70 mM, de préférence de 20 à 60 mM ; et/ou
(c) le deuxième tampon comprend en outre une substance de tamponnage, de préférence le tris(hydroxyméthyl)aminométhane (TRIS), et/ou un agent chélatant, de préférence l'EDTA ; et/ou
(d) à l'étape (ii)(2)
(α) le mélange de la préparation d'ARN et du matériau cellulosique obtenu à l'étape (ii)(1) est fourni dans un tube et l'étape (ii)(2) comprend (2a) l'application de gravité ou de force centrifuge au tube de sorte que les phases liquide et solide se séparent ; et (2b) soit l'élimination du surnageant, soit le recueil du matériau cellulosique auquel sont liés l'ARNdb et l'ARNsb ; ou
(β) le mélange de la préparation d'ARN et du matériau cellulosique obtenu à l'étape (ii)(1) est fourni dans une colonne de spin ou un dispositif de filtre et l'étape (ii)(2) comprend (2a') l'application de gravité, de force centrifuge, de pression, ou de vide à la colonne de spin ou au dispositif de filtre de sorte que les phases liquide et solide se séparent ; et (2b') le rejet de l'écoulement traversant ; et/ou
(e) l'étape (ii) comprend en outre (3) l'ajout d'une aliquote du deuxième tampon au matériau cellulosique auquel sont liés l'ARNdb et l'ARNsb ; (4) l'incubation du mélange résultant sous secousse et/ou agitation, de préférence pendant au moins 5 min, de manière davantage préférée pendant au moins 10 min ; et (5) la séparation du matériau cellulosique auquel sont liés l'ARNdb et l'ARNsb de la phase liquide ; et éventuellement (6) la répétition des étapes (3) à (5) une fois ou deux fois ou davantage.

9. Procédé selon l'une quelconque des revendications 2 et 6 à 8, dans lequel
(a) à l'étape (iii)(1) le matériau cellulosique auquel sont liés l'ARNdb et l'ARNsb est mélangé avec le premier tampon sous secousse et/ou agitation pendant au moins 5 min, de préférence pendant au moins 10 min; et/ou
(b) le sel compris dans le premier tampon est le chlorure de sodium ; et/ou
(c) la concentration d'éthanol dans le premier tampon est de 14 à 16 % (v/v) ; et/ou
(d) la concentration du sel dans le premier tampon est de 20 à 60 mM ; et/ou
(e) le premier tampon comprend un agent chélatant, de préférence l'EDTA ; et/ou
(f) à l'étape (iii)
(α) le mélange du matériau cellulosique et du premier tampon est fourni dans un tube et l'étape (iii)(2) comprend (2a) l'application de gravité ou de force centrifuge au tube de sorte que les phases liquide et solide se séparent ; et (2b) soit le recueil du surnageant comprenant de l'ARNsb, soit l'élimination du matériau cellulosique ; ou
(β) le mélange du matériau cellulosique et du premier tampon est fourni dans une colonne de spin ou un dispositif de filtre et l'étape (iii)(2) comprend (2a') l'application de gravité, de force centrifuge, de pression, ou de vide à la colonne de spin ou au dispositif de filtre ; et (2b') le recueil de l'écoulement traversant comprenant de l'ARNsb ; et/ou
(g) les étapes (ii) et (iii) sont répétées une fois ou deux fois ou davantage, dans lequel la préparation d'ARNsb obtenue après l'étape (iii) d'un cycle d'étapes (ii) et (iii) est utilisée en tant que préparation d'ARN à l'étape (ii) du cycle suivant et à l'étape (ii) de chaque cycle d'étapes (ii) et (iii) un matériau cellulosique neuf est utilisé.

10. Procédé selon la revendication 2, dans lequel à l'étape (ii) le matériau cellulosique est fourni dans une colonne, l'étape (ii) comprend le chargement de la préparation d'ARN sur la colonne dans des conditions qui permettent la liaison d'ARNdb et d'ARNsb au matériau cellulosique, et l'étape (iii) comprend l'élution de l'ARNsb du matériau cellulosique dans des conditions qui permettent la liaison d'ARNdb au matériau cellulosique et ne permettent pas la liaison d'ARNsb au matériau cellulosique.

11. Procédé selon la revendication 10, dans lequel à l'étape (ii) la préparation d'ARN est fournie et chargée sur la colonne sous la forme d'un liquide comprenant de l'ARNsb et un deuxième tampon, dans lequel le deuxième tampon comprend de l'eau, de l'éthanol et un sel, de préférence le chlorure de sodium, à une concentration qui permet la liaison d'ARNdb et d'ARNsb au matériau cellulosique.

12. Procédé selon la revendication 11, dans lequel
(a) la concentration d'éthanol dans le deuxième tampon est d'au moins 35 % (v/v), de préférence de 38 à 42 % (v/v) ; et/ou
(b) la concentration du sel dans le deuxième tampon est de 15 à 70 mM, de préférence de 20 à 60 mM ; et/ou
(c) le deuxième tampon comprend une substance de tamponnage, de préférence le tris(hydroxyméthyl)aminométhane (TRIS), et/ou un agent chélatant, de préférence l'EDTA ; et/ou
(d) l'étape (iii) est effectuée à l'aide du premier tampon en tant qu'éluant, dans lequel le premier tampon est de préférence le premier tampon défini dans la revendication 9(b), la revendication 9(c), la revendication 9(d), et/ou la revendication 9(e).

13. Procédé selon l'une quelconque des revendications 1 à 12, dans lequel
(a) la préparation d'ARN est produite par l'utilisation d'une ARN polymérase choisie dans le groupe constitué par les ARN polymérases T3, T7 et SP6 ; et/ou
(b) avant l'étape (ii) la préparation d'ARN est soumise à au moins un traitement de pré-purification, dans lequel l'au moins un traitement de pré-purification comprend de préférence au moins l'une parmi : la précipitation d'acides nucléiques ; la liaison d'acides nucléiques à des billes magnétiques ; l'ultrafiltration ; et la dégradation d'ADN ; et/ou
(c) l'ARNsb est un ARNm ou un ARN inhibiteur tel qu'une ARN antisens, un ARNsi, ou un ARNmi ; et/ou
(d) l'ARNsb a une longueur d'au moins 2 700 nt, de préférence d'au moins 3 000 nt, de manière davantage préférée d'au moins 3 500 nt, de manière davantage préférée d'au moins 4 500 nt ; et/ou
(e) le matériau cellulosique comprend des fibres de cellulose, de préférence des fibres de cellulose de qualité appropriée pour une utilisation en tant que réactif de chromatographie de partage ; et/ou
(f) avant la mise en contact avec la préparation d'ARN à l'étape (ii) le matériau cellulosique est fourni sous la forme d'un matériau cellulosique lavé.

14. Procédé selon la revendication 13(f), dans lequel le lavage du matériau cellulosique comporte
(I) le mélange du matériau cellulosique avec une solution de lavage sous secousse et/ou agitation, de préférence pendant au moins 5 min ; et
(II) soit l'élimination du liquide, soit le recueil du matériau cellulosique ; et éventuellement
(III) la répétition des étapes (I) et (II) une fois ou deux fois ou davantage.

15. Procédé selon la revendication 14, dans lequel
(A) si l'étape (ii) est effectuée dans des conditions qui permettent la liaison d'ARNdb au matériau cellulosique lavé et ne permettent pas la liaison d'ARNsb au matériau cellulosique lavé, la solution de lavage a la composition du premier tampon défini dans la revendication 1, la revendication 4(a), la revendication 4(b), la revendication 4(c), et/ou la revendication 4(d), ou
(B) si l'étape (ii) est effectuée dans des conditions qui permettent la liaison d'ARNdb et d'ARNsb au matériau cellulosique lavé, la solution de lavage a la composition du deuxième tampon défini dans la revendication 7, la revendication 8(a), la revendication 8(b), et/ou la revendication 8(c).
